(19) Europäisches Patentamt
European Patent Office
Office européen des brevets

(11) EP 1 876 239 A1

(12) **EUROPEAN PATENT APPLICATION**
published in accordance with Art. 153(4) EPC

(43) Date of publication:
**09.01.2008 Bulletin 2008/02**

(21) Application number: **06746044.4**

(22) Date of filing: **27.04.2006**

(51) Int Cl.:
***C12N 15/09*** *(2006.01)* ***A61K 31/7052*** *(2006.01)*
***A61K 39/395*** *(2006.01)* ***A61K 45/00*** *(2006.01)*
***A61K 48/00*** *(2006.01)* ***A61P 1/00*** *(2006.01)*
***A61P 1/04*** *(2006.01)* ***A61P 1/16*** *(2006.01)*
***A61P 3/04*** *(2006.01)* ***A61P 3/06*** *(2006.01)*
***A61P 3/10*** *(2006.01)* ***A61P 9/04*** *(2006.01)*
***A61P 9/06*** *(2006.01)* ***A61P 9/10*** *(2006.01)*
***A61P 11/00*** *(2006.01)* ***A61P 11/06*** *(2006.01)*
***A61P 13/00*** *(2006.01)* ***A61P 15/00*** *(2006.01)*
***A61P 17/00*** *(2006.01)* ***A61P 21/04*** *(2006.01)*
***A61P 27/02*** *(2006.01)*

(86) International application number:
**PCT/JP2006/309212**

(87) International publication number:
**WO 2006/118328 (09.11.2006 Gazette 2006/45)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **28.04.2005 JP 2005133503**

(71) Applicant: **Takeda Pharmaceutical Company Limited**
**Osaka-shi,**
**Osaka 541-0045 (JP)**

(72) Inventors:
- **OGI, Kazuhiro,**
**TAKEDA PHARMACEUTICAL COMPANY LTD.**
**Tsukuba-shi, Ibaraki 3004247 (JP)**
- **KIKUKAWA, Yusuke**
**TAKEDA PHARMACEUTICAL COMPANYLTD**
**Tsukuba-shi, Ibaraki 3004247 (JP)**

(74) Representative: **Jones, Nicholas Andrew**
**Withers & Rogers LLP**
**Goldings House**
**2 Hays Lane**
**London SE1 2HW (GB)**

(54) **DEGRANULATION INHIBITOR**

(57) The present invention provides a screening method/screening kit for a mast cell degranulation inhibitor which is **characterized by** using (a) a protein comprising the same or substantially the same amino acid sequence as the amino acid sequence represented by SEQ ID NO: 1, its partial peptide, or a salt thereof and (b) a ligand capable of specifically binding to the protein; a mast cell degranulation inhibitor obtained by the screening; and so on.

EP 1 876 239 A1

**Description**

TECHNICAL FIELD

The present invention relates to a screening method and screening kit for degranulation inhibitors, etc. using TGR12 or mast cells and a ligand for TGR12, degranulation inhibitors which are obtainable using the screening method or kit, and so on.

BACKGROUND ART

Mast cells cause degranulation by antigenic stimulation after sensitization with an antibody to release chemical mediators such as histamine, leukotriene, serotonin, etc., and are deeply involved in type I hypersensitivity reaction. Mast cells secrete various cytokines after antigenic stimulation to affect the functions of T cells and eosinophils and are also important as immune regulatory cells. Mast cells are located near peripheral nerve terminals and directly affected by neural factors such as substance P, calcitonin gene related peptide (CGRP) supposedly to cause degranulation (Proc. Natl. Acad. Sci., 84, 2975-2979, 1987; Arch. Dermatol. Res., 285, 341-346, 1993). In recent years stress or neural factors are considered to be deeply correlated to the development and exacerbation of allergic diseases. For example, it is reported that transfection of neurons occurs in the skin from stress-exposed mice to induce degranulation of mast cells (Brain Behav. Immun., 19, 252-262, 2005). It is reported that in patients with atopic dermatitis the levels of substance P and nerve growth factor (NGF) in local tissues and blood are deeply correlated to the severity in conditions (Br. J. Dermatol., 147, 71-79, 2002). It is also known that in patients with atopic dermatitis an increased number of mast cells are found in the lesional skin (Arch. Dermatol. Res., 289, 256-260, 1997). Substance P is considered to act also autocrinally since it is produced and released not only in neurons but also in mast cells (Arch. Dermatol. Res., 292, 418-421, 2000). Mast cells are considered to be deeply associated not only with allergic diseases but with pathological conditions such as irritable bowel syndrome, ulcerative colitis, interstitial cystitis, etc., suggesting that neural factors would involved in the development and exacerbation of these diseases (Gastroenterology, 126, 693-702, 2004; Scand. J. Gastroentorl., 40, 129-140, 2005; Am. J. Physiol. Gastrointest. Liver Physiol., 279, G1298-G1306, 2000; Am. J. Physiol. Renal Physiol., 283, F616-F629, 2002).

Human TGR12 (SEQ ID NO: 1), which is a G protein-coupled receptor, is also known as MRGX2, GPCRx11, GPCR44, or the like. It is reported that MRGX2 is activated by cortistatin, somatostatin, BAM (bovine adrenal medulla peptide)13-22, α-melanin stimulating hormone (αMSH), neuropeptide FF, dynorphin A and substance P; and agonists or antagonists for MRGX2 can be used as therapeutic agents for sleep disorders, pain, cerebral infarction, memory disorders, diabetes, cancers, obesity, heart failure, depression, sexual dysfunction, urinary tract and bladder disorders, anti-infective treatments, gastrointestinal disorders, etc. (WO 03/073107). It is reported that angiopeptin reacts specifically with GPCRx11 (WO 01/98330). It is further reported that PAMP-12 (proadrenomedullin N-terminal 20 peptide (9-20)) shows an agonistic effect against MRGX2 and MRGX2 is specifically expressed in the C-fiber dorsal root ganglion (DRG) (Biochem. Biophys. Res. Commun., 330, 1146-1152, 2005). Though it is reported that MRGX2 is expressed in human cord blood-derived mast cells and the expression is modified by IgE (WO 2005/028667), there is no report on any quantitative data of expression level of MRGX2 in mast cells and its expression in human mast cell line LAD 2.

It is known that human mast cells interact with substance P to induce a degranulation-promoting effect (Blood, 97, 2045-2052, 2001), an eicosanoid producing effect (Clin. Exp. Immunol., 124, 150-156, 2001) and a cytokine producing effect including IL-4, IL-8, TNFα, etc. (Br. J. Dermatol., 131, 348-353, 1994; Int. Arch. Allergy Immunol., 117, 48-51, 1994; Exp. Dermatol., 10, 312-320, 2001). However, these effects were considered to be non-specific reactions not mediated by receptors (M. Mousli et al., FEBS Lett., 259 (2), 260-262, 1990 January; R. Saban et al., Am. J. Physiol. Renal Physiol., 283, F616-F629, 2002 May; A. H. Y. Lau et al., European Journal of Pharmacology, 414, 295-303, 2001; D. Lorenz et al., J. Gen. Physiol., 112, 577-591, 1998 November) or those mediated by high-affinity receptors to substance P or NK-1 receptors (S. Guhl et al., J. Neuroimmunology, 163, 92-101, 2005 April).

It is known that in response to pituitary adenylate cyclase activating polypeptide (PACAP), rat peritoneal mast cells or human skin mast cells show a degranulation-promoting effect (Inflamm. Res., 47, 488-492, 1998; Ann. N. Y. Acad. Sci., 865, 141-146, 1998) and show a dermatitis-inducing effect in mice (Regul. Pept., 82, 65-69, 1999), but there is no report of expression levels of PACAP receptors in mast cells.

It is known that human skin mast cells show a degranulation-promoting effect in response to vasoactive intestinal polypeptide (VIP) (Br. J. Pharmacol., 95, 121-130, 1988) but there is no report of expression levels of VIP receptors in mast cells.

DISCLOSURE OF INVENTION

It has been desired to develop excellent degranulation inhibitors of mast cells and excellent preventive/therapeutic agents for immune, respiratory, urologic or circulatory disorders.

The present inventors have made extensive studies to solve the foregoing problems and as a result, found that substance P, cortistatin-17, PAMP-12, PACAP-27, PACAP-38 and vasoactive intestinal polypeptide exhibit an intracellular calcium increasing effect on TGR12-expressed CHO cell line; that human TGR12 is abundantly expressed in human mast cells but a high-affinity receptor or NK1 receptor responsive to substance P, low-affinity receptors or NK2 receptor and NK3 receptor responsive to substance P as well as VIP1 receptor, VIP2 receptor and PACAP receptor responsive to PACAP are rarely expressed in human mast cells; that human mast cell line or LAD 2 is stimulated by substance P, cortistatin-17, PAMP-12, PACAP-27, PACAP-38 and VIP to promote degranulation, and substance P-dependent degranulation in LAD 2 is not inhibited by NK1 receptor antagonist. From the foregoing the inventors have found that TGR12 is involved in degranulation dependent on neuropeptides including substance P in human mast cells. Based on these findings, the present inventors have found that TGR12 antagonists could be degranulation inhibitors of mast cells to provide a simple method for screening the degranulation inhibitors of mast cells and continued extensive studies. As a result, they have come to accomplish the present invention.

The present invention provides the following features and so on.

[1] A method of screening for a mast cell degranulation inhibitor, which comprises using (a) a protein comprising the same or substantially the same amino acid sequence as the amino acid sequence of SEQ ID NO: 1, its partial peptide, or a salt thereof and (b) a ligand capable of specifically binding to said protein.

[2] The screening method according to [1] above, wherein the ligand is substance P, cortistatin, a pituitary adenylate cyclase activating polypeptide (PACAP), a vasoactive intestinal peptide (VIP) or proadrenomedullin N-terminal 20 peptide (PAMP).

[2a] The screening method according to [2] above, wherein the ligand is pituitary adenylate cyclase activating polypeptide.

[3] The screening method according to [1] above, wherein a cell stimulating activity mediated by a protein comprising the same or substantially the same amino acid sequence as the amino acid sequence of SEQ ID NO: 1, its partial peptide, or a salt thereof, is assayed and used as a parameter.

[4] A method of screening for a compound or its salt having an action of inhibiting degranulation induced by a pituitary adenylate cyclase activating polypeptide in human mast cells, which comprises using (a) a protein comprising the same or substantially the same amino acid sequence as the amino acid sequence of SEQ ID NO: 1, its partial peptide, or a salt thereof and (b) a ligand capable of specifically binding to said protein.

[5] The screening method according to [1] above, which comprises:

(i) the step of contacting (a) a protein comprising the same or substantially the same amino acid sequence as the amino acid sequence of SEQ ID NO: 1, its partial peptide, or a salt thereof, with (b) a ligand capable of specifically binding to said protein in the absence of a test compound and measuring the binding amount of said ligand to said protein, its partial peptide, or a salt thereof;
(ii) the step of contacting (a) a protein comprising the same or substantially the same amino acid sequence as the amino acid sequence of SEQ ID NO: 1, its partial peptide, or a salt thereof, with (b) a ligand capable of specifically binding to said protein in the presence of a test compound and measuring the binding amount of said ligand to said protein, its partial peptide, or a salt thereof; and,
(iii) the step of comparing the binding amounts of said ligand to said protein, its partial peptide, or a salt thereof between the step (i) and the step (ii), and selecting said test compound as a mast cell degranulation inhibitor when the binding amount in the step (ii) is lower than the binding amount in the step (i).

[6] The screening method according to [5] above, wherein the test compound is selected as a mast cell degranulation inhibitor when the binding amount in the step (ii) is not greater than 50% than the binding amount in the step (i).

[7] The screening method according to [1] above, which comprises:

(i) the step of contacting (a) a protein comprising the same or substantially the same amino acid sequence as the amino acid sequence of SEQ ID NO: 1, its partial peptide, or a salt thereof, with (b) a ligand capable of specifically binding to said protein in the absence of a test compound and measuring the binding amount of said ligand to said protein, its partial peptide, or a salt thereof;
(ii) the step of contacting (a) a protein comprising the same or substantially the same amino acid sequence as the amino acid sequence of SEQ ID NO: 1, its partial peptide, or a salt thereof, with (b) a ligand capable of specifically binding to said protein in the presence of a test compound and measuring the binding amount of said ligand to said protein, its partial peptide, or a salt thereof;
(iii) the step of comparing the binding amounts of said ligand to said protein, its partial peptide, or a salt thereof between the step (i) and the step (ii), and selecting a test compound when the binding amount in the step (ii) is lower than the binding amount in the step (i); and,
(iv) the step of contacting the test compound obtained in (iii) above with mast cells and selecting a test compound inhibiting degranulation.

[7a] The screening method according to any one of [4] to [7] above, wherein the ligand is substance P, cortistatin, pituitary adenylate cyclase activating polypeptide (PACAP), vasoactive intestinal peptide (VIP) or proadrenomedullin N-terminal 20 peptide (PAMP).
[7b] The screening method according to [7a] above, wherein the ligand is adenylate cyclase activating polypeptide.
[8] The screening method according to [1] above, wherein the mast cell degranulation inhibitor is a preventive/therapeutic agent for immune disorders, urologic disorders, digestive disorders or respiratory disorders.
[9] A kit for screening for a mast cell degranulation inhibitor, which comprises (a) a protein comprising the same or substantially the same amino acid sequence as the amino acid sequence of SEQ ID NO: 1, its partial peptide, or a salt thereof and (b) a ligand capable of specifically binding to said protein.
[10] A mast cell degranulation inhibitor, which comprises an antagonist to a protein comprising the same or substantially the same amino acid sequence as the amino acid sequence of SEQ ID NO: 1, its partial peptide, or a salt thereof.
[11] A mast cell degranulation inhibitor, which comprises an antibody against a protein comprising the same or substantially the same amino acid sequence as the amino acid sequence of SEQ ID NO: 1, its partial peptide, or a salt thereof.
[12] A diagnostic agent for immune disorders, urologic disorders, digestive disorders or respiratory disorders, which comprises an antibody against a protein comprising the same or substantially the same amino acid sequence as the amino acid sequence of SEQ ID NO: 1, its partial peptide, or a salt thereof.
[13] A mast cell degranulation inhibitor, which comprises an antibody against an agonist for a protein comprising the same or substantially the same amino acid sequence as the amino acid sequence of SEQ ID NO: 1, its partial peptide, or a salt thereof.
[14] A diagnostic agent for immune disorders, urologic disorders, digestive disorders or respiratory disorders, which comprises a polynucleotide comprising a polynucleotide encoding a protein comprising the same or substantially the same amino acid sequence as the amino acid sequence of SEQ ID NO: 1, or its partial peptide.
[15] A mast cell degranulation inhibitor comprising an siRNA or shRNA for (i) a polynucleotide comprising an entire or part of a nucleotide sequence complementary or substantially complementary to a polynucleotide comprising a polynucleotide encoding a protein comprising the same or substantially the same amino acid sequence as the amino acid sequence of SEQ ID NO: 1, or its partial peptide or (ii) a polynucleotide comprising a polynucleotide encoding said protein or its partial peptide.
[16] The inhibitor according to [10], [11], [13] or [15] above, wherein the mast cell degranulation inhibitor is a preventive/therapeutic agent for immune disorders, urologic disorders, digestive disorders or respiratory disorders.
[17] A method of inhibiting degranulation of mast cells, which comprises inhibiting the activities of a protein comprising the same or substantially the same amino acid sequence as the amino acid sequence of SEQ ID NO: 1, its partial peptide, or a salt thereof, or the activities of a ligand capable of specifically binding to said protein.
[18] A method of screening for a mast cell degranulation inhibitor, which comprises using mast cells and a ligand capable of specifically binding to a protein comprising the same or substantially the same amino acid sequence as the amino acid sequence of SEQ ID NO: 1.
[19] The screening method according to [18] above, which comprises:

(i) the step of contacting mast cells with a ligand capable of specifically binding to a protein comprising the same or substantially the same amino acid sequence as the amino acid sequence of SEQ ID NO: 1 in the absence of a test compound and measuring the binding amount of said ligand to said mast cell;
(ii) the step of contacting mast cells with a ligand capable of specifically binding to a protein comprising the same or substantially the same amino acid sequence as the amino acid sequence of SEQ ID NO: 1 in the presence of a test compound and measuring the binding amount of said ligand to said mast cell;
(iii) the step of selecting a test compound when the binding amount in the step (ii) is lower than the binding amount in the step (i); and,
(iv) the step of contacting the test compound obtained in (iii) above with mast cells and selecting a test compound inhibiting degranulation.

[20] A kit for screening for a mast cell degranulation inhibitor, which comprises mast cells and a ligand capable of specifically binding to a protein comprising the same or substantially the same amino acid sequence as the amino acid sequence of SEQ ID NO: 1.
[21] A method of inhibiting a mast cell degranulation inhibitor, which comprises administering to a mammal an effective dose of (i) an antagonist to a protein comprising the same or substantially the same amino acid sequence as the amino acid sequence of SEQ ID NO: 1, its partial peptide, or a salt thereof, (ii) an antibody against said protein, its partial peptide, or a salt thereof, or (iii) a polynucleotide comprising an entire or part of a nucleotide sequence complementary or substantially complementary to a polynucleotide comprising a polynucleotide encoding said protein or its partial peptide.

[22] Use of (i) an antagonist to a protein comprising the same or substantially the same amino acid sequence as the amino acid sequence of SEQ ID NO: 1, its partial peptide, or a salt thereof, (ii) an antibody against said protein, its partial peptide, or a salt thereof, or (iii) a polynucleotide comprising an entire or part of a nucleotide sequence complementary or substantially complementary to a polynucleotide comprising a polynucleotide encoding said protein or its partial peptide, for the manufacture of a mast cell degranulation inhibitor.

The present invention further provides the following features and so on.

[23] A method of screening for a compound or its salt that alters the binding properties between a protein comprising the same or substantially the same amino acid sequence as the amino acid sequence of SEQ ID NO: 1, its partial peptide, or a salt thereof, and a pituitary adenylate cyclase activating polypeptide (PACAP), which comprises using (a) said protein, its partial peptide, or a salt thereof and (b) PACAP.

[24] The screening method according to [23] above, which comprises:

(i) the step of contacting (a) a protein comprising the same or substantially the same amino acid sequence as the amino acid sequence of SEQ ID NO: 1, its partial peptide, or a salt thereof, with (b) a pituitary adenylate cyclase activating polypeptide in the absence of a test compound and measuring the binding amount of the pituitary adenylate cyclase activating polypeptide to said protein, its partial peptide, or a salt thereof;
(ii) the step of contacting (a) a protein comprising the same or substantially the same amino acid sequence as the amino acid sequence of SEQ ID NO: 1, its partial peptide, or a salt thereof, with (b) a pituitary adenylate cyclase activating polypeptide in the presence of a test compound and measuring the binding amount of the pituitary adenylate cyclase activating polypeptide to said protein, its partial peptide, or a salt thereof; and,
(iii) the step of comparing the binding amounts of the pituitary adenylate cyclase activating polypeptide to said protein, its partial peptide, or a salt thereof between the step (i) and the step (ii), and selecting said test compound when the binding amount in the step (ii) changes from the binding amount in the step (i).

[25] The screening method according to [23] or [24] above, wherein the pituitary adenylate cyclase activating polypeptide is PACAP-27 or PACAP-38.

[26] A kit for screening for a compound or its salt that changes the binding properties between a protein comprising the same or substantially the same amino acid sequence as the amino acid sequence of SEQ ID NO: 1, its partial peptide, or a salt thereof, and a pituitary adenylate cyclase activating polypeptide, which comprises (a) said protein, its partial peptide, or a salt thereof and (b) the pituitary adenylate cyclase activating polypeptide.

[27] A method of screening for an eicosanoid (e.g., leukotriene, prostaglandin) production inhibitor, a cytokine production inhibitor, a mast cell growth inhibitor or a mast cell activation inhibitor (including, e.g., a MAPK activation inhibitor, etc.), which comprises using (a) a protein comprising the same or substantially the same amino acid sequence as the amino acid sequence of SEQ ID NO: 1, its partial peptide, or a salt thereof and (b) a ligand capable of specifically binding to said protein.

[28] The screening method according to [27] above, wherein the ligand is substance P, cortistatin, pituitary adenylate cyclase activating polypeptide, vasoactive intestinal peptide or proadrenomedullin N-terminal 20 peptide.

[29] The screening method according to [27] above, wherein cell stimulating activities mediated by a protein comprising the same or substantially the same amino acid sequence as the amino acid sequence of SEQ ID NO: 1, its partial peptide, or a salt thereof, are assayed and used as an indicator.

[30] The screening method according to [27] above, wherein the inhibitor is a preventive/therapeutic agent for immune disorders, urologic disorders, digestive disorders or respiratory disorders.

[31] A kit for screening for an eicosanoid (e.g., leukotriene, prostaglandin) production inhibitor, a cytokine production inhibitor, a mast cell growth inhibitor or a mast cell activation inhibitor (including, e.g., a MAPK activation inhibitor, etc.), which comprises (a) a protein comprising the same or substantially the same amino acid sequence as the amino acid sequence of SEQ ID NO: 1, its partial peptide, or a salt thereof and (b) a ligand capable of specifically binding to said protein.

[32] An eicosanoid (e.g., leukotriene, prostaglandin) production inhibitor, cytokine production inhibitor, mast cell growth inhibitor or mast cell activation inhibitor (including, e.g., a MAPK activation inhibitor, etc.), which comprises an antagonist to a protein comprising the same or substantially the same amino acid sequence as the amino acid sequence of SEQ ID NO: 1, its partial peptide, or a salt thereof.

[33] An eicosanoid (e.g., leukotriene, prostaglandin) production inhibitor, cytokine production inhibitor, mast cell growth inhibitor or mast cell activation inhibitor (including, e.g., a MAPK activation inhibitor, etc.), which comprises an antibody against a protein comprising the same or substantially the same amino acid sequence as the amino acid sequence of SEQ ID NO: 1, its partial peptide, or a salt thereof.

[34] An eicosanoid (e.g., leukotriene, prostaglandin) production inhibitor, cytokine production inhibitor, mast cell growth inhibitor or mast cell activation inhibitor (including, e.g., a MAPK activation inhibitor, etc.), which comprises an antibody against an agonist for a protein comprising the same or substantially the same amino acid sequence as the amino acid sequence of SEQ ID NO: 1, its partial peptide, or a salt thereof.

[35] An eicosanoid (e.g., leukotriene, prostaglandin) production inhibitor, cytokine production inhibitor, mast cell growth inhibitor or mast cell activation inhibitor (including, e.g., a MAPK activation inhibitor, etc.), which comprises (i) a polynucleotide comprising an entire or part of a nucleotide sequence complementary or substantially complementary to a polynucleotide comprising a polynucleotide encoding a protein comprising the same or substantially the same amino acid sequence as the amino acid sequence of SEQ ID NO: 1, or its partial peptide or (ii) an siRNA or shRNA for a polynucleotide comprising a polynucleotide encoding said protein or its partial peptide.

[36] The inhibitor according to [32], [33], [34] or [35] above, wherein the inhibitor is a preventive/therapeutic agent for immune disorders, urologic disorders, digestive disorders or respiratory disorders.

[37] A method of inhibiting eicosanoid (e.g., leukotriene, prostaglandin) production, cytokine production, mast cell growth or mast cell activation, which comprises inhibiting the activities of a protein comprising the same or substantially the same amino acid sequence as the amino acid sequence of SEQ ID NO: 1, its partial peptide, or a salt thereof, or the activities of a ligand capable of specifically binding to said protein.

[38] A method of screening for an eicosanoid (e.g., leukotriene, prostaglandin) production inhibitor, cytokine production inhibitor, mast cell growth inhibitor or mast cell activation inhibitor (including, e.g., a MAPK activation inhibitor, etc.), which comprises using a mast cell and a ligand capable of specifically binding to a protein comprising the same or substantially the same amino acid sequence as the amino acid sequence of SEQ ID NO: 1.

[39] A kit for screening for an eicosanoid (e.g., leukotriene, prostaglandin) production inhibitor, cytokine production inhibitor, mast cell growth inhibitor or mast cell activation inhibitor (including, e.g., a MAPK activation inhibitor, etc.), which comprises a mast cell and a ligand capable of specifically binding to a protein comprising the same or substantially the same amino acid sequence as the amino acid sequence of SEQ ID NO: 1.

[40] A method of inhibiting eicosanoid (e.g., leukotriene, prostaglandin) production, cytokine production, mast cell growth or mast cell activation, which comprises administering to a mammal an effective dose of (i) an antagonist to a protein comprising the same or substantially the same amino acid sequence as the amino acid sequence of SEQ ID NO: 1, its partial peptide, or a salt thereof, (ii) an antibody against said protein, its partial peptide, or a salt thereof, or (iii) a polynucleotide comprising an entire or part of a nucleotide sequence complementary or substantially complementary to a polynucleotide comprising a polynucleotide encoding said protein or its partial peptide.

[41] Use of (i) an antagonist to a protein comprising the same or substantially the same amino acid sequence as the amino acid sequence of SEQ ID NO: 1, its partial peptide, or a salt thereof, (ii) an antibody against said protein, its partial peptide, or a salt thereof, or (iii) a polynucleotide comprising an entire or part of a nucleotide sequence complementary or substantially complementary to a polynucleotide comprising a polynucleotide encoding said protein or its partial peptide, for the manufacture of an eicosanoid (e.g., leukotriene, prostaglandin) production inhibitor, cytokine production inhibitor, mast cell growth inhibitor or mast cell activation inhibitor (including, e.g., a MAPK activation inhibitor, etc.).

## BEST MODE FOR CARRYING OUT THE INVENTION

Hereinafter, the term "protein comprising the same or substantially the same amino acid sequence as the amino acid sequence represented by SEQ ID NO: 1, its partial peptide, or a salt thereof' is sometimes referred to as "the receptor of the present invention." Likewise, the term "ligand capable of specifically binding to the receptor of the present invention" is sometimes referred to as "the ligand of the present invention".

The protein comprising the same or substantially the same amino acid sequence as the amino acid sequence represented by SEQ ID NO: 1 may be any protein derived from any cells of human and warm-blooded animals (e.g., guinea pigs, rats, mice, fowl, rabbits, swine, sheep, bovine, monkeys, etc.) (e.g., retinal cells, hepatocytes, splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.); or any tissues where such cells are present, such as brain or any of brain regions (e.g., retina, olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; or proteins derived from hemocyte type cells or their cultured cells (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, LAD 1, LAD 2,etc.); these proteins may also be synthetic proteins.

The amino acid sequence which is substantially the same amino acid sequence as the amino acid sequence represented by SEQ ID NO: 1 includes amino acid sequences having at least about 70% homology, preferably at least about 80% homology, more preferably at least about 90% homology, to the amino acid sequence represented by SEQ ID NO: 1; and so on.

Examples of the protein comprising substantially the same amino acid sequence as the amino acid sequence represented by SEQ ID NO: 1 include proteins having substantially the same amino acid sequence as the amino acid sequence represented by SEQ ID NO: 1 and having an activity which is substantially the same property as that of the protein comprising the amino acid sequence represented by SEQ ID NO: 1, etc.

The substantially equivalent activity includes, for example, a ligand binding activity, a signal transduction activity, a cell stimulating activity, etc. The term substantially equivalent is used to mean that the activities are the same in nature. Therefore, it is preferred that activities such as the ligand binding and signal transduction activities, cell stimulating activities, etc. are equivalent (e.g., about 0.01 to 100 times, preferably about 0.5 to 20 times, more preferably 0.5 to 2 times), but differences in degree such as a level of these activities, quantitative factors such as a molecular weight of the protein may be present and allowable.

The activities such as ligand binding, signal transduction and cell stimulating activities, etc. can be determined according to publicly known methods with some modifications.

The proteins of the present invention used include those having the following amino acid sequences: (i) the amino acid sequence represented by SEQ ID NO: 1, wherein at least 1 or 2 (e.g., approximately 1 to 100, preferably approximately 1 to 50, preferably approximately 1 to 30, more preferably approximately 1 to 10, and most preferably several (1 to 5)) amino acids are deleted; (ii) the amino acid sequence represented by SEQ ID NO: 1, to which at least 1 or 2 (e.g., approximately 1 to 100, preferably approximately 1 to 50, preferably approximately 1 to 30, more preferably approximately 1 to 10, and most preferably several (1 to 5)) amino acids are added; (iii) the amino acid sequence represented by SEQ ID NO: 1, in which at least 1 or 2 amino acids (e.g., approximately 1 to 100, preferably approximately 1 to 50, preferably approximately 1 to 30, more preferably approximately 1 to 10, and most preferably several (1 to 5)) amino acids are substituted by other amino acids; (iv) the amino acid sequence represented by SEQ ID NO: 1, in which at least 1 or 2 amino acids (e.g., approximately 1 to 100, preferably approximately 1 to 50, preferably approximately 1 to 30, more preferably approximately 1 to 10, and most preferably several (1 to 5)) amino acids are inserted; or (v) combination of these amino acid sequences; etc.

The partial peptide of the receptor of the present invention (hereinafter sometimes referred to as the partial peptide of the present invention) may be any partial peptide so long as it is a partial peptide which can be used for the methods of screening medicaments later described. Among the protein molecules of the present invention, for example, those having the site exposed to the outside of a cell membrane and retaining substantially the same ligand binding activity, etc. may be employed.

Specifically, the partial peptide of the receptor protein comprising the amino acid sequence represented by SEQ ID NO: 1 is a peptide containing the portion analyzed to be an extracellular domain (hydrophilic domain) in the hydrophobic plotting analysis. A peptide containing a hydrophobic domain in part can be used as well. In addition, the peptide may contain each domain separately or a plurality of domains together.

In the partial peptides of the present invention, preferred are peptides having at least 20, preferably at least 50, and more preferably at least 100 amino acids, in the amino acid sequence which constitutes the protein of the present invention.

Herein, the term "substantially equivalent activity" is intended to mean the same significance as defined above. The "substantially equivalent activity" can be assayed in the same way as described above.

The partial peptide of the present invention may contain amino acid sequences, (i) wherein at least 1 or 2 (preferably approximately 1 to 10, and more preferably several (1 to 5)) amino acids are deleted; (ii) wherein at least 1 or 2 (preferably approximately 1 to 20, more preferably approximately 1 to 10, and further more preferably several (1 to 5)) amino acids are added; or, (iii) wherein at least 1 or 2 (preferably approximately 1 to 10, and more preferably several (1 to 5)) amino acids are substituted by other amino acids.

Specific examples are partial peptides containing the amino acid sequences of 1st to 27th, 5 1 st to 69th, 93rd to 102nd, 126th to 144th, 168th to 185th, 209th to 223rd, 247th to 259th or 283rd to 330th in the amino acid sequence represented by SEQ ID NO: 1, and the like.

The receptor of the present invention and the partial peptide of the present invention are represented in accordance with the conventional way of describing peptides that is, the N-terminus (amino terminus) at the left hand and the C-terminus (carboxyl terminus) at the right hand. The C-terminus may be in any form of a carboxyl group (-COOH), a carboxylate (-COO-), an amide (-$CONH_2$) or an ester (-COOR).

As used herein, examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, $\alpha$-naphthyl, etc.; a $C_{7-14}$ aralkyl such as a phenyl-$C_{1-2}$ alkyl group, e.g., benzyl, phenethyl, etc.; an a-naphthyl-$C_{1-2}$ alkyl group such as $\alpha$-naphthylmethyl, etc.; pivaloyloxymethyl which is widely used as an ester for oral use and the like.

Where the receptor of the present invention and partial peptide of the present invention contain a carboxyl group (or a carboxylate) at a position other than the C-terminus, the carboxyl group may be amidated or esterified and such an amide or ester is also included within the receptor of the present invention or the partial peptide of the present invention. Examples of the ester group in this case may be the C-terminal esters described above, etc.

Furthermore, the receptor of the present invention and the partial peptide of the present invention include variants wherein the amino group at the N-terminal amino acid residues (e.g., methionine residue) is protected with a protecting group (e.g., a $C_{1-6}$ acyl group such as a $C_{1-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{1-6}$ alkanoyl group, e.g., formyl group, acetyl group, etc.), or conjugated proteins such as glycoproteins having sugar chains; etc.

As salts of the receptor of the present invention or the partial peptide of the present invention, salts with physiologically acceptable acids (e.g., inorganic acids or organic acids) or bases (e.g., alkali metal salts) may be employed, preferably in the form of physiologically acceptable acid addition salts. Examples of such salts include salts with inorganic acids (e.g., hydrochloric acid, phosphoric acid, hydrobromic acid, and sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The ligand of the present invention can be any ligand so long as the ligand specifically binds to the receptor of the present invention. The ligand includes, for example, those having a dissociation constant in binding to the protein or its salt of 10 $\mu$M or less, preferably not greater than 2 $\mu$M, more preferably not greater than 1 $\mu$M, much more preferably not greater than 200 nM, and most preferably not greater than 100 nM, and the like.

Examples of the ligand of the present invention used are substance P, PACAP (e.g., PACAP-27, PACAP-38, etc.), VIP, cortistatins (e.g., cortistatin-14, cortistatin-17, etc.), PAMP (e.g., PAMP-12, PAMP-20, etc.), somatostatins (e.g., somatostatin 3-14, somatostatin 3-10, somatostatin-14, somatostatin-28, somatostatin 7-14, D-Trp-somatostatin, cyclosomatostatin, somatostatin2-9, etc.), BAM (e.g., BAM13-22, BAM-22, etc.), $\alpha$-melanocyte-stimulating hormone (e.g., HS024-$\alpha$MSH(3-11)amide, etc.), neuropeptide FF, dynorphin A, oxytocin (e.g., (Ser4, Ile8)-oxytocin, etc.), vasopressin (e.g., [Arg8]-vasopressin, etc.), angiopeptin, etc., preferably, substance P, PACAP (e.g., PACAP-27, PACAP-38, etc.), VIP, cortistatins (e.g., cortistatin-17, etc.), PAMP (e.g., PAMP-12, PAMP-20, etc.) and the like.

The labeled ligand is also included in the ligand of the present invention.

The labeling agent includes radioisotopes (e.g., [$^{3}$H], [$^{125}$I], [$^{14}$C], [$^{32}$P], [$^{33}$P], [$^{35}$S], etc.), fluorescent substances (e.g., fluorescein, etc.), luminescent substances (e.g., luminol, etc.), enzymes (e.g., a peroxidase, etc.), a lanthanide, etc. Among others, radioisotopes are preferred, with particular preference of [$^{125}$I].

The labeled ligand preferably includes [Tyr8]-substance P, [TyrO]-cortistatin-14, PAMP, somatostatin, BAM, $\alpha$-melanocyte-stimulating hormone, neuropeptide FF, dynorphin A, oxytocin, vasopressin, and the like, which are labeled with radioisotope [$^{125}$I], etc.

Salts of the ligand are also included in the ligand of the present invention. As salts of the ligand, there are, for example, metal salts, ammonium salts, salts with organic bases, salts with inorganic acids, salts with organic acids, salts with basic or acidic amino acids, etc. Preferred examples of the metal salts include alkali metal salts such as sodium salts, potassium salts, etc.; alkaline earth meal salts such as calcium salts, magnesium salts, barium salts, etc.; aluminum salts, etc. Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc. Preferred examples of the salts with inorganic acids include salts with hydrochloric acid, hydrobromic acid, nitric acid, sulfuric acid, phosphoric acid, etc. Preferred examples of the salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc. Preferred examples of the salts with basic amino acids include salts with arginine, lysine, ornithine, etc., and preferred examples of the salts with acidic amino acids include salts with aspartic acid, glutamic acid, etc.

Of these salts, pharmacologically acceptable salts are preferred. For example, where the compounds contain acidic functional groups within compounds, examples include inorganic salts such as alkali metal salts (e.g., sodium salts, potassium salts, etc.), alkaline earth metal salts (e.g., calcium salts, magnesium salts, barium salts, etc.), ammonium salts, etc., and when the compounds contain basic functional groups therein, examples include salts with inorganic acids such as hydrobromic acid, nitric acid, sulfuric acid, phosphoric acid, etc., salts with organic acids such as acetic acid, phthalic acid, fumaric acid, oxalic acid, tartaric acid, maleic acid, citric acid, succinic acid, methanesulfonic acid, p-toluenesulfonic acid, etc.

The receptor of the present invention and the partial peptide of the present invention can be manufactured from the aforesaid human or warm-blooded animal cells or tissues by publicly known methods for purification of polypeptides, or can also be manufactured by culturing transformants transformed by DNAs encoding the polypeptides. In addition, they can also be manufactured by modifications of peptide synthesis. For example, the receptor and partial peptide can also be manufactured by the methods described in, e.g., Genomics, 56, 12-21, 1999, Biochim. Biophys. Acta, 1446, 57-70, 1999, etc., or by these methods with modifications.

Where the receptor and partial peptide are manufactured from human or mammalian tissues or cells, human or mammalian tissues or cells are homogenized, then extracted with an acid or the like, and the extract is isolated and purified by a combination of chromatography techniques such as reverse phase chromatography, ion exchange chromatography, and the like.

To synthesize the receptor of the present invention or partial peptides or salts thereof, commercially available resins that are used for polypeptide synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids in which $\alpha$-amino groups and functional groups on the side chains are appropriately protected are condensed on the resin in the order of the sequence of the objective polypeptide according to various condensation methods publicly known in the art. At the end of the reaction, the polypeptide is cut out from the resin and at the same time, the protecting groups are removed. Then, intramolecular disulfide bond-forming reaction is performed in a highly diluted solution to obtain the objective polypeptide, receptor, partial peptide or its amides.

For condensation of the protected amino acids described above, a variety of activation reagents for polypeptide synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents known to be usable for polypeptide condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to polypeptide binding reactions and is usually selected in the range of approximately -20°C to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower ($C_{1-6}$) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups used in the starting compounds include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)], etc. As the activated amino acids, in which the amino groups are activated in the starting material, for example, the corresponding phosphoric amides are employed.

To eliminate (split off) the protecting groups, there are used catalytic reduction under hydrogen gas flow in the presence of a catalyst such as Pd-black, Pd-carbon, etc.; an acid treatment with anhydrous hydrogen fluoride, methanesulfonic acid, trifluoromethane-sulfonic acid or trifluoroacetic acid, or a mixture solution of these acids; a treatment with a base such as diisopropylethylamine, triethylamine, piperidine, piperazine, etc.; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of the functional groups that should not be involved in the reaction of the starting materials, protecting groups, elimination of the protecting groups, activation of functional groups involved in the reaction, or the like may be appropriately chosen from publicly known groups and publicly known means.

In another method for obtaining the receptor or partial peptide of the present invention, for example, the α-carboxyl group of the carboxy terminal amino acid is first protected by amidation; the peptide (polypeptide) chain is then extended from the amino group side to a desired length. Thereafter, a polypeptide in which only the protecting group of the N-terminal α-amino group in the peptide chain has been eliminated from the polypeptide and a polypeptide in which only the protecting group of the C-terminal carboxyl group has been eliminated are prepared. The two polypeptides are condensed in a mixture of the solvents described above. The details of the condensation reaction are the same as described above. After the protected polypeptide obtained by the condensation is purified, all the protecting groups are eliminated by the method described above to give the desired crude polypeptide. This crude polypeptide is purified by various known purification means. Lyophilization of the major fraction gives the amide of the desired receptor or its partial peptide.

To prepare the esterified receptor of the present invention or partial peptides or salts thereof, for example, the α-carboxyl group of the carboxy terminal amino acid is condensed with a desired alcohol to prepare the amino acid ester, which is followed by procedures similar to the preparation of the amidated receptor or partial peptide above to give the desired esterified receptor or partial peptide.

The receptor or partial peptide of the present invention can be prepared by publicly known methods for peptide synthesis, or the partial peptide of the receptor can be prepared by cleaving the receptor with an appropriate peptidase. For the methods for peptide synthesis, for example, either solid phase synthesis or liquid phase synthesis may be used. That is, the partial peptide or amino acids that can constitute the receptor or partial peptide of the present invention are condensed with the remaining part. Where the product contains protecting groups, these protecting groups are removed to give the desired peptide. Publicly known methods for condensation and elimination of the protecting groups are described in (i) - (v) below.

(i) M. Bodanszky & M.A. Ondetti: Peptide Synthesis, Interscience Publishers, New York (1966)
(ii) Schroeder & Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975)
(iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
(v) Haruaki Yajima, ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the receptor or partial peptide of the present invention. When the receptor or partial peptide obtained by the above methods is in a free form, the receptor or partial peptide can be converted into an appropriate salt by a publicly known method or its modification; conversely when the receptor or partial peptide is obtained in a salt form, it can be converted into a free form or other different salt form by a publicly known method or its modifications.

The polynucleotide encoding the receptor or partial peptide of the present invention may be any polynucleotide so long as it contains the nucleotide sequence encoding the receptor or partial peptide of the present invention described above. Preferably, the polynucleotide is a DNA. The DNA may also be any one of genomic DNA, genomic DNA library, cDNA derived from the cells or tissues described above, cDNA library derived from the cells or tissues described above and synthetic DNA.

The vector used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

The DNA encoding the receptor of the present invention may be any one of, for example, a DNA containing the nucleotide sequence represented by SEQ ID NO: 2, or any DNA containing a nucleotide sequence hybridizable to the nucleotide sequence represented by SEQ ID NO: 2 under high stringent conditions and encoding the receptor which has the properties of substantially equivalent to those of the protein comprising the amino acid sequence represented by SEQ ID NO: 1.

Examples of the DNA that is hybridizable to the nucleotide sequence represented by SEQ ID NO: 2 under high stringent conditions include DNAs having at least about 70% homology, preferably at least about 80% homology, more preferably at least about 90% homology, much more preferably at least about 95% homology, to the nucleotide sequence represented by SEQ ID NO: 2; and the like.

The hybridization can be carried out by publicly known methods or by a modification thereof, for example, according to the method described in Molecular Cloning, 2nd. (J. Sambrook et al., Cold Spring Harbor Lab. Press, 1989). A commercially available library can also be used according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, as the DNA encoding the receptor containing the amino acid sequence represented by SEQ ID NO: 1, there may be employed a DNA containing the nucleotide sequence represented by SEQ ID NO: 2, etc.

The DNA encoding the partial peptide of the present invention may be any DNA so long as it contains the nucleotide sequence encoding the partial peptide of the receptor of the present invention. The DNA may also be any one of genomic DNA, genomic DNA library, cDNA derived from the cells or tissues described above, cDNA library derived from the cells or tissues described above and synthetic DNA. Specifically, there are used a DNA having a part of the nucleotide sequence of a DNA having the nucleotide sequence represented by SEQ ID NO: 2 or a DNA having a part of the nucleotide sequence of a DNA having the nucleotide sequence represented by SEQ ID NO: 2 under high stringent conditions and containing a part of DNA encoding the receptor having the activities substantially equivalent to those of the protein comprising the amino acid sequence represented by SEQ ID NO: 1, and so on.

The DNA hybridizable to the nucleotide sequence represented by SEQ ID NO: 2 has the same significance as described above.

Methods for the hybridization and the high stringent conditions that can be used are the same as those described above.

The polynucleotide (e.g., DNA) encoding the receptor or partial peptide of the present invention may be labeled by methods public known. The labeled agents include radioisotopes, fluorescent substances (e.g., fluorescein, etc.), luminescent substances, enzymes, biotin, lanthanides, or the like.

For cloning of DNAs that completely encode the receptor or partial peptide of the present invention, the DNA can be either amplified by PCR using synthetic DNA primers containing a part of the nucleotide sequence of the receptor or partial peptide of the present invention, or the DNA inserted into an appropriate vector can be selected by hybridization with a labeled DNA fragment or synthetic DNA that encodes a part or entire region of the receptor or partial peptide of the present invention. The hybridization can be carried out, for example, according to the method described in Molecular Cloning, 2nd (J. Sambrook et al., Cold Spring Harbor Lab. Press, 1989). Where the hybridization is carried out using commercially available library, the procedures may be conducted in accordance with the protocol described in the attached instructions.

Conversion of the nucleotide sequence of DNA can be effected by publicly known methods such as the ODA-LA PCR method, the Gapped duplex method, the Kunkel method, etc., or its modification, using a publicly known kit, e.g., Mutant™-super Express Km (Takara Shuzo Co., Ltd.) or Mutan™-K (Takara Shuzo Co., Ltd.), etc.

The cloned DNA encoding the receptor can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the receptor or partial peptide of the present invention can be manufactured, for example, by (a) excising the desired DNA fragment from the DNA encoding the receptor or partial peptide of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNA I/Neo, etc.

The promoter used in the present invention may be any promoter if it matches well with a host to be used for gene expression. In the case of using animal cells as the host, examples of the promoter include SRα promoter, SV40 promoter, HIV-LTR promoter, CMV promoter, HSV-TK promoter, etc.

Among them, it is preferred to use CMV (cytomegalovirus) promoter, SRα promoter, etc. Where the host is bacteria of the genus Escherichia, preferred examples of the promoter include trp promoter, lac promoter, recA promoter, $\lambda P_L$ promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^r$, G418 resistance), etc. In particular, when dhfr gene is used as the selection marker using dhfr gene-deficient Chinese hamster cells, selection can also be made on a thymidine free medium.

If necessary, a signal sequence that matches with a host is added to the N-terminus of the receptor of the present invention. Examples of the signal sequence that can be used are PhoA-signal sequence, OmpA signal sequence, etc. when bacteria of the genus Escherichia is used as the host; α-amylase signal sequence, subtilisin signal sequence, etc. when bacteria of the genus Bacillus is used as the host; MFα signal sequence, SUC2 signal sequence, etc. when yeast is used as the host; and insulin signal sequence, α-interferon signal sequence, antibody molecule signal sequence, etc. when animal cells are used as the host, respectively.

Using the vector containing the DNA encoding the receptor or partial peptide of the present invention thus constructed, transformants can be manufactured.

Examples of the host, which may be employed, are bacteria belonging to the genus Escherichia, bacteria belonging to the genus Bacillus, yeast, insect cells, insects, animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, $AH22R^-$, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977)), etc.

As the insect, for example, a larva of Bombyx mori can be used [Maeda et al., Nature, 315, 592 (1985)].

Examples of animal cells include monkey cell COS-7, Vero, Chinese hamster cell CHO (hereinafter referred to as CHO cell), dhfr gene-deficient Chinese hamster cell CHO (hereinafter simply referred to as CHO ($dhfr^-$) cell), mouse L cell, mouse AtT-20, mouse myeloma cell, rat GH3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55 (1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors containing the DNAs encoding the receptor or partial peptide can be obtained.

Where the host is bacteria belonging to the genus Escherichia or the genus Bacillus, the transformant can be appropriately cultured in a liquid medium, which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.

As described above, the receptor or partial peptide of the present invention can be produced within the transformant, in the cell membrane of the transformant, or outside of the transformant.

The receptor or partial peptide of the present invention can be separated and purified from the culture described above by the following procedures.

When the receptor or partial peptide of the present invention is extracted from the bacteria or cells, the bacteria or cell is collected after culturing by a publicly known method and suspended in an appropriate buffer. The bacteria or cell is then disrupted by publicly known methods such as ultrasonication, a treatment with lysozyme and/or freeze-thaw cycling, followed by centrifugation, filtration, etc to produce crude extract of the polypeptide. The buffer may contain a protein modifier such as urea or guanidine hydrochloride, or a surfactant such as Triton X-100™, etc. When the polypeptide is secreted in the culture broth, the supernatant can be separated, after completion of the cultivation, from the bacteria or cell to collect the supernatant by a publicly known method.

The receptor or partial peptide contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the receptor or partial peptide thus obtained is in a free form, the receptor or partial peptide can be converted into the salt by publicly known methods or modifications thereof. On the other hand, when the receptor or partial peptide is obtained in the form of a salt, it can be converted into the free form or in the form of a different salt by publicly known methods or modifications thereof.

The receptor or partial peptide produced by the recombinant can be treated, prior to or after the purification, with an appropriate protein-modifying enzyme so that the receptor or partial peptide can be appropriately modified to partially remove the polypeptide. Examples of the protein-modifying enzyme include trypsin, chymotrypsin, arginyl endopeptidase, protein kinase, glycosidase and the like.

The ligand capable of specifically binding to the receptor of the present invention can be used as it is when commercially available, or can be extracted or manufactured by publicly known methods or its modifications.

The antibodies against the protein comprising the same or substantially the same amino acid sequence as the amino acid sequence represented by SEQ ID NO: 1, its partial peptide or a salt thereof (hereinafter sometimes collectively referred to as the antibody of the present invention) may be either polyclonal antibodies or monoclonal antibodies, as long as they are antibodies capable of recognizing antibodies against the receptor of the present invention. The antibodies against the receptor of the present invention include the antibodies that inactivate the signal transduction of the receptor, antibodies that activate the signal transduction of the receptor, etc.

The antibodies against the receptor of the present invention can be produced by a publicly known method of producing an antibody or antiserum, using the receptor of the present invention as an antigen.

[Preparation of monoclonal antibody]

(a) Preparation of monoclonal antibody-producing cells

The receptor of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every about 2 to about 6 weeks and about 2 to about 10 times in total. Examples of the applicable warm-blooded animals are monkeys, rabbits, dogs, guinea pigs, mice, rats, sheep, goats and fowl, with the use of mice and rats being preferred.

In the preparation of monoclonal antibody-producing cells, a warm-blooded animal, e.g., mouse, immunized with an antigen wherein the antibody titer is noted is selected, then spleen or lymph node is collected after 2 to 5 days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled polypeptide, which will be described later, with the antiserum followed by assaying the binding activity of the labeling agent bound to the antibody. The fusion may be carried out, for example, by the known method by Koehler and Milstein [Nature, 256, 495, (1975)]. Examples of the fusion promoter are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g., a microplate) adsorbed with the polypeptide (protein) as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the polypeptide labeled with a radioactive substance or an enzyme and detecting the monoclonal antibody bound to the solid phase, or the like.

The monoclonal antibody can be screened according to publicly known methods or their modifications. In general, the screening can be performed in a medium for animal cells supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20 to 40°C, preferably at 37°C, for about 5 days to about 3 weeks, preferably 1 to 2 weeks, normally in 5% $CO_2$. The antibody titer of the culture supernatant of a hybridoma can be determined as in the assay for the antibody titer in antisera described above.

(b) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.]

[Preparation of polyclonal antibody]

The polyclonal antibody of the present invention can be manufactured by publicly known methods or modifications thereof. For example, a warm-blooded animal is immunized with an immunogen (polypeptide antigen) per se, or a complex of immunogen and a carrier protein is formed and the animal is immunized with the complex in a manner similar to the method described above for the manufacture of monoclonal antibodies. The product containing the antibody against the receptor of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

The polynucleotide (e.g., DNA) containing a complementary or substantially complementary nucleotide sequence to the polynucleotide (e.g., DNA) or a part thereof encoding the protein comprising the same or substantially the same amino acid sequences as the amino acid sequence represented by SEQ ID NO: 1, as its partial peptide or as its salt can be any polynucleotide (antisense polynucleotide), so long as it contains a nucleotide sequence complementary or substantially complementary to the polynucleotide, or a part of the nucleotide sequence and capable of suppressing expression of the polynucleotide.

Specific examples of the polynucleotide include antisense DNAs (hereinafter these DNAs are sometimes simply referred to as the antisense DNA) having a nucleotide sequence complementary or substantially complementary to polynucleotides (e.g., DNAs) encoding the receptor of the present invention (hereinafter these DNAs are sometimes briefly referred to as the DNA of the present invention) or a part of the nucleotide sequence, and can be any antisense DNA, so long as it contains the complementary or substantially complementary nucleotide sequence to the DNA of the present invention, or a part of the nucleotide sequence and capable of suppressing expression of the DNA.

The nucleotide sequence substantially complementary to the DNA of the present invention may include, for example, a nucleotide sequence having at least about 70% homology, preferably at least about 80% homology, more preferably at least about 90% homology and most preferably at least about 95% homology, to the entire nucleotide sequence or to its partial nucleotide sequence (i.e., complementary strand to the DNA of the present invention), and the like. Especially in the entire nucleotide sequence of the complementary strand to the DNA of the present invention, preferred are an antisense DNA having at least about 70% homology, preferably at least about 80% homology, more preferably at least about 90% homology and most preferably at least about 95% homology, to the complementary strand of the nucleotide sequence which encodes the N-terminal region of the receptor of the present invention (e.g., the nucleotide sequence around the initiation codon). These antisense DNAs can be prepared using publicly known DNA synthesizer, etc.

Specific examples include an antisense polynucleotide containing the entire or part of a nucleotide sequence complementary or substantially complementary to a nucleotide sequence of DNA comprising the nucleotide sequence represented by SEQ ID NO: 2, preferably an antisense polynucleotide containing the entire or part of a nucleotide sequence complementary to a nucleotide sequence of DNA comprising the nucleotide sequence represented by SEQ ID NO: 2, etc.

The antisense polynucleotide is generally constituted with nucleotides of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. These antisense polynucleotides may be synthesized using a publicly known DNA synthesizer, etc.

According to the present invention, the antisense polynucleotide capable of inhibiting the replication or expression of a gene for the receptor of the present invention (nucleic acid) can be designed and synthesized based on the nucleotide sequence information of cloned or identified protein-encoding DNA. Such a polynucleotide (nucleic acid) is hybridizable to RNA of a gene for the receptor of the present invention to inhibit the synthesis or function of said RNA or is capable of modulating and/or controlling the expression of a gene for the receptor of the present invention via interaction with RNA associated with the receptor of the present invention. Polynucleotides complementary to the selected sequences of RNA associated with the receptor of the present invention and polynucleotides specifically hybridizable to RNA associated with the receptor of the present invention are useful in modulating and/or controlling the in vivo and in vitro expression of the receptor gene of the present invention, and are useful for the treatment or diagnosis of diseases, etc. The term "corresponding" is used to mean homologous to or complementary to a particular sequence of the nucleotide including the gene, nucleotide sequence or nucleic acid. The term "corresponding" between nucleotides, nucleotide sequences or nucleic acids and peptides (proteins) usually refer to amino acids of a peptide (protein) under the order derived from the sequence of nucleotides (nucleic acids) or their complements. In the protein genes, the 5' end hairpin loop, 5' end 6-base-pair repeats, 5' end untranslated region, protein translation initiation codon, protein coding region, ORF translation termination codon, 3' end untranslated region, 3' end palindrome region, and 3' end hairpin loop, may be selected as preferred target regions, though any other region may be selected as a target in the protein genes.

The relationship between the targeted nucleic acids and the polynucleotides complementary to at least a part of the target region, specifically the relationship between the target nucleic acids and the polynucleotides hybridizable to the target region, can be denoted to be "antisense." Examples of the antisense polynucleotides include polynucleotides containing 2-deoxy-D-ribose, polynucleotides containing D-ribose, any other type of polynucleotides which are N-glycosides of a purine or pyrimidine base, or other polymers containing non-nucleotide backbones (e.g., commercially available protein nucleic acids and synthetic sequence-specific nucleic acid polymers) or other polymers containing nonstandard linkages (provided that the polymers contain nucleotides having such a configuration that allows nucleotide pairing or nucleotide stacking, as is found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleotide of the present invention can be modified preferably based on the following design, that is, by increasing the intracellular stability of the antisense nucleotide, enhancing the cell permeability of the antisense nucleotide, increasing the affinity of the nucleic acid to the targeted sense strand to a higher level, or minimizing the toxicity, if any, of the antisense nucleotide.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense polynucleotide of the present invention may contain altered or modified sugars, bases or linkages. The antisense polynucleotide may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleotide can be examined using the transformant of the present invention, the gene expression system of the present invention in vivo and in vitro, or the translation system for the receptor of the present invention in vivo and in vitro. The nucleic acid can be applied to cells by a variety of publicly known methods.

Hereinafter, (i) the receptor of the present invention, (ii) the polynucleotide encoding the receptor of the present invention (the polynucleotide of the present invention), (iii) the antibody against the receptor of the present invention (the antibody of the present invention) (iv) the antisense polynucleotide of the receptor of the present invention (e.g., the antisense DNA of the present invention), (v) the ligand capable of specifically binding to the receptor of the present invention (the ligand of the present invention), etc. are described in terms of their applications.

[1] Screening of drug candidate compounds having the mast cell degranulation-inhibitory action, eicosanoid production inhibitory action, cytokine production inhibitory action, mast cell growth inhibitory action, mast cell activation inhibitory action, etc.

The ligand of the present invention has the mast cell degranulation-promoting action, eicosanoid (e.g., leukotriene, prostaglandin, etc.) production promoting action, cytokine (e.g., TNF-$\alpha$, IL-4, IL-5, IL-6, IL-8, IL-13, TGF-$\beta$, etc.) production promoting action, mast cell growth promoting action, mast cell activating (promoting) action, and so on.

The compound or its salt that inhibits the function/activity (e.g., the mast cell degranulation-promoting action, eicosanoid production promoting action, cytokine production promoting action, mast cell growth promoting action, mast cell activation promoting action, etc.) of the ligand of the present invention or the receptor of the present invention is useful as a mast cell degranulation-inhibitor, eicosanoid production inhibitor, cytokine production inhibitor, mast cell growth inhibitor, mast cell activation inhibitor (including, e.g., a MAPK activation inhibitor, etc.), or the like, and can be used as an agent for preventing/treating, for example, immune disorders [e.g., inflammatory disorders (pituitary tumor, thyroiditis, peritonitis, Crohn's disease, ulcerative colitis, erythema nodosum, chronic articular rheumatism, systemic lupus erythematosus, etc.), allergy (e.g., allergic conjunctivitis, allergic rhinitis, pollinosis, metal allergy, etc.), asthma, secretory otitis media, Meniere's disease, contact dermatitis, anaphylaxis, urticaria, myasthenia gravis, glomerulonephritis, Sjögren's syndrome, Basedow's disease, insulin resistant diabetes, atopic dermatitis, leukocyte abnormality, etc.], urinary tract disorders (e.g., renal tubulointerstitial injury (fibril formation), interstitial cystitis, allergic cystitis, etc.), digestive disorders [e.g., irritable bowel syndrome, chronic liver disease, food allergy, allergic enteritis, milk protein-induced proctitis, digestive ulcer (e.g., gastric ulcer, duodenal ulcer, marginal ulcer, Zollinger-Ellison syndrome, etc.), gastritis, reflux esophagitis, NUD (non-ulcer dyspepsia), gastric MALT lymphoma, nonsteroidal anti-inflammatory drug-induced ulcer, acid indigestion, postoperative stress-induced hyperacidity and ulcer, etc.], respiratory disorders [e.g., chronic obstructive pulmonary disease (e.g., chronic bronchitis, pulmonary emphysema), diffuse panbronchiolitis, cystic fibrosis, hypersensitivity penumonitis, idiopathic interstitial pneumonia, pulmonary fibrosis, etc.], circulatory disorders (e.g., arteriosclerosis, acute coronary syndrome, atherosclerotic aortic aneurysm, cardiac anaphylaxis, heart failure, myocardial infarction, angina pectoris, arrhythmia, deep vein thrombosis, restenosis after PTCA, etc.), ophthalmic disorders (e.g., pterygium, vernal conjunctivitis, dry eye, etc.), cancer (e.g., papillary thyroid carcinoma, non-small-cell lung cancer, endometrial cancer, cervical cancer, gastric cancer, pancreatic cancer, lung cancer, renal cancer, hepatic cancer, ovarian cancer, prostate cancer, bladder cancer, breast cancer, colon cancer, rectal cancer, Kaposi's sarcoma, mastocytoma, etc.), cerebral infarction, hyperlipemia, acute renal failure, diabetes mellitus, obesity, edema, granuloma, atopic myelitis, neurofibroma, nasal mucosa hypersensitivity, Hodgkin's disease, endometrial hyperplasia, etc., or the like.

The mast cell degranulation inhibitor preferably includes agents for preventing/treating immune disorders, urinary tract disorders, digestive disorders, circulatory diseases, etc.

The eicosanoid production inhibitor preferably includes agents for preventing/treating immune disorders, etc.

The cytokine production inhibitor preferably includes agents for preventing/treating immune disorders, etc.

The mast cell growth inhibitor preferably includes agents for preventing/treating urinary tract disorders, etc.

The mast cell activation inhibitor (including, e.g., a MAP kinase activation inhibitor, etc.) preferably includes agents for preventing/treating digestive disorders, etc.

By using the receptor of the present invention or by using the ligand-receptor assay system using the expression system of the receptor of the present invention in its recombinant form, compounds (e.g., peptides, proteins, antibodies, non-peptide compounds, synthetic compounds, fermentation products, cell extracts, plant extracts, animal tissue extracts, blood plasma, etc.) or their salts that change the binding properties of the receptor of the present invention to the ligand of the present invention can be efficiently screened.

The compounds or salts thereof include (i) compounds having the cell stimulating activities (for example, the activities that promote arachidonic acid release, acetylcholine release, intracellular $Ca^{2+}$ release, intracellular cAMP production, intracellular cAMP production suppression, intracellular cGMP production, inositol phosphate production, change in cell membrane potential, phosphorylation of intracellular proteins, activation of c-fos, pH reduction, GTPγS binding activity, activation of cAMP-dependent protein kinase, activation of cGMP-dependent protein kinase, activation of phospholipid-dependent protein kinase, activation of mitogen-activated protein kinase (MAP kinase) (e.g., ERK1/2 (p42/44 MAP kinase), p38 MAPK, JNK/SAPK, ERK5, etc.), or the like) mediated by the receptor of the present invention (agonists), (ii) compounds that do not have these cell-stimulating activities (antagonists), (iii) compounds that promote the binding properties of the receptor of the present invention to the ligand of the present invention, (iv) compounds that inhibit the binding properties of the receptor of the present invention to the ligand of the present invention, and the like.

Specifically, comparison is made between (i) when the ligand of the present invention is brought in contact with the receptor of the present invention and (ii) when the ligand of the present invention and a test compound are brought in contact with the receptor of the present invention. The comparison is made by assaying, for example, the binding amount of the ligand of the present invention to the receptor of the present invention, the cell stimulating activities, or the like.

Specific examples of the screening method of the present invention include:

(a) a method of screening a compound or its salt that changes the binding properties of the ligand of the present invention to the receptor of the present invention, which comprises measuring the binding amounts of the ligand of the present invention to the receptor of the present invention when the ligand of the present invention is brought in contact with the receptor of the present invention and when the ligand of the present invention and a test compound are brought in contact with the receptor of the present invention; and comparing the binding amounts;

(b) a method of screening a compound or its salt that changes the binding amounts of the ligand of the present invention to the receptor of the present invention, which comprises measuring the binding amounts of the ligand of the present invention to a cell containing the receptor of the present invention or a membrane fraction of the cell, when the ligand of the present invention is brought in contact with the cell containing the receptor of the present invention or the membrane fraction of the cell and when the ligand of the present invention and a test compound are brought in contact with the cell or its cell membrane fraction, and comparing the binding amounts; and,

(c) the screening method according to (b) above, wherein the receptor of the present invention is the receptor of the present invention expressed on a cell membrane by culturing a transformant bearing a DNA encoding the receptor of the present invention;

(d) the receptor-binding assay system such as the screening method described in (a) to (c) above, wherein the ligand of the present invention is a labeled ligand;

(e) a method of screening a compound or its salt that changes the binding properties of the ligand of the present invention to the receptor of the present invention, which comprises assaying the cell stimulating activities mediated by the receptor of the present invention, when the ligand of the present invention is brought in contact with the receptor of the present invention and when the ligand of the present invention and a test compound are brought in contact with the receptor of the present invention; and comparing the activities;

(f) a method of screening a compound or its salt that changes the binding properties of the ligand of the present invention to the receptor of the present invention, which comprises assaying the cell stimulating activities mediated by the receptor of the present invention, when the ligand of the present invention is brought in contact with a cell containing the receptor of the present invention or a membrane fraction of the cell, and when the ligand of the present invention and a test compound are brought in contact with the cell containing the receptor of the present invention or its cell membrane fraction; and comparing the activities; and,

(g) the screening method according to (f) above, wherein the receptor of the present invention is the receptor of the present invention expressed on a cell membrane by culturing a transformant bearing a DNA encoding the receptor of the present invention; and so on.

The screening method of the present invention is specifically described below.

As the receptor of the present invention, membrane fractions from human or warm-blooded animal organs are preferably employed. However, it is very difficult to obtain human-derived organs among others, and the receptor of the present invention, etc. expressed abundantly by use of recombinants are suitable for use in the screening.

To produce the receptor of the present invention, the aforesaid methods for producing the receptor of the present invention, etc. are applied.

When cells containing the receptor of the present invention or membrane fractions of these cells are employed in the screening methods of the present invention, these cells or membrane fractions may be prepared according to the procedures later described.

Where cells containing the receptor of the present invention are employed, the cells may be fixed using glutaraldehyde, formalin, etc. The fixation can be made by publicly known methods.

The cells containing the receptor of the present invention refer to host cells where the receptor of the present invention is expressed, and such host cells include Escherichia coli, Bacillus subtilis, yeast, insect cells, animal cells, etc. described above. The host cells can be prepared in a manner similar to the method described above.

The cell membrane fraction is used to mean a fraction abundant in cell membrane obtained by cell disruption and subsequent fractionation by publicly known methods. The cell disruption methods include cell squashing using a Potter-Elvehjem homogenizer, disruption using a Waring blender or Polytron (manufactured by Kinematica Inc.), disruption by ultrasonication, disruption by cell spraying through thin nozzles under an increased pressure using a French press, and the like. Cell membrane fractionation is effected mainly by fractionation using a centrifugal force, such as fractional centrifugation, density gradient centrifugation, etc. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor of the present invention expressed and membrane components such as cell-derived phospholipids, membrane proteins, etc.

The amount of the receptor of the present invention in the cells or cell membrane fractions containing the receptor of the present invention is preferably $10^3$ to $10^8$ molecules, more preferably $10^5$ to $10^7$ molecules, per cell. As the amount of expression increases, the ligand binding activity per unit of the membrane fraction (specific activity) increases so that not only the highly sensitive screening system can be constructed but also large quantities of samples can be assayed on the same lot.

To perform the screening methods such as the receptor-binding assay system, the cell stimulating assay system and the like, for example, a fraction of the receptor of the present invention and a labeled form of the ligand of the present invention (e.g., a labeled form of the ligand of the present invention), etc. are employed. For the fraction of the receptor of the present invention, a fraction from naturally occurring type of the receptor of the present invention or a fraction from recombinant type of the receptor of the present invention having an activity equivalent thereto, or the like, are desirable. Herein, the equivalent activity is used to mean an equivalent ligand binding activity, etc. As the labeled ligands, there may be used ligands labeled with, e.g., radioisotope (e.g., [$^3$H], [$^{125}$I], [$^{14}$C], [$^{32}$P], [$^{33}$P], [$^{35}$S], etc.), fluorescent substances (e.g., fluorescein, etc.), luminescent substances (e.g., luminol, etc.), enzymes (e.g., peroxidase, etc.), lanthanide, or the like.

Specifically, screening of the compound that changes the binding properties of the ligand of the present invention to the receptor of the present invention can be performed by the following procedures. First, a receptor preparation is prepared by suspending cells containing the receptor of the present invention or their membrane fractions in a buffer appropriate for screening. Any buffer can be used so long as it does not interfere with ligand-receptor binding, such buffer including a phosphate buffer, a Tris-HCl buffer, etc. having pH of 4 to 10 (desirably pH of 6 to 8). For the purpose of minimizing non-specific binding, a surfactant such as CHAPS, Tween-80™ (manufactured by Kao-Atlas Inc.), digitonin, deoxycholate, etc. may be added to the buffer. Further for the purpose of suppressing degradation of the receptor of the present invention by a protease, a protease inhibitor such as PMSF, leupeptin, E-64 (manufactured by Peptide Institute, Inc.), pepstatin, etc. may also be added. A given quantity (5,000 cpm to 500,000 cpm) of a labeled form of the ligand of the present invention is added to 0.01 ml to 10 ml of the receptor solution, and at the same time, $10^{-10}$ to $10^{-7}$ $\mu$M of a test compound is allowed to be co-present. To determine the amount of non-specific binding (NSB), a reaction tube containing a large excess of the ligand of the present invention in an unlabeled form is also provided. The reaction is carried out at 0°C to 50°C, preferably about 4°C to 37°C for 20 minutes to 24 hours, preferably 30 minutes to 3 hours. After completion of the reaction, the reaction mixture is filtrated through glass fiber filter paper, etc. and washed with an appropriate volume of the same buffer. The residual radioactivity in the glass fiber filter paper is then measured by means of a liquid scintillation counter or a $\gamma$-counter. When the nonspecific binding (NSB) is subtracted from the count ($B_0$) when any antagonizing compound is absent and the thus obtained count ($B_0$ - NSB) is made 100%, a test compound having the specific binding (B - NSB) of, e.g., 50% or less, can be selected as a compound capable of competitive inhibition.

In addition, the compounds which bind to the receptor of the present invention can also be screened by utilizing the surface plasmon sensor technique.

Specifically, the receptor of the present invention is immobilized on the sensor chip surface of Biacore 3000 (Biacore, Inc.), and then the solution of a test compound in phosphate-buffered saline (PBS), etc. is applied onto the chip surface. By monitoring the changes on the surface plasmon, the test compound bound to the receptor of the present invention is screened. For example, the test compound, which gives the measurement data of 5 resonance units or more in the changes at the surface plasmon, is screened as a substance having the binding properties to the receptor of the present invention.

To perform the screening methods of the cell stimulating assay system described above, the cell-stimulating activities mediated by the receptor of the present invention (e.g., the activity that promotes or suppresses arachidonic acid release, acetylcholine release, intracellular $Ca^{2+}$ release, intracellular cAMP production, intracellular cAMP production suppression intracellular cGMP production, inositol phosphate production, change in cell membrane potential, phosphorylation of intracellular proteins, activation of c-fos, pH reduction, GTP$\gamma$S binding activity, activation of cAMP-dependent protein kinase, activation of cGMP-dependent protein kinase, activation of phospholipid-dependent protein kinase, activation of mitogen-activated protein kinase (MAP kinase) (e.g., ERK1/2 (p42/44 MAP kinase), p38 MAPK, JNK/SAPK, ERK5, etc.), or the like) can be assayed by publicly known methods, or using assay kits commercially available. Specifically, the cells containing the receptor of the present invention are first cultured on a multi-well plate, etc. Prior to screening, the medium is replaced with a fresh medium or with an appropriate non-cytotoxic buffer, and a test compound or the like is added thereto, followed by culturing for a given period of time. Subsequently, the cells are extracted or the supernatant is recovered and the resulting product is quantified by the respective methods. Where it is difficult to detect the production of an indicator substance for the cell stimulating activities (e.g., arachidonic acid, etc.) due to a degrading enzyme contained in the cells, an inhibitor against such a degrading enzyme may be added prior to the assay. For detecting activities such as the cAMP production suppressing activity, the baseline production in the cells is increased by forskolin or the like and the suppressing effect on the increased baseline production can be detected.

To perform the screening by assaying the cell stimulating activities, cells in which an appropriate form of the receptor of the present invention is expressed are required. As the cells where the receptor of the present invention is expressed, the aforesaid cell line where the receptor of the present invention is expressed, etc. are desired.

Examples of the test compound include peptides, proteins, antibodies, non-peptide compounds, synthetic compounds, fermentation products, cell extracts, plant extracts, animal tissue extracts, blood plasma, and the like.

In more detail, the screening methods of the cell stimulating assay system described above are described in (1) to (13) below.

(1) When the receptor-expressed cells are stimulated by the receptor agonist, G protein in the cells is activated and GTP binds thereto. This phenomenon is observed also in a membrane fraction of the receptor-expression cells. Usually, GTP is hydrolyzed and changes to GDP; when GTPγS is previously added to the reaction solution, GTPγS binds to G protein as GTP does, but does not undergo hydrolysis so that the state bound to the G protein-containing cell membrane is maintained. When labeled GTPγS is used, the cell stimulating activities of the receptor agonist-expressed cell can be assayed by determining the labeled GTPγS remained on the cell membrane.

Utilizing this reaction, the compound that changes the binding properties of the ligand of the present invention to the receptor of the present invention can be screened by assaying the stimulating activities of the ligand of the present invention on the cells where the receptor of the present invention is expressed.

This method is carried out using the membrane fraction containing the receptor of the present invention. In this assay method, the substance showing the activity of promoting the binding of GTPγS to the membrane fraction containing the receptor of the present invention is an agonist.

Specifically, the compound that changes the binding properties of the ligand of the present invention to the receptor of the present invention can be screened by assaying the GTPγS binding promoting activities on the membrane fraction containing the receptor of the present invention in the presence of labeled GTPγS, when the ligand of the present invention is brought in contact with the membrane fraction containing the receptor of the present invention and when the ligand of the present invention and a test compound are brought in contact with the membrane fraction containing the receptor of the present invention; and comparing the activities.

In this method, the test compound showing the activity of suppressing the GTPγS binding promoting activity by the ligand of the present invention against the membrane fraction containing the receptor of the present invention can be selected as an antagonist candidate compound.

On the other hand, when a test compound alone is brought into contact with the cell membrane fraction of the receptor of the present invention, the agonist can also be screened by assaying the GTPγS binding-promoting activities in the cell membrane fraction containing the receptor of the present invention.

A specific example of the screening method is described below.

The membrane fraction containing the receptor of the present invention, which is prepared by a modification of publicly known methods, is diluted with a buffer for membrane dilution (50 mM Tris, 5 mM $MgCl_2$, 150 mM NaCl, 1 μM GDP, 0.1% BSA, pH 7.4). A degree of dilution varies depending upon the amount of a receptor expressed. The dilution is dispensed by 0.2 ml each in Falcon 2053, to which the ligand of the present invention or the ligand of the present invention and a test compound is/are added, and $[^{35}S]$GTPγS is further added to the mixture in a final concentration of 200 pM. After maintaining at 25°C for an hour, 1.5 ml of ice-cooled wash buffer (50 mM Tris, 5 mM $MgCl_2$, 150 mM NaCl, 0.1% BSA, 0.05% CHAPS, pH 7.4) is added to the mixture followed by filtration through a glass fiber filter paper GF/F. After keeping at 65°C for 30 minutes, the mixture is dried and the radioactivity of $[^{35}S]$ GTPγS bound to the membrane fraction remained on the filter paper is measured with a liquid scintillation counter. When the radioactivity in the experimental zone added with the ligand of the present invention alone is defined as 100% and the radioactivity in the experimental zone not added with the ligand of the present invention is defined as 0%, an effect of the test compound on the GTPγS binding promoting activity by the ligand of the present invention is worked out. The test compound showing the GTPγS binding promoting activity of, for example, 50% or less can be selected as an antagonist candidate compound.

(2) In the cells where the receptor of the present invention is expressed, the intracellular cAMP production is suppressed by stimulation of the ligand of the present invention. Utilizing this reaction, the compound that changes the binding properties of the ligand of the present invention to the receptor of the present invention can be screened by assaying the stimulating activities of the ligand of the present invention on the cells where the receptor of the present invention is expressed.

Specifically, the compound that changes the binding properties of the ligand of the present invention to the receptor of the present invention can be screened by assaying intracellular cAMP production suppressing activities on the cells in the presence of a substance capable of increasing the intracellular cAMP level, when the ligand of the present invention is brought in contact with the cells where the receptor of the present invention is expressed and when the ligand of the present invention and a test compound are brought in contact with the cells where the receptor of the present invention is expressed; and comparing the activities.

As the substance capable of increasing the intracellular cAMP level, there are employed, e.g., forskolin, calcitonin, etc.

The amount of cAMP produced in the cells where the receptor of the present invention is expressed can be assayed by the RIA system using an anti-cAMP antibody, whose antibody is obtained from immunized mouse, rat, rabbit, goat, bovine, etc., and [$^{125}$I]-labeled cAMP (both commercially available) or by the EIA system using an anti-cAMP antibody and labeled cAMP in combination. It is also possible to quantify by the SPA (Scintillation Proximity Assay) method, using beads, which contain scintillants bearing anti-cAMP antibodies immobilized using protein A or antibodies against IgG, etc. of the animal used to produce the anti-cAMP antibodies, [$^{125}$I]-labeled cAMP and the quantification by a competitive cAMP assay kit (Perkin Elmer) applying AlphaScreen (Perkin Elmer) or a chemical amplification type luminescence proximity homogeneous assay system.

In this method, the test compound showing the activity of inhibiting the cAMP production suppressing activity by the ligand of the present invention against the cells wherein the receptor of the present invention is expressed can be selected as an antagonist candidate compound.

On the other hand, when a test compound alone is brought into contact with the cells where the receptor of the present invention is expressed, a compound showing an agonist activity can be screened by inspecting the cAMP production suppressing activity.

One specific example of the screening method is described below.

The cells where the receptor of the present invention is expressed (e.g., animal cells such as CHO cells, etc.) are plated on a 24-well plate in 5 x $10^4$ cells/well followed by cultivation for 48 hours. The cells are washed with Hanks' balanced salt solution (pH 7.4) containing 0.2 mM 3-isobutyl-methylxanthine, 0.05% BSA and 20 mM HEPES (hereinafter simply referred to as a reaction buffer). Thereafter, 0.5 ml of the reaction buffer is added to the cells and the mixture is kept warm in the medium for 30 minutes. The reaction buffer is removed and 0.25 ml of a fresh reaction buffer is added to the cells. Then, 0.25 ml of a 2 μM forskolin-containing reaction buffer, in which 20 μM of the ligand of the present invention or 20 μM of the ligand of the present invention and a test compound is/are incorporated, is added to the cells, followed by reacting at 37°C for 24 minutes. The reaction is terminated by adding 100 μl of 20% perchloric acid. The reaction mixture is then put on ice for an hour to extract intracellular cAMP. The amount of cAMP in the extract is measured using a cAMP EIA kit (Amersham Pharmacia Biotech). Taking the amount of cAMP produced by forskolin stimulation as 100% and the amount of cAMP inhibited by addition of 1 μM of the ligand of the present invention as 0%, an effect of the test compound on the cAMP production suppressing activity by the ligand of the present invention is calculated. The test compound that inhibits the activity of the ligand of the present invention to increase the cAMP producing activity, e.g., to 50% or more, can be selected as an antagonist candidate compound.

Further in the case of using the cells where the receptor of the present invention is expressed and which show the property of increasing the intracellular cAMP level through stimulation by the ligand of the present invention, the compound that changes the binding properties of the ligand of the present invention to the receptor of the present invention can be screened by assaying the intracellular cAMP production promoting activities on the cells, when the ligand of the present invention is brought in contact with the cells where the receptor of the present invention is expressed and when the ligand of the present invention and a test compound are brought in contact with the cells where the receptor of the present invention is expressed; and comparing the activities.

In this method, the test compound showing the activity of inhibiting the cAMP production promoting activity by the ligand of the present invention against the cells where the receptor of the present invention is expressed can be selected as an antagonist candidate compound.

On the other hand, when a test compound alone is brought into contact with the cell where the receptor of the present invention is expressed, a compound showing an agonist activity can be screened by monitoring the cAMP producing activity.

The cAMP production promoting activity is assayed by the method described above, through quantification of cAMP produced by adding the ligand of the present invention or the ligand of the present invention and a test compound to the cell where the receptor of the present invention is expressed (e.g., animal cells such as CHO cells, etc.), without adding forskolin in the screening method described above.

(3) The compound that changes the binding properties of the ligand of the present invention to the receptor of the present invention can be screened by assaying the stimulating activities of the ligand of the present invention on the cell where the receptor of the present invention is expressed, using a CRE-reporter gene vector.

A DNA containing CRE (cAMP response element) is inserted into a vector upstream the reporter gene to acquire CRE-reporter gene vector. In the CRE-reporter gene vector-transfected cells where the receptor of the present invention is expressed, stimulation accompanied by increased cAMP induces expression of the reporter gene mediated by CRE and subsequent production of the gene product (protein) of the reporter gene. That is, changes in the amount of cAMP in the CRE-reporter gene vector-transfected cells can be detected by assaying the enzyme activity of the reporter gene protein.

Specifically, the compound that changes the binding properties of the ligand of the present invention to the receptor of the present invention can be screened by assaying the enzyme activities of the reporter gene protein on the cells in the presence of a substance capable of increasing the intracellular cAMP level, when the ligand of the present invention is brought in contact with the CRE-reporter gene vector-transfected cells where the receptor of the present invention is expressed and when the ligand of the present invention and a test compound are brought in contact with the CRE-reporter gene vector-transfected cells where the receptor of the present invention is expressed; and comparing the activities.

As the substance that increases the intracellular cAMP level, there are employed, e.g., forskolin, calcitonin, etc.

As the vector, there may be employed, e.g., PicaGene Basic Vector, PicaGene Enhancer Vector (Toyo Ink Mfg. Co., Ltd.), and the like. A CRE-bearing DNA is inserted into the vector described above at the multicloning site upstream the reporter gene, e.g., luciferase gene, which is made a CRE-reporter gene vector.

In this method, a test compound which recovers the enzyme activity suppression of the reporter gene protein by the ligand of the present invention can be selected as an antagonist candidate compound.

On the other hand, the agonist can be screened as well by contacting a test compound alone with the cell where the receptor of the present invention is expressed and assaying the suppression of luminescence level increased by forskolin stimulation as in the ligand of the present invention.

Taking as an example in which luciferase is used as a reporter gene, a specific example of this screening method is described below.

The CRE-reporter gene (luciferase)-transfected cells where the receptor of the present invention is expressed are plated on a 24-well plate in 5 x $10^3$ cells/well followed by cultivation for 48 hours. The cells are washed with Hanks' balanced salt solution (pH 7.4) containing 0.2 mM 3isobutyl-methylxanthine, 0.05% BSA and 20 mM HEPES (hereinafter merely referred to as a reaction buffer). Thereafter, 0.5 ml of the reaction buffer is added to the cells and the mixture is kept warm in the medium for 30 minutes. The reaction buffer is removed and 0.25 ml of a fresh reaction buffer is added to the cells. Then, 100 μM of the ligand of the present invention or 100 μM of the ligand of the present invention and a test compound is/are added to 0.25 ml of the reaction buffer containing 2 μM forskolin, which is added to the cells. The reaction is then carried out at 37°C for 24 minutes. The cells are dissolved in a cell lysis agent for PicaGene (Toyo Ink Mfg. Co., Ltd.) and a luminescent substrate (Toyo Ink Mfg. Co., Ltd.) is added to the lysate. Luminescence by luciferase is measured with a luminometer, a liquid scintillation counter or a top counter. The levels of luminescence by luciferase are measured when only the ligand of the present invention is added and when 100 μM of the ligand of the present invention and a test compound are added, and compared therebetween.

The ligand of the present invention suppresses the increase in luminescent level by luciferase, based on forskolin stimulation. The compound that recovers the suppression can be selected as an antagonist candidate compound.

As the reporter gene, there may be employed genes, e.g., alkaline phosphatase, chloramphenicol acetyltransferase, β-galactosidase, etc. The enzyme activities of these reporter gene proteins are assayed in accordance with methods publicly know, or using commercially available assay kits. The alkaline phosphatase activity can be assayed by using, e.g., Lumi-Phos 530 manufactured by Wako Pure Chemical Industries, Ltd.; the chloramphenicol acetyltransferase activity by using, e.g., FAST CAT chloramphenicol Acetyltransferase Assay Kit manufactured by Wako Pure Chemical Industries, Ltd.; and the β-galactosidase activity by using, e.g., Aurora Gal-XE manufactured by Wako Pure Chemical Industries, Ltd.

(4) The compound that changes the binding properties of the ligand of the present invention to the receptor of the present invention can be screened by assaying the stimulating activities of the ligand of the present invention on the cell where the receptor of the present invention is expressed, using a SRE-reporter gene vector.

A DNA containing SRE (serum response element) is inserted into a vector upstream its reporter gene to acquire the SRE-reporter gene vector. In the SRE-reporter gene vector-transfected cells where the receptor of the present invention is expressed, activation of proliferative signals such as MAP kinase activity in response to serum stimulation, etc. induces expression of the reporter gene mediated by SRE and subsequent production of the gene product (protein) of the reporter gene. That is, activation of proliferative signals in the SRE-reporter gene vector-transfected cells can be detected by assaying the enzyme activity of the reporter gene protein.

Specifically, the compound that changes the binding properties of the ligand of the present invention to the receptor of the present invention can be screened by assaying the enzyme activities of the reporter gene protein, in the case of contacting the ligand of the present invention with the SRE-reporter gene vector-transfected cells where the receptor of the present invention is expressed and in the case of contacting the ligand of the present invention and a test compound with the SRE-reporter gene vector-transfected cells where the receptor of the present invention is expressed; and comparing the activities.

As the vector, there may be employed, e.g., PicaGene Basic Vector, PicaGene Enhancer Vector (Toyo Ink Mfg. Co., Ltd.), and the like. A DNA containing SRE is inserted into the vector described above at the multicloning site upstream the reporter gene, e.g., luciferase gene, which is made a SRE-reporter gene vector.

In this method, the test compound that suppresses the enzyme activity of the reporter gene protein by the ligand of the present invention can be selected as an antagonist candidate compound.

On the other hand, the agonist can be screened as well by contacting a test compound alone with the cell where the receptor of the present invention is expressed and measuring an increase of luminescence level as in the ligand of the present invention.

Taking as an example in which luciferase is used as a reporter gene, a specific example of this screening method is described below.

The SRE-reporter gene (luciferase)-transfected cells where the receptor of the present invention is expressed are plated on a 24-well plate in 5 x $10^3$ cells/well followed by cultivation for 48 hours. The cells are washed with Hanks' buffer (pH 7.4) containing 0.05% BSA and 20 mM HEPES (hereinafter merely referred to as a reaction buffer). Thereafter, 0.5 ml of the reaction buffer is added to the cells and the mixture is kept warm in the medium for 30 minutes. The reaction buffer is removed and 0.25 ml of a fresh reaction buffer is added to the cells. Thereafter, 0.25 ml of the reaction buffer supplemented with 100 μM of the ligand of the present invention or 100 μM of the ligand of the present invention and a test compound is added to the cells. The reaction is then carried out at 37°C for 24 minutes. The cells are dissolved in a cell lysis agent for PicaGene (Toyo Ink Mfg. Co., Ltd.) and a luminescent substrate (Toyo Ink Mfg. Co., Ltd.) is added to the lysate. Luminescence by luciferase is measured with a luminometer, a liquid scintillation counter or a top counter. The levels of luminescence by luciferase are measured when only the ligand of the present invention is added and when 100 μM of the ligand of the present invention and a test compound are added, and compared therebetween.

The ligand of the present invention increases the luminescence level by luciferase. The compound that suppresses the increase can be selected as an antagonist candidate compound.

As the reporter gene, there may be employed genes, e.g., alkaline phosphatase, chloramphenicol acetyltransferase, β-galactosidase, etc. The enzyme activities of these reporter gene proteins are assayed in accordance with methods publicly know, or using commercially available assay kits. The alkaline phosphatase activity can be assayed by using, e.g., Lumi-Phos 530 manufactured by Wako Pure Chemical Industries, Ltd.; the chloramphenicol acetyltransferase activity by using, e.g., FAST CAT chloramphenicol Acetyltransferase Assay Kit manufactured by Wako Pure Chemical Industries, Ltd.; and the β-galactosidase activity by using, e.g., Aurora Gal-XE manufactured by Wako Pure Chemical Industries, Ltd.

(5) The cells where the receptor of the present invention is expressed extracellularly release arachidonic acid metabolites by stimulation of the ligand of the present invention. Utilizing this reaction, the stimulating activities of the ligand of the present invention on the cell where the receptor of the present invention is expressed are assayed, whereby the compound that changes the binding properties of the ligand of the present invention to the receptor of the present invention can be screened.

Labeled arachidonic acid is previously taken up into the cell where the receptor of the present invention is expressed. Thus, the arachidonic acid metabolite releasing activity can be assayed by measuring the labeled arachidonic acid metabolite released at the outside of the cell.

Specifically, the compound that changes the binding properties of the ligand of the present invention to the receptor of the present invention can be screened by assaying arachidonic acid metabolite-releasing activities, when the ligand of the present invention is brought in contact with the labeled arachidonic acid-containing cells where the receptor of the present invention is expressed and when the ligand of the present invention and a test compound are brought in contact with the labeled arachidonic acid-containing cells where the receptor of the present invention is expressed; and comparing the activities.

In this method, the test compound that inhibits the arachidonic acid metabolite-releasing activity by the ligand of the present invention can be selected as an antagonist candidate compound.

Also, a test compound alone is brought into contact with the cell where the receptor of the present invention is expressed and the arachidonic acid metabolite-releasing activity in the cell where the receptor of the present invention is expressed is examined by publicly known methods. Thus, the compound showing the agonist activity can be screened as well.

A specific example of this screening method is described below.

The cells where the receptor of the present invention is expressed are plated on a 24-well plate in 5 x $10^4$ cells/well. After cultivation for 24 hours, [$^3$H] arachidonic acid is added to the cells in 0.25 μCi/well. Sixteen hours later, the cells are washed with Hanks' balanced salt solution (pH 7.4) containing 0.05% BSA and 20 mM HEPES (hereinafter simply referred to as a reaction buffer). To each well is added 500 μl of the reaction buffer containing the ligand of the present invention in the final concentration of 20 μM, or the ligand of the present invention in the final concentration of 20 μM and a test compound. After incubation at 37°C for 60 minutes, 400 μl of the reaction solution is charged in a scintillator and the amount of [$^3$H] arachidonic acid metabolites released in the reaction solution is measured using a scintillation counter.

When the amount of [$^3$H] arachidonic acid metabolites when 500 μl of the reaction buffer alone is added (neither the ligand of the present invention nor the test compound is added) is taken as 0% and the amount of [$^3$H] arachidonic acid metabolites when the reaction buffer containing 20 μM of the ligand of the present invention is added (no test compound is added) is taken as 100%, the amount of [$^3$H] arachidonic acid metabolites released where the test compound is added is calculated.

The compound showing the arachidonic acid metabolite-releasing activity of, e.g., 50% or less, can be selected as an antagonist candidate compound.

(6) In the cells where the receptor of the present invention is expressed, the intracellular Ca level increases by stimulation of the ligand of the present invention. Utilizing this reaction, the stimulating activities of the ligand of the present invention on the cells where the receptor of the present invention is expressed are assayed, whereby the compound that changes the binding properties of the ligand of the present invention to the receptor of the present invention can be screened.

Specifically, the compound that changes the binding properties of the ligand of the present invention to the receptor of the present invention is screened by assaying the intracellular calcium level increasing activities when the ligand of the present invention is brought in contact with the cells where the receptor of the present invention is expressed and when the ligand of the present invention and a test compound are brought in contact with the cells where the receptor of the present invention is expressed; and comparing the activities. The assay is carried out in accordance with methods publicly known.

In this method, the test compound that suppresses the intracellular calcium level increasing activity by the ligand of the present invention can be selected as an antagonist candidate compound.

On the other hand, the agonist can be screened as well by assaying an increase of fluorescence intensity by the addition of a test compound alone.

One specific example of the screening method is described below.

The cells where the receptor of the present invention is expressed are plated on a sterilized cover glass for microscopy. Two days after, the culture medium is replaced by HBSS in which 4 mM Fura-2 AM (Dojin Chemical Laboratory) is suspended, followed by allowing to stand at room temperature for 2 hours and 30 minutes. After washing with HBSS, the cover glass is set on a cuvette, and an increased ratio of fluorescence intensity at 505 nm is measured with a fluorescence spectrophotometer at excited wavelengths of 340 nm and 380 nm, when the ligand of the present invention or the ligand of the present invention and a test compound is/are added, and comparison is made.

Also, FLIPR (manufactured by Molecular Device) may be used. Fluo-3 AM (manufactured by Dojin Kagaku Kenkyusho) is added to a suspension of the cells where the receptor of the present invention is expressed, thereby to take Fluo-3 AM into the cells. After the supernatant is washed several times through centrifugation and the cells are plated on a 96-well plate. After setting in the FLIPR device, the ligand of the present invention or the ligand of the present invention and a test compound is/are added thereto. Using a fluorescence spectrophotometer, an increase in the ratio of fluorescence intensity is measured and comparison is made, as in Fura-2.

Furthermore, the compound that changes the binding properties of the ligand of the present invention to the receptor of the present invention can also be screened by co-expressing a gene (e.g., aequorin, etc.) for the protein that emits light in response to increased Ca ions in the cells where the receptor of the present invention is expressed, and utilizing the luminescence emitted by conformational switch of the gene protein (e.g., aequorin,. etc.) to the Ca-bound protein.

The cells where the receptor of the present invention is expressed and the gene of protein capable of emitting light by increasing the intracellular Ca ions is co-expressed, are plated on a 96-well plate. The ligand of the present invention or the ligand of the present invention and a test compound is/are added thereto and using a fluorescence spectrophotometer, an increase in the ratio of fluorescence intensities is measured and comparison is made as described above.

The test compound that suppresses the increase in fluorescence intensity by the ligand of the present invention can be selected as an antagonist candidate compound.

(7) When the receptor agonist is added to receptor-expressing cells, the level of intracellular inositol triphosphate increases. By utilizing the intracellular inositol triphosphate producing activity in the cells where the receptor of the present invention is expressed, the compound that changes the binding properties of the ligand of the present invention to the receptor of the present invention can be screened.

Specifically, the compound that changes the binding properties of the ligand of the present invention to the receptor of the present invention is screened by assaying the inositol triphosphate producing activities in the presence of labeled inositol, when the ligand of the present invention is brought in contact with the cells where the receptor of the present invention is expressed and when the ligand of the present invention and a test compound are brought in contact with the cells where the receptor of the present invention is expressed; and comparing the activities. The assay is carried out in accordance with methods publicly known.

In this method, the test compound that suppresses the inositol triphosphate producing activities can be selected as an antagonist candidate compound.

On the other hand, an agonist can also be screened by contacting a test compound alone with the cells where the receptor of the present invention is expressed and measuring an increase in the inositol triphosphate production.

One specific example of the screening method is described below.

The cells wherein the receptor of the present invention is expressed are plated on a 24-well plate and cultured for a day. Then, the cells are cultured for a day in medium supplemented with myo-[2-$^3$H] inositol (2.5 $\mu$Ci/well). The cells are thoroughly washed with radioactive inositol-free medium. After the ligand of the present invention or the ligand of the present invention and a test compound is/are added to the cells, 10% perchloric acid is added to terminate the reaction. The reaction mixture is neutralized with 1.5 M KOH and 60 mM HEPES solution and then passed through a column packed with 0.5 ml of AG1 x 8 resin (Bio-Rad). After washing with 5 mM sodium tetraborate ($Na_2B_4O_7$) and 60 mM ammonium formate, the radioactivity eluted with 1M ammonium formate and 0.1M formic acid is assayed with a liquid scintillation counter. When the radioactivity without adding the ligand of the present invention is made 0% and the radioactivity when the ligand of the present invention is added is made 100%, an effect of the test compound on the binding of the ligand of the present invention to the receptor of the present invention is calculated.

A test compound which reduces the inositol triphosphate production activity to, e.g., 50% or less, can be selected as an antagonist candidate compound.

(8) The compound that changes the binding properties of the ligand of the present invention to the receptor of the present invention can be screened by assaying the stimulating activities of the ligand of the present invention on the cells where the receptor of the present invention is expressed, using a TRE-reporter gene vector.

A DNA containing TRE (TPA response element) is inserted into a vector upstream the reporter gene to acquire a TRE-reporter gene vector. In the TRE-reporter gene vector-transfected cells where the receptor of the present invention is expressed, stimulation accompanied by an increase of the intracellular Ca level induces expression of TRE-mediated reporter gene and production of the reporter gene product (protein) subsequent thereto. That is, changes in the calcium level in the TRE-reporter gene vector-transfected cells can be detected by assaying the enzyme activity of the reporter gene protein.

Specifically, the compound that changes the binding properties of the ligand of the present invention to the receptor of the present invention is screened by assaying the enzyme activities of the reporter gene protein, when the ligand of the present invention is brought in contact with the TRE-reporter gene vector-transfected cells where the receptor of the present invention is expressed and when the ligand of the present invention and a test compound are brought in contact with the TRE-reporter gene vector-transfected cells where the receptor of the present invention is expressed; and comparing the activities.

As the vector, there can be employed, e.g., PicaGene Basic Vector, PicaGene Enhancer Vector (Toyo Ink Mfg. Co., Ltd.), and the like. A DNA containing TRE is inserted into the vector described above at the multicloning site upstream the reporter gene, e.g., luciferase gene, which is made a TRE-reporter gene vector.

In this method, the test compound that suppresses the enzyme activity of the reporter gene protein by the ligand of the present invention can be selected as an antagonist candidate compound.

On the other hand, the agonist can also be screened by contacting a test compound alone with the TRE-reporter gene vector-transfected cells where the receptor of the present invention is expressed and measuring the increased luminescence level as in the ligand of the present invention.

Taking as an example the embodiment wherein luciferase is used as the reporter gene, a specific example of this screening method is described below.

The TRE-reporter gene (luciferase)-transfected cells where the receptor of the present invention is expressed are plated on a 24-well plate in 5 x $10^3$ cells/well followed by cultivation for 48 hours. After the cells are washed with Hanks' balanced salt solution (pH 7.4) containing 0.05% BSA and 20 mM HEPES, 100 nμM of the ligand of the present invention or 100μM of the ligand of the present invention and a test compound is/are added to the cells, followed by reacting at 37°C for 60 minutes. The cells are dissolved in a cell lysis agent for PicaGene (Toyo Ink Mfg. Co., Ltd.) and a luminescence substrate (Toyo Ink Mfg. Co., Ltd.) is added to the lysate. The luminescence by luciferase is measured by a luminometer, a liquid scintillation counter or a top counter. The amounts of luminescence by luciferase are measured when the ligand of the present invention is added and when 100μM of the ligand of the present invention and a test compound are added, and compared therebetween.

In response to the increased intracellular calcium by the ligand of the present invention, the amount of luminescence by luciferase increases. The compound that suppresses the increase can be selected as an antagonist candidate compound.

As the reporter gene, there may be employed genes, e.g., alkaline phosphatase, chloramphenicol acetyltransferase, β-galactosidase, etc. The enzyme activities of these reporter gene proteins are assayed in accordance with methods publicly known, or by using assay kits commercially available. The alkaline phosphatase activity can be assayed by using, e.g., Lumi-Phos 530 manufactured by Wako Pure Chemical Industries, Ltd.; the chloramphenicol acetyltransferase activity using, e.g., FAST CAT chloramphenicol Acetyltransferase Assay Kit manufactured by Wako Pure Chemical Industries, Ltd.; and the β-galactosidase activity using, e.g., Aurora Gal-XE manufactured by Wako Pure Chemical Industries, Ltd.

(9) In the cell where the receptor of the present invention is expressed, MAP kinase is activated and increased by stimulation of the ligand of the present invention. Utilizing the reaction, the compound that changes the binding properties of the ligand of the present invention to the receptor of the present invention can be screened by assaying the stimulation activities of the ligand of the present invention on the cell where the receptor of the present invention is expressed.

Specifically, the compound that changes the binding properties of the ligand of the present invention to the receptor of the present invention is screened by assaying the cell growth, when the ligand of the present invention is brought in contact with the cells where the receptor of the present invention is expressed and when the ligand of the present invention and a test compound are brought in contact with the cells where the receptor of the present invention is expressed; and comparing the cell growth.

The growth of the cells where the receptor of the present invention is expressed may be determined by assaying, e.g., the MAP kinase activity, the thymidine uptake activity, the ATP level, the cell count, etc.

In a specific example, the MAP kinase activity is assayed as follows. The ligand of the present invention or the ligand of the present invention and a test compound is/are added to the cell where the receptor of the present invention is expressed; immunoprecipitation is carried out using an anti-MAP kinase antibody against obtain a MAP kinase fraction from a cell lysate; then using, e.g., MAP Kinase Assay Kit manufactured by Wako Pure Chemical Industries, Ltd. and γ-[$^{32}$P]-ATP, the MAP kinase activity is assayed; and comparison is made.

The thymidine uptake activity can be assayed by plating on a 24-well plate the cell where the receptor of the present invention is expressed, followed by incubation. After the ligand of the present invention or the ligand of the present invention and a test compound is/are added to the cells, and radioactively labeled thymidine (e.g., [methyl-$^3$H]-thymidine, etc.) is added thereto. Then the cells are lysed and by counting the radioactivity of the labeled thymidine taken up into the cells with a liquid scintillation counter, the thymidine uptake activity is assayed and comparison is made.

In assaying the ATP levels, cells wherein the receptor of the present invention is expressed are plated on a 96-well plate and incubated. The ligand of the present invention or the ligand of the present invention and a test compound is/are added thereto, and intracellular ATP levels are assayed using, e.g., CellTiter-Glo (Promega) and compared.

To determine the cell counting, the cells where the receptor of the present invention is expressed are plated on a 24-well plate, followed by incubation. The ligand of the present invention or the ligand of the present invention and a test compound is/are added to the cells, and MTT (3-(4,5-dimethyl-2-thiazolyl)-2,5-diphenyl-2H-tetrazolium bromide) is further added thereto. MTT taken up into the cells changes to MTT formazan, which absorption is measured at 570 nm, after cell lysis with an aqueous isopropanol solution rendered acidic with hydrochloric acid. Then, comparison is made.

In this method, the test compound that suppresses the growth of the cells where the receptor of the present invention is expressed can be selected as an antagonist candidate compound.

On the other hand, the agonist may be screened as well by contacting a test compound alone with the cells where the receptor of the present invention is expressed and assaying the cell growth activity as in the ligand of the present invention.

A specific example of the screening method utilizing the thymidine uptake activity is described below.

The cells where the receptor of the present invention is expressed are plated on a 24-well plate in 5000 cells/well followed by incubation for one day. Next, the cells are incubated in a serum-free medium for 2 days to bring the cells under starvation. The ligand of the present invention or the ligand of the present invention and a test compound is/are added to the cells. After incubation for 24 hours, [methyl $^3$H] thymidine is added in 0.015 MBq/well, followed by incubation for 6 hours. After the cells are washed with PBS, methanol is added to the cells. The mixture is allowed to stand for 10 minutes. Next, 5% trichloroacetic acid is added and the mixture is allowed to stand for 15 minutes. The immobilized cells are washed 4 times with distilled water. After cell lysis with a 0.3 N sodium hydroxide solution, the radioactivity in the lysate is assayed with a liquid scintillation counter.

The compound that suppresses the increase in radioactivity by the addition of the ligand of the present invention can be selected as an antagonist candidate compound.

(10) In the cell where the receptor of the present invention is expressed, the potassium channel is activated by stimulation of the ligand of the present invention so that K ions present within the cells are effluxed outside the cells. Utilizing this reaction, the compound that changes the binding properties of the ligand of the present invention to the receptor of the present invention can be screened by assaying the stimulating activities of the ligand of the present invention on the cells where the receptor of the present invention is expressed.

Rb ions (rubidium ions) in the related elements to K ions flow out of the cells through the potassium channel without being discriminated from K ions. Thus, radioactive isotope Rb ([$^{86}$Rb]) is previously incorporated into the cells where the receptor of the present invention is expressed, and the efflux of $^{86}$Rb that flows out in response to stimulation by the ligand of the present invention (efflux activity) is determined thereby to assay the stimulating activities of the ligand of the present invention on the cells where the receptor of the present invention is expressed.

Specifically, the compound that changes the binding properties of the ligand of the present invention to the receptor of the present invention is screened by assaying $^{86}$Rb efflux activities in the presence of $^{86}$Rb, when the ligand of the present invention is brought in contact with the cells where the receptor of the present invention is expressed and when the ligand of the present invention and a test compound are brought in contact with the cells where the receptor of the present invention is expressed; and comparing the activities.

According to this method, the test compound that suppresses the increase of the $^{86}$Rb efflux activities associated with stimulation by the ligand of the present invention can be selected as an antagonist candidate compound.

On the other hand, the agonist can be screened as well by contacting a test compound alone with the cell where the receptor of the present invention is expressed and measuring the increase in the efflux activity of $^{86}$Rb.

A specific example of the screening method is described below.

The cells where the receptor of the present invention is expressed are plated on a 24-well plate and cultured for 2 days. Thereafter, the cells are kept warm for 2 hours in a medium containing 1 mCi/ml of $^{86}$RbCl. The medium is thoroughly washed to completely remove $^{86}$RbCl in the outer liquid. The ligand of the present invention or the ligand of the present invention and a test compound is/are added to the cells. After the outer liquid is recovered 30 minutes after, the radioactivity is measured with a $\gamma$ counter, and comparison is made.

The test compound which suppresses the increase in the efflux activity of $^{86}$Rb by stimulation of the ligand of the present invention can be selected as an antagonist candidate compound.

(11) The cell where the receptor of the present invention is expressed reacts with the ligand of the present invention so that the extracellular pH changes. Utilizing this reaction, the compound that changes the binding properties of the ligand of the present invention to the receptor of the present invention can be screened by assaying the stimulating activities of the ligand of the present invention on the cell where the receptor of the present invention is expressed.

Specifically, the compound that changes the binding properties of the ligand of the present invention to the receptor of the present invention is screened by measuring changes in extracellular pH, when the ligand of the present invention is brought in contact with the cells where the receptor of the present invention is expressed and when the ligand of the present invention and a test compound are brought in contact with the cells where the receptor of the present invention is expressed; and comparing the changes.

The extracellular pH change is determined using, e.g., Cytosensor Device (Molecular Device, Inc.).

In this method, the test compound that suppresses the extracellular pH change by the ligand of the present invention can be selected as an antagonist candidate compound.

On the other hand, the agonist can be screened as well by contacting a test compound alone with the cell where the receptor of the present invention is expressed and measuring the extracellular pH changes, as in the ligand of the present invention.

A specific example of the screening method is described below.

The cells where the receptor of the present invention is expressed are cultured overnight in a capsule for Cytosensor Device, which is set in a chamber of the device to reflux 0.1% BSA-containing RPMI 1640 medium (manufactured by Molecular Device, Inc.) until the extracellular pH becomes stable. After the pH becomes stable, a medium containing the ligand of the present invention or the ligand of the present invention and a test compound is refluxed onto the cells. The pH changes in the medium caused by reflux are measured and compared.

The compound that suppresses the extracellular pH change by the ligand of the present invention can be selected as an antagonist candidate compound.

(12) In yeast (Saccharomyces Cerevisiae), the sex pheromone receptor STe2 of haploid α-mating type (MATα) is coupled to G protein Gpal and activates MAP kinase in response to the sex pheromone α-mating factor, whereby Far1 (cell-cycle arrest) and the transcription activator Ste 12 are activated. Ste 12 induces expression of various proteins (e.g., FUS1 which takes part in mating). On the other hand, regulator Sst2 functions to inhibit the foregoing process. In this system, an attempt has been made to construct the assay system for the reaction of a receptor agonist with a receptor, which involves preparing a receptor gene-transfected yeast, activating the intracellular signal transduction system in yeast by stimulation with the receptor agonist and using the resulting growth, etc. as an indicator (Trends in Biotechnology, 15, 487-494, 1997). Utilizing this receptor gene-transfected yeast system, the compound that changes the binding properties of the ligand of the present invention to the receptor of the present invention can be screened.

A specific example is described below.

Ste2 in MATα yeast and the gene encoding Gpa1 are removed and instead, a gene for the receptor of the present invention and a gene encoding the Gpal-Gai2 fused protein are introduced. The gene encoding Far is removed to cause no cell-cycle arrest and the gene encoding Sst2 is removed to increase the sensitivity in response to the ligand of the present invention. Furthermore, FUS1-HIS3 gene, which is FUS1 ligated with histidine biosynthesis gene HIS3, is introduced. The foregoing genetic recombinant engineering can be carried out by the method described in, e.g., Molecular and Cellular Biology, 15, 6188-6195, 1995, using the receptor of the present invention in place of somatostatin receptor type 2 (SSTR2) gene.

The thus constructed transformant yeast is responsive to the ligand of the present invention with a high sensitivity so that MAP kinase is activated to cause synthesis of histidine biosynthesis enzyme. Thus, the transformant becomes capable of growing in a histidine-deficient medium.

Accordingly, the compound that changes the binding properties of the ligand of the present invention to the receptor of the present invention can be screened by incubating the yeast described above where the receptor of the present invention is expressed (MATα yeast wherein Ste2 gene and Gpa1 gene are removed, the receptor gene of the present invention and the Gpa1-Gai2 fused protein-encoding gene, Far gene and Sst2 gene are removed, and FUS1-HIS3 gene is transfected) in a histidine-deficient medium, contacting the ligand of the present invention or the ligand of the present invention and a test compound with the yeast, assaying growth of the yeast, and comparing the growth.

In this method, the test compound that suppresses growth of the yeast can be selected as an antagonist candidate compound.

On the other hand, the agonist can be screened as well by contacting a test compound alone with the yeast where the receptor of the present invention is expressed and assaying growth of the yeast as in the ligand of the present invention.

A specific example of the screening method is described below.

The yeast described above where the receptor of the present invention is expressed thus produced is incubated overnight in a complete synthesis liquid medium and then added to a histidine-free, dissolved agar medium in a concentration of 2 x $10^4$ cells/ml. Then, the yeast is plated on a square Petri dish of 9 x 9 cm. After the agar is solidified, a sterilized filter paper impregnated with the ligand of the present invention or the ligand of the present invention and a test compound is put on the agar surface, which is incubated at 30°C for 3 days. To determine the effect of the test compound, growth of yeast around the filter paper is compared to the case wherein the sterilized filter paper impregnated only with the ligand of the present invention. Alternatively, the assay can be made by previously adding the ligand of the present invention to a histidine-free agar medium, impregnating the sterilized, filter paper with a test compound alone to incubate the yeast and monitoring that growth of the yeast over the entire surface of the Petri dish is affected at the periphery of the filter paper.

The compound that suppresses growth of the yeast can be selected as an antagonist candidate compound.

(13) When the receptor gene RNA of the present invention is injected into Xenopus laevis oocytes and stimulated by the ligand of the present invention, the intracellular Ca ion level increases to cause a calcium-activated chloride current, which can be taken as fluctuation in membrane potential (the same applies also to the case where fluctuation occurs in K ion level gradient). Utilizing the above reaction caused by the ligand of the present invention in Xenopus laevis oocytes where the receptor of the present invention is transfected, the compound that changes the binding properties of the ligand of the present invention to the receptor of the present invention can be screened by assaying the stimulating activities of the ligand of the present invention on the cells where the receptor of the present invention is expressed.

Specifically, the compound that changes the binding properties of the ligand of the present invention to the receptor of the present invention can be screened by assaying changes in cell membrane potential, when the ligand of the present invention is brought in contact with Xenopus laevis oocytes where the receptor gene RNA of the present invention is transfected and when the ligand of the present invention and a test compound are brought in contact with Xenopus laevis oocytes where the receptor gene RNA of the present invention is transfected; and comparing the changes.
In this method, the test compound that suppresses the changes in cell membrane potential can be selected as an antagonist candidate compound.
On the other hand, the agonist can be screened as well by contacting a test compound alone with Xenopus laevis oocytes where the receptor gene RNA of the present invention is transfected and assaying the changes in cell membrane potential as in the ligand of the present invention.
A specific example of the screening method is described below.
A female individual of Xenopus laevis anesthetized by immersing in ice water is anatomized to withdraw oocytes. The oocyte clusters are treated with collagenase (0.5 mg/ml) dissolved in an MBS solution (88 mM NaCl, 1 mM KCl, 0.41 mM $CaCl_2$, 0.33 mM $Ca(NO_3)_2$, 0.82 mM $MgSO_4$, 2.4 mM $NaHCO_3$, 10 mM HEPES; pH 7.4) at 19°C for 1 to 6 hours at 150 rpm, until the oocytes are loosen. Washing is performed 3 times by replacing the outer liquid by the MBS solution followed by microinjection of the receptor gene of the present invention or poly A-added cRNA (50 ng/50 nl) with a micromanipulator.
The receptor gene mRNA of the present invention may be prepared from tissues or cells, or may be transcribed from plasmids in vitro. The receptor gene mRNA of the present invention is incubated in the MBS solution at 20°C for 3 days. The oocytes are placed in a dimple of a voltage clamp device, which is continuously perfused with Ringer's solution, and impaled into the cells with glass microelectrodes for voltage clamp and glass microelectrodes for potential recording, in which (-) electrode is placed outside the oocytes. When the holding potential stabilizes, Ringer's solution containing the ligand of the present invention or the ligand of the present invention and a test compound is perfused to record a change in potential. An effect of the compound can be determined by comparing a change in cell membrane potential of the Xenopus laevis oocytes where the receptor gene RNA of the present invention is transfected with the case when the Ringer's solution containing the ligand of the present invention alone is perfused.
The compound that suppresses the changes in cell membrane potential can be selected as an antagonist candidate compound.
In the system described above, the changes in potential can be monitored more easily when the variations in potential increase. Therefore, polyA-added RNA of various G protein genes may be introduced. Also, the amount of luminescence, not the changes in membrane potential, can be measured by co-injecting polyA-added RNA of a gene for the protein (e.g., aequorin, etc.) that emits light in the presence of calcium.
Furthermore, the screening methods using mammal-derived mast cells and microglial cells are described below.
Using mast cells (including mast cell lines, etc.) derived from mammal (preferably, human, chimpanzee, monkey, etc.), preferably mast cells derived from skin, bladder, stomach, small intestine, lung, etc., compounds having the mast cell degranulation inhibitory action, eicosanoid production inhibitory action, cytokine production inhibitory action, mast cell growth inhibitory action, mast cell activation inhibitory action, etc. can be screened.

(1) The cytokine production inhibitor (compound having the cytokine production inhibitory action, etc.) is screened by contacting the ligand of the present invention with mast cells in the presence and absence of a test compound to prepare RNA from the cells, assaying the RNA levels of cytokine (e.g., TNF-α, IL-4, IL-5, IL-6, IL-8, IL-13, TGF-β, etc.) (by, e.g., RT-PCR, quantitative real-time-PCR, etc.) and comparing the levels.
(2) The cytokine (e.g., TNF-α, IL-4, IL-5, IL-6, IL-8, IL-13, TGF-β, etc.) protein levels in the supernatant (by, e.g., EIA, RIA, etc.) production inhibitor (compound having the cytokine production inhibitory action, etc.) is screened by contacting the ligand of the present invention with mast cells in the presence and absence of a test compound, assaying the protein levels of cytokine (e.g., TNF-α, IL-4, IL-5, IL-6, IL-8, IL-13, TGF-β, etc.) in the supernatant (by, e.g., EIA, RIA, etc.) and comparing the levels.
(3) The cytokine production inhibitor (compound having the cytokine production inhibitory action, etc.) is screened by contacting the ligand of the present invention with mast cells in the presence and absence of a test compound, assaying the counts of cytokine(e.g., TNF-α, IL-4, IL-5, IL-6, IL-8, IL-13, TGF-β, etc.) producing cells (by, e.g., the ELISPOT method) and comparing the counts.
(4) The cytokine production inhibitor (compound having the cytokine production inhibitory action, etc.) is screened by producing a number of siRNA vectors (e.g., siRNA vector, antisense oligonucleotide, antisense oligonucleotide expression vector, etc.) bearing siRNA for polynucleotide encoding the receptor of the present invention to prepare the siRNA library, transfecting the vectors into mast cells, contacting with the ligand of the present invention in the presence and absence of a test compound, assaying the expression levels (e.g., cytokine RNA levels, cytokine protein levels, etc.) of cytokine (e.g., TNF-$\alpha$, IL-4, IL-5, IL-6, IL-8, IL-13, TGF-$\beta$, etc.) in the cells and examining the nucleotide sequence of siRNA inducing a reduction in expression level. In contacting with the ligand, costimulation may be provided by an anti-IgE antibody, an antibody, an antibody-specific antigen, etc. where mast cells undergo active or passive sensitization and in other cases, costimulation may be provided by NGF, stem cell factor (SCF), cytokine, lectin, etc.

(5) The compound the eicosanoid production inhibitory action (compound having the eicosanoid production inhibitory action, etc.) is screened by contacting the ligand of the present invention with mast cells, in which radioactively labeled arachidonate is incorporated, in the presence and absence of a test compound, assaying the radioactivities in the culture supernatant and comparing the radioactivities.

(6a) The compound that has the degranulation inhibitory action (compound having the degranulation inhibitory action, etc.) is screened by contacting the ligand of the present invention with mast cells in the presence and absence of a test compound, assaying the amounts of granule contents (e.g., histamine, serotonin, $\beta$-hexosaminidase, $\beta$-glucuronidase, tryptase, chymase, carboxypeptidase, etc.) in the culture supernatant, and comparing the amounts. The amounts of granule contents can be quantified by known methods, e.g., EIA, RIA, HPLC, enzyme activity measurement, etc. Alternatively, mast cells may be visualized (e.g., a video equipment, etc.) to count the degranulated cells.

(6b) The degranulation inhibitor (compound having the degranulation inhibitory action, etc.) is screened by contacting the ligand of the present invention with mast cells, in which radioactively labeled granule contents are incorporated, in the presence and absence of a test compound, assaying the radioactivities in the culture supernatant, and comparing the radioactivities.

(6c) The degranulation inhibitor (compound having the degranulation inhibitory action, etc.) is screened by contacting the ligand of the present invention with mast cells in the presence and absence of a test compound, assaying the binding amounts of annexin V to the mast cells (e.g., FACS, etc.), and comparing the amounts.

(7) The mast cell activation inhibitor (e.g., the compound that has the mast cell activation inhibitory action, including the MAP kinase activation inhibitor, etc.) is screened by contacting the ligand of the present invention with mast cells in the presence or absence of a test compound, separating the cell lysate using polyacrylamide gel, transferring the gel to a filter, detecting the MAP kinase activities by an image analyzer, etc. using a MAP kinase-specific antibody and chemiluminescent reagents, and comparing the intensities of chemiluminescence.

(8) The mast cell growth inhibitor (compound having the mast cell growth inhibitory action, etc.) is screened by contacting the ligand of the present invention with mast cells (e.g., mast cells plated on a 24-well plate and then incubated) in the presence or absence of a test compound, adding radioactively labeled thymidine (e.g., [methyl-$^3$H]-thymidine, etc.), lysing the cells, counting the radioactivities of thymidine taken up into the cells with a liquid scintillation counter, assaying the thymidine uptake activities and comparing the activities.

(9) The mast cell growth inhibitor (compound having the mast cell growth inhibitory action) is screened by contacting the ligand of the present invention with mast cells (e.g., mast cells plated on a 24-well plate and then incubated) in the presence or absence of a test compound, adding MTT (3-(4,5-dimethyl -2-thiazolyl)-2,5-diphenyl-2H-tetrazolium bromide), lysing the cells in an aqueous isopropanol solution rendered acidic with hydrochloric acid, measuring the amounts of MTT formazan changed from the MTT taken up into the cells by absorption at 570 nm, and comparing the amounts.

The kit for screening the compound or its salt that changes the binding properties of the ligand of the present invention to the receptor of the present invention comprises the receptor of the present invention or the cell or cell membrane fraction containing the receptor of the present invention, and the ligand of the present invention.
Examples of the screening kits of the present invention are as follow.

1. Reagents for screening

(i) Assay buffer and wash buffer

Hanks' balanced salt solution (manufactured by Gibco Co.) supplemented with 0.05% bovine serum albumin (manufactured by Sigma Co.).
The solution is sterilized by filtration through a 0.45 $\mu$m filter, and stored at 4°C or may be prepared at use.

(ii) Receptor preparation of the present invention

CHO cells where the receptor of the present invention is expressed are subcultured on a 12-well plate at a density of 5 x $10^5$ cells/well and cultured at 37°C under 5% $CO_2$ and 95% air for 2 days.

(iii) Labeled ligand

The ligand of the present invention labeled with radioisotope such as [$^3$H], [$^{125}$I], [$^{14}$C], [$^{32}$P], [$^{33}$P], [$^{35}$S], etc. A solution of the ligand dissolved in an appropriate solvent or buffer is stored at 4°C or -20°C and upon use, diluted to 1 μM with the assay buffer.

(iv) Standard ligand solution

The ligand of the present invention is dissolved in PBS containing 0.1% bovine serum albumin (manufactured by Sigma Co.) in a volume of 1 mM, and the solution is stored at -20°C.

2. Assay method

(i) The cells where the receptor of the present invention is expressed are cultured on a 12-well culture plate. After washing twice with 1 ml of the assay buffer, 490 μl of the assay buffer is added to each well.
(ii) After 5 μl of a solution of test compound in $10^{-3}$ to $10^{-10}$ M is added, 5 μl of a labeled form of the ligand of the present invention is added thereto. The reaction is carried out at room temperature for an hour. To examine the non-specific binding, 5 μl of the ligand of the present invention of $10^{-3}$ M is previously added in place of the test compound.
(iii) The reaction solution is removed and the wells are washed 3 times with 1 ml of the wash buffer. The labeled ligand of the present invention bound to the cells is dissolved in 0.2N NaOH-1 % SDS, and mixed with 4 ml of liquid scintillator A (manufactured by Wako Pure Chemical Industries, Ltd.).
(iv) The radioactivity is measured using a liquid scintillation counter (manufactured by Beckman Co.), and the percent maximum binding (PMB) is calculated in accordance with the following equation.

$$PMB = [(B - NSB)/(B_0 - NSB)] \times 100$$

PMB : Percent maximum binding
B : Value obtained in the presence of a test compound
NSB : Non-specific binding
Bo : Maximum binding

The compound or its salt, which is obtainable by using the screening methods or the screening kits of the present invention, is the compound that changes the binding of the ligand of the present invention to the receptor of the present invention, or the compound that promotes or inhibits the activity of the receptor of the present invention and specifically, includes (i) the compound or its salt having the cell stimulating activities mediated by the receptor of the present invention (the agonist to the receptor of the present invention); (ii) the compound having no stimulating activity (the antagonist to the receptor of the present invention); (iii) the compound that promotes the binding affinity of the receptor of the present invention and the ligand of the present invention; (iv) the compound that inhibits the binding affinity of the receptor of the present invention and the ligand of the present invention; or the like. Examples of these compounds include those selected from peptides, proteins, non-peptide compounds, synthetic compounds, fermentation products, cell extracts, plant extracts, animal tissue extracts, blood plasma, etc. These compounds may be novel or publicly known compounds.
The same salts given for the receptor of the present invention above apply to the salts of these compounds.
Evaluation of whether the compound is an agonist or antagonist to the receptor of the present invention described above is determined by, e.g., i) or ii) below.

i) The binding assay according to the screening methods (i) to (iii) is performed to obtain the compound that changes the binding properties of the ligand of the present invention to the receptor of the present invention (especially inhibits the binding). It is then determined if the compound has the cell stimulating activities mediated by the receptor of the present invention as described above. The compound or its salt that has the cell-stimulating activities is the receptor agonist of the present invention (agonist), whereas the compound having no such activities or its salt is the receptor antagonist of the present invention (antagonist).

ii) (a) A test compound is brought in contact with cells containing the receptor of the present invention to assay the cell stimulating activities mediated by the receptor of the present invention. The compound or its salts having the cell stimulating activities is the receptor agonist of the present invention.

(b) The cell stimulating activities mediated by the receptor of the present invention are assayed when the ligand of the present invention is brought in contact with the cell containing the receptor of the present invention and when the ligand of the present invention and a test compound are brought in contact with the cell containing the receptor of the present invention, and comparison is made on the cell stimulating activities. The compound or its salt capable of reducing the cell stimulating activities by the compound that activates the receptor of the present invention is the receptor antagonist of the present invention.

The receptor antagonist of the present invention can suppress the physiological activities (e.g., the mast cell degranulation promoting action, eicosanoid production promoting action, cytokine production promoting action, mast cell growth promoting action, mast cell activating action, etc.) possessed by the receptor of the present invention or the ligand of the present invention and can be used as a low-toxic, safe and excellent mast cell degranulation inhibitor, eicosanoid production inhibitor, cytokine production inhibitor, mast cell growth inhibitor, mast cell activation inhibitor as an agent for preventing/treating, for example, immune disorders [e.g., inflammatory disorders (pituitary tumor, thyroiditis, peritonitis, Crohn's disease, ulcerative colitis, erythema nodosum, chronic articular rheumatism, systemic lupus erythematosus, etc.), allergy (e.g., allergic conjunctivitis, allergic rhinitis, pollinosis, metal allergy, etc.), asthma, secretory otitis media, Meniere's disease, contact dermatitis, anaphylaxis, urticaria, myasthenia gravis, glomerulonephritis, Sjögren's syndrome, Basedow's disease, insulin resistant diabetes, atopic dermatitis, leukocyte abnormality, etc.], urinary tract disorders (e.g., renal tubulointerstitial injury (fibril formation), interstitial cystitis, allergic cystitis, etc.), digestive disorders [e.g., irritable bowel syndrome, chronic liver disease, food allergy, allergic enteritis, milk protein-induced proctitis, digestive ulcer (e.g., gastric ulcer, duodenal ulcer, marginal ulcer, Zollinger-Ellison syndrome, etc.), gastritis, reflux esophagitis, NUD (non-ulcer dyspepsia), gastric MALT lymphoma, nonsteroidal anti-inflammatory drug-induced ulcer, acid indigestion, postoperative stress-induced hyperacidity and ulcer, etc.], respiratory disorders [e.g., chronic obstructive pulmonary disease (e.g., chronic bronchitis, pulmonary emphysema), diffuse panbronchiolitis, cystic fibrosis, hypersensitivity penumonitis, idiopathic interstitial pneumonia, pulmonary fibrosis, etc.], circulatory disorders (e.g., arteriosclerosis, acute coronary syndrome, atherosclerotic aortic aneurysm, cardiac anaphylaxis, heart failure, myocardial infarction, angina pectoris, arrhythmia, deep vein thrombosis, restenosis after PTCA, etc.), ophthalmic disorders (e.g., pterygium, vernal conjunctivitis, dry eye, etc.), cancer (e.g., papillary thyroid carcinoma, non-small-cell lung cancer, endometrial cancer, cervical cancer, gastric cancer, pancreatic cancer, lung cancer, renal cancer, hepatic cancer, ovarian cancer, prostate cancer, bladder cancer, breast cancer, colon cancer, rectal cancer, Kaposi's sarcoma, mastocytoma, etc.), cerebral infarction, hyperlipemia, acute renal failure, diabetes mellitus, obesity, edema, granuloma, atopic myelitis, neurofibroma, nasal mucosa hypersensitivity, Hodgkin's disease, endometrial hyperplasia, etc., or the like.

The compound that inhibits the binding affinity of the receptor of the present invention to the ligand of the present invention is used as in the receptor antagonist of the present invention.

The compound that promotes the binding affinity of the receptor of the present invention to the ligand of the present invention is used as in the receptor agonist of the present invention.

In addition, the present invention provides the method of screening the compound or its salt that promotes or inhibits the expression of a gene for the receptor of the present invention, which comprises using the polynucleotide of the present invention encoding the receptor of the present invention, etc.

Specifically, the compound or its salt that promotes or inhibits the expression of a gene for the receptor of the present invention is screened by comparing the case (i) where a cell capable of producing the receptor of the present invention is cultured, with the case (ii) where a mixture of the cell capable of producing the receptor of the present invention and a test compound is cultured.

In the screening method described above, the expression level of the receptor gene of the present invention (specifically, the amount of the receptor of the present invention or the amount of mRNA encoding the receptor of the present invention, etc.) is measured in the cases (i) and (ii), and comparison is made.

Examples of the test compound include peptides, proteins, antibodies, non-peptide compounds, synthetic compounds, fermentation products, cell extracts, plant extracts, animal tissue extracts, blood plasma, and the like. These compounds may be novel or publicly known compounds.

To perform the screening method described above, the cells capable of producing the polypeptide of the present invention or the receptor of the present invention are suspended in a buffer suitable for the screening, and the suspension is prepared. Any buffer can be used so long as it does not interfere the activities of the receptor of the present invention, including a phosphate buffer or a borate buffer, having pH of about 4 to about 10 (preferably pH of about 6 to about 8), etc.

As the cells capable of producing the receptor of the present invention, there are used, for example, a host (transformant) transformed with a vector containing the DNA encoding the receptor of the present invention. Preferably, animal cells such as CHO cells, etc. are used as the host. For the screening, the transformant, in which the receptor of the present invention has been secreted extracellularly by culturing through the procedures described above, is preferably employed.

The protein level of the receptor of the present invention can be determined by publicly known methods, e.g., by measuring the polypeptide or receptor present in the cell extract, etc., using an antibody of the present invention, in accordance with methods like western blot analysis, ELISA, etc., or their modifications.

The expression level of the gene for the receptor of the present invention can be determined by publicly known methods, e.g., in accordance with methods including Northern blotting, reverse transcription-polymerase chain reaction (RT-PCR), real time PCR monitoring system (manufactured by ABI, TaqMan polymerase chain reaction), etc., or their modifications.

For example, when a test compound promotes the expression of a gene for the receptor in the case (ii) described above by at least about 20%, preferably at least 30% and more preferably at least about 50%, as compared to the case (i) above, the test compound can be selected as the compound or its salts that promote the expression of the gene for the receptor of the present invention.

For example, when a test compound inhibits the expression of the gene for the receptor of the present invention in the case (ii) described above by at least about 20%, preferably at least 30% and more preferably at least about 50%, as compared to the case (i) above, the test compound can be selected to be the compound or its salts that inhibit the expression of the gene for the receptor of the present invention.

The compound or its salt that inhibits the expression of a gene for the receptor of the present invention is used as in the receptor antagonist of the present invention.

The compound or its salt that promotes the expression (increases the expression level) of a gene for the receptor of the present invention is used as in the receptor agonist of the present invention.

The compound or its salt, which is obtained using the screening method or screening kit of the present invention, is a compound selected from, for example, peptides, proteins, non-peptide compounds, synthetic compounds, fermentation products, cell extracts, plant extracts, animal tissue extracts, blood plasma, etc. and is the compound that changes the binding properties of the receptor of the present invention to the ligand of the present invention, the compound that promotes or inhibits the activities or functions of the receptor of the present invention, the compound that promotes or inhibits the expression (increase or decrease the expression level) of a gene for the receptor of the present invention, etc.

The same examples given as the salts of the receptor of the present invention described above apply to the salts of the compounds described above.

When the compound or its salts obtained by the screening methods or kits of the present invention are used as he aforesaid medicaments (as prophylactic/therapeutic agents, etc.), the use can be performed in a conventional manner.

The compound or its salt can be administered orally, for example, in the form of tablets which may be sugar coated, if necessary, capsules, elixirs, microcapsules etc., or parenterally in the form of injections such as sterile solutions or suspensions in water or in pharmaceutically acceptable solutions other than water. For example, the compound or its salts can be mixed with carriers, flavoring agents, excipients, vehicles, preservatives, stabilizers, binders, etc. in a unit dosage form generally accepted. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, a flavoring agent such as peppermint, akamono oil and cherry, etc. When the unit dosage is in the form of a capsule, liquid carriers such as oils and fats may further be used together with the additives described above. A sterile composition for injection may be formulated in a conventional manner used to make pharmaceutical preparations, e.g., by dissolving or suspending the active ingredients in a vehicle such as water for injection with a naturally occurring vegetable oil such as sesame oil and coconut oil, etc. to prepare the pharmaceutical preparations.

Examples of an aqueous medium for injection include physiological saline and an isotonic solution containing glucose and other auxiliary agents (e.g., D-sorbitol, D-mannitol, sodium chloride, etc.), etc. and may be used in combination with an appropriate dissolution aid such as an alcohol (e.g., ethanol, etc.), a polyalcohol (e.g., propylene glycol and polyethylene glycol, etc.), a nonionic surfactant (e.g., polysorbate 80™, HCO-50, etc.), etc. Examples of the oily medium include sesame oil, soybean oil, etc., which may also be used in combination with a dissolution aid such as benzyl benzoate, benzyl alcohol, etc. The compound or its salt may further be formulated together with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

Since the pharmaceutical preparation thus obtained is safe and low toxic, it can be administered to human or other warm-blooded animal (e.g., mouse, rat, rabbit, sheep, swine, bovine, horse, fowl, cat, dog, monkey, chimpanzee, etc.).

The dose of the compound or its salt may vary depending upon the action, target disease, subject to be administered, route of administration, etc.

For example, in oral administration, the antagonist is administered to the patient (as 60 kg body weight) with, e.g., interstitial cystitis normally in a dose of about 0.1 to about 100 mg, preferably about 1.0 to about 50 mg, more preferably about 1.0 to about 20 mg per day. When the antagonist is parenterally administered to the patient (as 60 kg body weight) with, e.g., interstitial cystitis in the form of an injection, it is advantageous to administer the antagonist intravenously at a daily dose of about 0.01 to about 30 mg, preferably about 0.1 to about 20 mg, more preferably about 0.1 to about 10 mg. For other animal species, the corresponding dose as converted per 60 kg weight can be administered.

[2] Quantification of the receptor of the present invention

The antibody against the receptor of the present invention (hereinafter sometimes briefly referred to as the antibody of the present invention) can specifically recognize the receptor of the present invention. Therefore, the antibody can be used to quantify the receptor of the present invention in a test fluid, especially for quantification by the sandwich immunoassay, etc.

That is, the present invention provides, for example, the following methods of quantification:

(i) a method of quantifying the receptor of the present invention in a test fluid, which comprises competitively reacting the antibody of the present invention with the test fluid and a labeled form of the receptor of the present invention, and measuring the ratio of the labeled receptor of the present invention bound to the antibody; and,
(ii) a method of quantifying the receptor of the present invention in a test fluid, which comprises reacting the test fluid with the antibody of the present invention immobilized on a carrier and a labeled form of the antibody of the present invention simultaneously or sequentially, and measuring the activity of the label on the immobilized carrier.

In the quantifying method (ii) described above, it is preferred that one antibody is capable of recognizing the N-terminal region in the receptor of the present invention, while another antibody is capable of reacting with the C-terminal region in the receptor of the present invention.

Using a monoclonal antibody against the receptor of the present invention, the receptor of the present invention can be assayed and can further be detected by tissue staining, or the like. For these purposes, the antibody molecule itself may be used, or $F(ab')_2$, Fab' or Fab fractions of the antibody molecule may be used as well.

The method of quantifying the receptor of the present invention using the antibody of the present invention is not particularly limited, and any method may be used, so long as the amount of antibody, antigen, or antibody-antigen complex in response to the amount of antigen (e.g., the amount of the polypeptide) in a test fluid can be detected by chemical or physical means and can be calculated from a standard curve prepared from standard solutions containing known amounts of the antigen. Advantageously used are, for example, nephrometry, competitive method, immunometric method and sandwich method; in terms of sensitivity and specificity, the sandwich method, which will be described later, is particularly preferred.

Examples of labeling agents, which are employed for the assay method using the same are radioisotopes, enzymes, fluorescent substances, luminescent substances, etc. Examples of the radioisotopes employed are [$^{125}I$], [$^{131}I$], [$^{3}H$], [$^{14}C$], etc. As the enzymes described above, stable enzymes with a high specific activity are preferred; for example, β-galactosidase, β-glucosidase, alkaline phosphatase, peroxidase, malate dehydrogenase and the like are used. Examples of the fluorescent substance used are fluorescamine, fluorescein isothiocyanate and the like. As the luminescent substances, there are employed, for example, luminol, luminol derivatives, luciferin, lucigenin and the like. Furthermore, the biotin-avidin system may also be used for binding an antibody or antigen to the label.

In the immobilization of antigens or antibodies, physical adsorption may be used. Alternatively, chemical binding that is conventionally used for immobilization of polypeptides, enzymes, etc. may be used as well. Examples of the carrier include insoluble polysaccharides such as agarose, dextran, cellulose, etc.; synthetic resins such as polystyrene, polyacrylamide, silicone, etc.; or glass; and the like.

In the sandwich method, the immobilized monoclonal antibody of the present invention is reacted with a test fluid (primary reaction), then with a labeled form of another monoclonal antibody of the present invention (secondary reaction), and the activity of the labeling agent on the immobilizing carrier is assayed, whereby the amount of the receptor in the test fluid can be quantified. The order of the primary and secondary reactions may be reversed, and the reactions may be performed simultaneously or with some time intervals. The methods of labeling and immobilization can be performed by modifications of those methods described above. In the immunoassay by the sandwich method, the antibody used for immobilized or labeled antibody is not necessarily from one species, but a mixture of two or more species of antibodies may be used to increase the measurement sensitivity.

In the methods of assaying the receptor of the present invention by the sandwich method, antibodies that bind to different sites of the receptor of the present invention are preferably used as the monoclonal antibodies of the present invention for the primary and secondary reactions. That is, in the antibodies used for the primary and secondary reactions, for example, when the antibody used in the secondary reaction recognizes the C-terminal region of the receptor of the present invention, it is preferable to use the antibody capable of recognizing the region other than the C-terminal region for the primary reaction, e.g., the antibody capable of recognizing the N-terminal region.

The monoclonal antibody of the present invention can be used for the assay systems other than the sandwich method, for example, the competitive method, immunometric method, nephrometry, etc.

In the competitive method, an antigen in a test fluid and a labeled antigen are competitively reacted with an antibody, and the unreacted labeled antigen (F) and the labeled antigen bound to the antibody (B) are separated (B/F separation). The amount of the labeling agent in B or F is measured, and the amount of the antigen in the test fluid is quantified. This reaction method includes a liquid phase method using a soluble antibody as an antibody, polyethylene glycol for B/F separation and a secondary antibody, etc. to the soluble antibody, and an immobilized method either using an immobilized antibody as the primary antibody, or using a soluble antibody as the primary antibody and an immobilized antibody as the secondary antibody.

In the immunometric method, an antigen in a test fluid and an immobilized antigen are competitively reacted with a definite amount of labeled antibody, the immobilized phase is separated from the liquid phase, or an antigen in a test fluid is reacted with an excess amount of labeled antibody, the immobilized antigen is then added to bind the unreacted labeled antibody against the immobilized phase, and the immobilized phase is separated from the liquid phase. Then, the amount of the labeling agent in either phase is measured to quantify the antigen in the test fluid.

In the nephrometry, insoluble precipitates produced after the antigen-antibody reaction in a gel or solution are quantified. Even when the amount of an antigen in a test fluid is small and only a small amount of precipitates is obtained, laser nephrometry using scattering of laser can be advantageously employed.

For applying these immunological assay methods to the quantification methods of the present invention, any particular conditions or procedures are not required. The assay systems for the receptor of the present invention may be constructed by adding ordinary technical consideration in the art to conventional conditions and procedures in the respective methods. For the details of these general technical means, reference can be made to the following reviews and texts.

For example, reference can be made on Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (all published by Academic Press Publishing), etc.

As described above, the receptor of the present invention can be quantified with high sensitivity, by using the antibody of the present invention.

Further when an increased level of the receptor of the present invention is detected by quantifying the level of the receptor of the present invention using the antibody of the present invention, for example, the mast cell degranulation promoting action, eicosanoid production promoting action, cytokine production promoting action, mast cell growth promoting action, mast cell activating action, etc.) is enhanced. Then it can be diagnosed that it is highly likely to suffer from diseases in the future, such as immune disorders [e.g., inflammatory disorders (pituitary tumor, thyroiditis, peritonitis, Crohn's disease, ulcerative colitis, erythema nodosum, chronic articular rheumatism, systemic lupus erythematosus, etc.), allergy (e.g., allergic conjunctivitis, allergic rhinitis, pollinosis, metal allergy, etc.), asthma, secretory otitis media, Meniere's disease, contact dermatitis, anaphylaxis, urticaria, myasthenia gravis, glomerulonephritis, Sjögren's syndrome, Basedow's disease, insulin resistant diabetes, atopic dermatitis, leukocyte abnormality, etc.], urinary tract disorders (e.g., renal tubulointerstitial injury (fibril formation), interstitial cystitis, allergic cystitis, etc.), digestive disorders [e.g., irritable bowel syndrome, chronic liver disease, food allergy, allergic enteritis, milk protein-induced proctitis, digestive ulcer (e.g., gastric ulcer, duodenal ulcer, marginal ulcer, Zollinger-Ellison syndrome, etc.), gastritis, reflux esophagitis, NUD (non-ulcer dyspepsia), gastric MALT lymphoma, nonsteroidal anti-inflammatory drug-induced ulcer, acid indigestion, postoperative stress-induced hyperacidity and ulcer, etc.], respiratory disorders [e.g., chronic obstructive pulmonary disease (e.g., chronic bronchitis, pulmonary emphysema), diffuse panbronchiolitis, cystic fibrosis, hypersensitivity penumonitis, idiopathic interstitial pneumonia, pulmonary fibrosis, etc.], circulatory disorders (e.g., arteriosclerosis, acute coronary syndrome, atherosclerotic aortic aneurysm, cardiac anaphylaxis, heart failure, myocardial infarction, angina pectoris, arrhythmia, deep vein thrombosis, restenosis after PTCA, etc.), ophthalmic disorders (e.g., pterygium, vernal conjunctivitis, dry eye, etc.), cancer (e.g., papillary thyroid carcinoma, non-small-cell lung cancer, endometrial cancer, cervical cancer, gastric cancer, pancreatic cancer, lung cancer, renal cancer, hepatic cancer, ovarian cancer, prostate cancer, bladder cancer, breast cancer, colon cancer, rectal cancer, Kaposi's sarcoma, mastocytoma, etc.), cerebral infarction, hyperlipemia, acute renal failure, diabetes mellitus, obesity, edema, granuloma, atopic myelitis, neurofibroma, nasal mucosa hypersensitivity, Hodgkin's disease, endometrial hyperplasia, etc., or the like.

Besides, the antibody of the present invention can be used for detecting the receptor of the present invention present in test samples such as body fluids, tissues, etc. The antibody can also be used for preparation of antibody columns used to purify the receptor of the present invention, for detection of the receptor of the present invention in each fraction upon purification, for analysis of the behavior of the receptor of the present invention in test cells; etc.

[3] Gene diagnostic agent

By using the polynucleotide (DNA) of the present invention, e.g., as a probe, an abnormality (gene abnormality) of the DNA or mRNA encoding the receptor of the present invention in human or other warm-blooded animal (e.g., rat, mouse, guinea pig, rabbit, fowl, sheep, swine, bovine, horse, cat, dog, monkey, etc.) can be detected. Therefore, the polynucleotide (DNA) of the present invention is useful as a gene diagnostic agent for damages to the DNA or mRNA, its mutation or decreased expression or increased expression, or overexpression of the DNA or mRNA.

The gene diagnosis described above using the DNA of the present invention can be performed by, for example, publicly known Northern hybridization or PCR-SSCP assay (Genomics, 5, 874-879 (1989); Proceedings of the National Academy of Sciences of the United States of America, 86, 2766-2770 (1989)), etc.

When overexpression of the receptor of the present invention is detected, for example, the mast cell degranulation-inhibitory action, eicosanoid production inhibitory action, cytokine production inhibitory action, mast cell growth inhibitory action or mast cell activation inhibitory action is enhanced. Then, it can be diagnosed that it is highly likely to suffer from diseases in the future, such as immune disorders [e.g., inflammatory disorders (pituitary tumor, thyroiditis, peritonitis, Crohn's disease, ulcerative colitis, erythema nodosum, chronic articular rheumatism, systemic lupus erythematosus, etc.), allergy (e.g., allergic conjunctivitis, allergic rhinitis, pollinosis, metal allergy, etc.), asthma, secretory otitis media, Meniere's disease, contact dermatitis, anaphylaxis, urticaria, myasthenia gravis, glomerulonephritis, Sjögren's syndrome, Basedow's disease, insulin resistant diabetes, atopic dermatitis, leukocyte abnormality, etc.], urinary tract disorders (e.g., renal tubulointerstitial injury (fibril formation), interstitial cystitis, allergic cystitis, etc.), digestive disorders [e.g., irritable bowel syndrome, chronic liver disease, food allergy, allergic enteritis, milk protein-induced proctitis, digestive ulcer (e.g., gastric ulcer, duodenal ulcer, marginal ulcer, Zollinger-Ellison syndrome, etc.), gastritis, reflux esophagitis, NUD (non-ulcer dyspepsia), gastric MALT lymphoma, nonsteroidal anti-inflammatory drug-induced ulcer, acid indigestion, postoperative stress-induced hyperacidity and ulcer, etc.], respiratory disorders [e.g., chronic obstructive pulmonary disease (e.g., chronic bronchitis, pulmonary emphysema), diffuse panbronchiolitis, cystic fibrosis, hypersensitivity penumonitis, idiopathic interstitial pneumonia, pulmonary fibrosis, etc.], circulatory disorders (e.g., arteriosclerosis, acute coronary syndrome, atherosclerotic aortic aneurysm, cardiac anaphylaxis, heart failure, myocardial infarction, angina pectoris, arrhythmia, deep vein thrombosis, restenosis after PTCA, etc.), ophthalmic disorders (e.g., pterygium, vernal conjunctivitis, dry eye, etc.), cancer (e.g., papillary thyroid carcinoma, non-small-cell lung cancer, endometrial cancer, cervical cancer, gastric cancer, pancreatic cancer, lung cancer, renal cancer, hepatic cancer, ovarian cancer, prostate cancer, bladder cancer, breast cancer, colon cancer, rectal cancer, Kaposi's sarcoma, mastocytoma, etc.), cerebral infarction, hyperlipemia, acute renal failure, diabetes mellitus, obesity, edema, granuloma, atopic myelitis, neurofibroma, nasal mucosa hypersensitivity, Hodgkin's disease, endometrial hyperplasia, etc., or the like.

[4] Pharmaceuticals comprising antisense polynucleotide (e.g., DNA)

The antisense polynucleotide (e.g., antisense DNA) that can bind complementarily to the polynucleotide (e.g., DNA) of the present invention to suppress the expression of the polynucleotide (e.g., DNA) is useful as, e.g., the mast cell degranulation-inhibitor, eicosanoid production inhibitor, cytokine production inhibitor, mast cell growth inhibitor, mast cell activation inhibitor (including, e.g., a MAPK activation inhibitor, etc.), etc., for a low toxic and safe medicament such as an agent for preventing/treating, for example, immune disorders [e.g., inflammatory disorders (pituitary tumor, thyroiditis, peritonitis, Crohn's disease, ulcerative colitis, erythema nodosum, chronic articular rheumatism, systemic lupus erythematosus, etc.), allergy (e.g., allergic conjunctivitis, allergic rhinitis, pollinosis, metal allergy, etc.), asthma, secretory otitis media, Meniere's disease, contact dermatitis, anaphylaxis, urticaria, myasthenia gravis, glomerulonephritis, Sjögren's syndrome, Basedow's disease, insulin resistant diabetes, atopic dermatitis, leukocyte abnormality, etc.], urinary tract disorders (e.g., renal tubulointerstitial injury (fibril formation), interstitial cystitis, allergic cystitis, etc.), digestive disorders [e.g., irritable bowel syndrome, chronic liver disease, food allergy, allergic enteritis, milk protein-induced proctitis, digestive ulcer (e.g., gastric ulcer, duodenal ulcer, marginal ulcer, Zollinger-Ellison syndrome, etc.), gastritis, reflux esophagitis, NUD (non-ulcer dyspepsia), gastric MALT lymphoma, nonsteroidal anti-inflammatory drug-induced ulcer, acid indigestion, postoperative stress-induced hyperacidity and ulcer, etc.], respiratory disorders [e.g., chronic obstructive pulmonary disease (e.g., chronic bronchitis, pulmonary emphysema), diffuse panbronchiolitis, cystic fibrosis, hypersensitivity penumonitis, idiopathic interstitial pneumonia, pulmonary fibrosis, etc.], circulatory disorders (e.g., arteriosclerosis, acute coronary syndrome, atherosclerotic aortic aneurysm, cardiac anaphylaxis, heart failure, myocardial infarction, angina pectoris, arrhythmia, deep vein thrombosis, restenosis after PTCA, etc.), ophthalmic disorders (e.g., pterygium, vernal conjunctivitis, dry eye, etc.), cancer (e.g., papillary thyroid carcinoma, non-small-cell lung cancer, endometrial cancer, cervical cancer, gastric cancer, pancreatic cancer, lung cancer, renal cancer, hepatic cancer, ovarian cancer, prostate cancer, bladder cancer, breast cancer, colon cancer, rectal cancer, Kaposi's sarcoma, mastocytoma, etc.), cerebral infarction, hyperlipemia, acute renal failure, diabetes mellitus, obesity, edema, granuloma, atopic myelitis, neurofibroma, nasal mucosa hypersensitivity, Hodgkin's disease, endometrial hyperplasia, etc., or the like.

For example, the antisense DNA is administered solely, or the antisense DNA is inserted into an appropriate vector such as retrovirus vector, adenovirus vector, adenovirus-associated virus vector, etc., which is then administered in a conventional manner. The antisense DNA may be administered in an intact form, or may be prepared into a dosage form together with a physiologically acceptable carrier to increase its uptake and administered by gene gun or through a catheter such as a catheter with a hydrogel.

In addition, the antisense DNA may also be employed as an oligonucleotide probe for diagnosis to examine the presence of the DNA of the present invention in tissues or cells and the conditions of its expression.

As in the antisense polynucleotide described above, the double-stranded RNA [e.g., siRNA (small (short) interfering RNA), shRNA (small (short) hairpin RNA) to the receptor of the present invention] containing a part of the RNA encoding the receptor of the present invention, the ribozyme containing a part of the RNA encoding the receptor of the present invention, etc., can also suppress the expression of the polynucleotide of the present invention and can suppress the in vivo the functions of the receptor of the present invention or the polynucleotide of the present invention. Therefore, they are useful as, e.g., the mast cell degranulation-inhibitor, eicosanoid production inhibitor, cytokine production inhibitor, mast cell growth inhibitor, mast cell activation inhibitor (including, e.g., a MAPK activation inhibitor, etc.), etc., for low toxic and safe medicaments such as agents for preventing/treating, for example, immune disorders [e.g., inflammatory disorders (pituitary tumor, thyroiditis, peritonitis, Crohn's disease, ulcerative colitis, erythema nodosum, chronic articular rheumatism, systemic lupus erythematosus, etc.), allergy (e.g., allergic conjunctivitis, allergic rhinitis, pollinosis, metal allergy, etc.), asthma, secretory otitis media, Meniere's disease, contact dermatitis, anaphylaxis, urticaria, myasthenia gravis, glomerulonephritis, Sjögren's syndrome, Basedow's disease, insulin resistant diabetes, atopic dermatitis, leukocyte abnormality, etc.], urinary tract disorders (e.g., renal tubulointerstitial injury (fibril formation), interstitial cystitis, allergic cystitis, etc.), digestive disorders [e.g., irritable bowel syndrome, chronic liver disease, food allergy, allergic enteritis, milk protein-induced proctitis, digestive ulcer (e.g., gastric ulcer, duodenal ulcer, marginal ulcer, Zollinger-Ellison syndrome, etc.), gastritis, reflux esophagitis, NUD (non-ulcer dyspepsia), gastric MALT lymphoma, nonsteroidal anti-inflammatory drug-induced ulcer, acid indigestion, postoperative stress-induced hyperacidity and ulcer, etc.], respiratory disorders [e.g., chronic obstructive pulmonary disease (e.g., chronic bronchitis, pulmonary emphysema), diffuse panbronchiolitis, cystic fibrosis, hypersensitivity penumonitis, idiopathic interstitial pneumonia, pulmonary fibrosis, etc.], circulatory disorders (e.g., arteriosclerosis, acute coronary syndrome, atherosclerotic aortic aneurysm, cardiac anaphylaxis, heart failure, myocardial infarction, angina pectoris, arrhythmia, deep vein thrombosis, restenosis after PTCA, etc.), ophthalmic disorders (e.g., pterygium, vernal conjunctivitis, dry eye, etc.), cancer (e.g., papillary thyroid carcinoma, non-small-cell lung cancer, endometrial cancer, cervical cancer, gastric cancer, pancreatic cancer, lung cancer, renal cancer, hepatic cancer, ovarian cancer, prostate cancer, bladder cancer, breast cancer, colon cancer, rectal cancer, Kaposi's sarcoma, mastocytoma, etc.), cerebral infarction, hyperlipemia, acute renal failure, diabetes mellitus, obesity, edema, granuloma, atopic myelitis, neurofibroma, nasal mucosa hypersensitivity, Hodgkin's disease, endometrial hyperplasia, etc., or the like.

The double-stranded RNA can be manufactured by designing the same based on the sequence of the polynucleotide of the present invention, by publicly known methods (e.g., Nature, 411, 494, 2001) with a modification.

The ribozyme can be manufactured by designing the same based on the sequence of the polynucleotide of the present invention, by a modification of publicly known methods (e.g., TRENDS in Molecular Medicine, 7, 221, 2001). For example, the ribozyme designing the same based on the sequence of the polynucleotide of the present invention, can be manufactured by ligating a publicly known ribozyme to a part of the RNA encoding the receptor of the present invention. The part of the RNA encoding the receptor of the present invention includes a contiguous part (RNA fragment) to the cleavage site on the RNA of the present invention, which can be cleaved by a publicly known ribozyme.

Where the double-stranded RNA or ribozyme described above is used as the agent for the prevention/treatment described above, the RNA or ribozyme can be prepared into pharmaceutical preparations, which are provided for administration, as in the antisense polynucleotide.

[5] Medicament comprising the antibody of the present invention

The antibody against the receptor of the present invention (e.g., an antibody having the action of neutralizing the receptor of the present invention, an antibody inactivating signal transduction, etc.) is useful as, e.g., the mast cell degranulation-inhibitor, eicosanoid production inhibitor, cytokine production inhibitor, mast cell growth inhibitor, mast cell activation inhibitor (including, e.g., a MAPK activation inhibitor, etc.), etc., for a low toxic and safe medicament such as an agent for preventing/treating, for example, immune disorders [e.g., inflammatory disorders (pituitary tumor, thyroiditis, peritonitis, Crohn's disease, ulcerative colitis, erythema nodosum, chronic articular rheumatism, systemic lupus erythematosus, etc.), allergy (e.g., allergic conjunctivitis, allergic rhinitis, pollinosis, metal allergy, etc.), asthma, secretory otitis media, Meniere's disease, contact dermatitis, anaphylaxis, urticaria, myasthenia gravis, glomerulonephritis, Sjögren's syndrome, Basedow's disease, insulin resistant diabetes, atopic dermatitis, leukocyte abnormality, etc.], urinary tract disorders (e.g., renal tubulointerstitial injury (fibril formation), interstitial cystitis, allergic cystitis, etc.), digestive disorders [e.g., irritable bowel syndrome, chronic liver disease, food allergy, allergic enteritis, milk protein-induced proctitis, digestive ulcer (e.g., gastric ulcer, duodenal ulcer, marginal ulcer, Zollinger-Ellison syndrome, etc.), gastritis, reflux esophagitis, NUD (non-ulcer dyspepsia), gastric MALT lymphoma, nonsteroidal anti-inflammatory drug-induced ulcer, acid indigestion, postoperative stress-induced hyperacidity and ulcer, etc.], respiratory disorders [e.g., chronic obstructive pulmonary disease (e.g., chronic bronchitis, pulmonary emphysema), diffuse panbronchiolitis, cystic fibrosis, hypersensitivity penumonitis, idiopathic interstitial pneumonia, pulmonary fibrosis, etc.], circulatory disorders (e.g., arteriosclerosis, acute coronary syndrome, atherosclerotic aortic aneurysm, cardiac anaphylaxis, heart failure, myocardial infarction, angina pectoris, arrhythmia, deep vein thrombosis, restenosis after PTCA, etc.), ophthalmic disorders (e.g., pterygium, vernal conjunctivitis, dry eye, etc.), cancer (e.g., papillary thyroid carcinoma, non-small-cell lung cancer, endometrial cancer, cervical cancer, gastric cancer, pancreatic cancer, lung cancer, renal cancer, hepatic cancer, ovarian cancer, prostate cancer, bladder cancer, breast cancer, colon cancer, rectal cancer, Kaposi's sarcoma, mastocytoma, etc.), cerebral infarction, hyperlipemia, acute renal failure, diabetes mellitus, obesity, edema, granuloma, atopic myelitis, neurofibroma, nasal mucosa hypersensitivity, Hodgkin's disease, endometrial hyperplasia, etc., or the like.

The medicaments comprising the antibody of the present invention described above can be administered to human or other warm-blooded animal (e.g., rats, rabbits, sheep, swine, bovine, cats, dogs, monkeys, etc.) orally or parenterally, directly as a liquid preparation, or as a pharmaceutical composition in an appropriate preparation form. The dose may vary depending upon subject to be administered, target disease, conditions, route of administration, etc. For example, when the antibody of the present invention is used to treat/prevent interstitial cystitis in an adult patient, it is advantageous to administer the antibody in the form of intravenous injection normally in a single dose of about 0.01 to about 20 mg/kg body weight, preferably about 0.1 to about 10 mg/kg body weight, and more preferably about 0.1 to about 5 mg/kg body weight, approximately 1 to 5 times per day preferably approximately 1 to 3 times per day. In other parenteral administration and oral administration, the antibody can be administered in a dose corresponding to the dose given above. When the condition is especially severe, the dose may be increased according to the condition.

The antibody of the present invention may be administered directly as it is or as an appropriate pharmaceutical composition. The pharmaceutical composition used for the administration described above contains a pharmacologically acceptable carrier with the aforesaid compounds or salts thereof, a diluent or excipient. Such a composition is provided in the preparation suitable for oral or parenteral administration.

That is, examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, etc. These injectable preparations may be prepared by methods publicly known. The injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule. The suppository used for rectal administration may be prepared by blending the aforesaid antibody or its salt with conventional bases for suppositories.

Advantageously, the pharmaceutical compositions for oral or parenteral use described above are prepared into pharmaceutical preparations in a unit dose suited to fit a dose of the active ingredients. Such unit dose preparations include, for example, tablets, pills, capsules, injections (ampoules), suppositories, etc. The amount of the aforesaid antibody contained is generally about 5 to 500 mg per dosage unit form; especially in the form of injection, it is preferred that the aforesaid antibody is contained in about 5 to 100 mg and in about 10 to 250 mg for the other forms.

Each composition described above may further contain other active components unless formulation causes any adverse interaction with the antibody described above.

[6] DNA transgenic animal

The present invention provides a non-human mammal bearing DNA encoding the receptor of the present invention, which is exogenous (hereinafter abbreviated as the exogenous DNA of the present invention) or its variant DNA (sometimes simply referred to as the exogenous variant DNA of the present invention).

That is, the present invention provides:

(1) a non-human mammal bearing the exogenous DNA of the present invention or its variant DNA;
(2) the mammal according to (1), wherein the non-human mammal is a rodent;
(3) the mammal according to (2), wherein the rodent is mouse or rat; and,
(4) a recombinant vector containing the exogenous DNA of the present invention or its variant DNA and capable of expressing in a mammal; etc.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to prepare the DNA transgenic animal of the present invention.

Examples of the non-human mammal that can be used include bovine, swine, sheep, goat, rabbits, dogs, cats, guinea pigs, hamsters, mice, rats, etc. Above all, preferred are rodents, especially mice (e.g., C57B1/6 strain, DBA2 strain, etc. for a pure line and for a cross line, $B6C3F_1$ strain, $BDF_1$ strain $B6D2F_1$ strain, BALB/c strain, ICR strain, etc.), rats (Wistar, SD, etc.) or the like, since they are relatively short in ontogeny and life cycle from a standpoint of creating model animals for disease.

"Mammals" in a recombinant vector that can be expressed in the mammals include the aforesaid non-human mammals, human, etc.

The exogenous DNA of the present invention refers to the DNA of the present invention that is once isolated/ extracted from mammals, not the DNA of the present invention inherently possessed by the non-human mammals.

The mutant DNA of the present invention includes mutants resulting from variation (e.g., mutation, etc.) in the nucleotide sequence of the original DNA of the present invention, specifically DNAs resulting from addition or deletion of nucleotides, substitution with other nucleotides, etc. and further including abnormal DNA.

The abnormal DNA is intended to mean such a DNA that expresses the receptor of the present invention which is abnormal and exemplified by the DNA, etc. that expresses a polypeptide to suppress the functions of the receptor of the present invention which is normal.

The exogenous DNA of the present invention may be any one of those derived from a mammal of the same species as, or a different species from, the mammal as the target animal. In transfecting the DNA of the present invention into the target animal, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target mammal, e.g., a mouse fertilized egg, downstream various promoters which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the receptor of the present invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used. Examples of these promoters for regulating the DNA expression described above include (i) promoters for DNA derived from viruses (e.g., simian virus, cytomegalovirus, Moloney leukemia virus, JC virus, breast cancer virus, poliovirus, etc.), and (ii) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), polypeptide chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human polypeptide elongation factor 1α (EF-1α) promoters, human and fowl β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of enhancing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal receptor of the present invention can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal polypeptide obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by crossing.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the DNA of the present invention is excessively present in all of the germinal cells and somatic cells thereof. The offspring of the animal that inherits the exogenous DNA of the present invention have excessively the DNA of the present invention in all of the germinal cells and somatic cells thereof.

It is possible to obtain homozygotic animals having the transfected DNA in both homologous chromosomes and breed male and female of the animal so that all the progeny have this DNA in excess.

In a non-human mammal bearing the normal DNA of the present invention, the normal DNA of the present invention has expressed at a high level, and may eventually develop hyperfunction in the function of the receptor of the present invention by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the function of the receptor of the present invention and the pathological mechanism of the disease associated with the receptor of the present invention and to investigate how to treat these diseases.

Furthermore, a mammal transfected with the exogenous normal DNA of the present invention exhibits an increasing symptom of the receptor of the present invention librated, and can be used as, e.g., the mast cell degranulation-promoting animals, eicosanoid production promoting animals, cytokine production promoting animals, mast cell growth promoting animals, mast cell activating animals [e.g., including the MAPK activation (promoting) animals, etc.), etc., in screening tests of agents for preventing/treating, for example, immune disorders [e.g., inflammatory disorders (pituitary tumor, thyroiditis, peritonitis, Crohn's disease, ulcerative colitis, erythema nodosum, chronic articular rheumatism, systemic lupus erythematosus, etc.), allergy (e.g., allergic conjunctivitis, allergic rhinitis, pollinosis, metal allergy, etc.), asthma, secretory otitis media, Meniere's disease, contact dermatitis, anaphylaxis, urticaria, myasthenia gravis, glomerulonephritis, Sjögren's syndrome, Basedow's disease, insulin resistant diabetes, atopic dermatitis, leukocyte abnormality, etc.], urinary tract disorders (e.g., renal tubulointerstitial injury (fibril formation), interstitial cystitis, allergic cystitis, etc.), digestive disorders [e.g., irritable bowel syndrome, chronic liver disease, food allergy, allergic enteritis, milk protein-induced proctitis, digestive ulcer (e.g., gastric ulcer, duodenal ulcer, marginal ulcer, Zollinger-Ellison syndrome, etc.), gastritis, reflux esophagitis, NUD (non-ulcer dyspepsia), gastric MALT lymphoma, nonsteroidal anti-inflammatory drug-induced ulcer, acid indigestion, postoperative stress-induced hyperacidity and ulcer, etc.], respiratory disorders [e.g., chronic obstructive pulmonary disease (e.g., chronic bronchitis, pulmonary emphysema), diffuse panbronchiolitis, cystic fibrosis, hypersensitivity penumonitis, idiopathic interstitial pneumonia, pulmonary fibrosis, etc.], circulatory disorders (e.g., arteriosclerosis, acute coronary syndrome, atherosclerotic aortic aneurysm, cardiac anaphylaxis, heart failure, myocardial infarction, angina pectoris, arrhythmia, deep vein thrombosis, restenosis after PTCA, etc.), ophthalmic disorders (e.g., pterygium, vernal conjunctivitis, dry eye, etc.), cancer (e.g., papillary thyroid carcinoma, non-small-cell lung cancer, endometrial cancer, cervical cancer, gastric cancer, pancreatic cancer, lung cancer, renal cancer, hepatic cancer, ovarian cancer, prostate cancer, bladder cancer, breast cancer, colon cancer, rectal cancer, Kaposi's sarcoma, mastocytoma, etc.), cerebral infarction, hyperlipemia, acute renal failure, diabetes mellitus, obesity, edema, granuloma, atopic myelitis, neurofibroma, nasal mucosa hypersensitivity, Hodgkin's disease, endometrial hyperplasia, etc., or the like.

On the other hand, a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the target mammal. The fact that the abnormal DNA of the present invention is present in the germinal cells of the animal after DNA transfection means that all of the offspring of the prepared animal have the abnormal DNA of the present invention in all of the germinal and somatic cells. Such an offspring that passaged the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention has expressed to a high level, and may eventually develop the function inactive type inadaptability to the receptor of the present invention by inhibiting the functions of endogenous normal DNA. Therefore, the animal can be utilized as a pathologic model animal for such a disease. For example, using the abnormal DNA transgenic animal of the present invention, it is possible to elucidate the mechanism of the function inactive type inadaptability to the receptor of the present invention and to investigate how to treat the disease.

More specifically, the transgenic animal of the present invention expressing the abnormal DNA of the present invention at a high level is expected to serve as an experimental model to elucidate the mechanism of the functional inhibition (dominant negative effect) of a normal polypeptide or receptor by the abnormal polypeptide of the present invention or receptor of the present invention in the function inactive type inadaptability of the receptor of the present invention.

A mammal bearing the abnormal exogenous DNA of the present invention is also expected to serve for screening a candidate drug for the treatment of the function inactive type inadaptability to the receptor of the present invention, since the receptor of the present invention is increased in such an animal in its free form.

Other potential applications of two kinds of the DNA transgenic animals of the present invention described above further include:

(i) use as a cell source for tissue culture;
(ii) elucidation of the relation to a polypeptide or a receptor that is specifically expressed or activated by the receptor of the present invention, by direct analysis of DNA or RNA in tissues of the DNA transgenic animal of the present invention or by analysis of the polypeptide or receptor tissues expressed by the DNA;
(iii) research on the function of cells derived from tissues that are usually cultured only with difficulty, using cells in tissues bearing the DNA cultured by a standard tissue culture technique;
(iv) screening of a drug that enhances the functions of cells using the cells described in (iii) above; and,
(v) isolation and purification of the variant polypeptide or the receptor of the present invention and preparation of an antibody thereto; etc.

In addition, clinical conditions of a disease associated wit the receptor of the present invention, including the function inactive type inadaptability to the receptor of the present invention can be determined by using the DNA transgenic animal of the present invention. Also, pathological findings on each organ in a disease model associated with the receptor of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve to identify cells capable of producing the receptor of the present invention, and to study in association with apoptosis, differentiation or proliferation or on the mechanism of signal transduction in these properties to inspect any abnormality therein. Accordingly, the DNA transgenic animal can provide an effective research material to elucidate the receptor of the present invention and its function and effect.

To develop a drug for the treatment of diseases associated with the receptor of the present invention, including the function inactive type inadaptability to the receptor of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the receptor of the present invention, using the DNA transgenic animal of the present invention or a vector capable of expressing the exogenous DNA of the present invention.

In the specification and drawings, the codes of nucleotides, amino acids, etc. are denoted in accordance with the IUPAC-IUB Commission on Biochemical Nomenclature or by the common codes in the art, examples of which are shown below. For amino acids that may have the optical isomer, L form is presented unless otherwise indicated.

DNA : deoxyribonucleic acid
cDNA : complementary deoxyribonucleic acid
A : adenine
T : thymine
G : guanine
C : cytosine
I : inosine
RNA : ribonucleic acid
mRNA : messenger ribonucleic acid
dATP : deoxyadenosine triphosphate
dTTP : deoxythymidine triphosphate
dGTP : deoxyguanosine triphosphate
dCTP : deoxycytidine triphosphate
ATP : adenosine triphosphate
EDTA : ethylenediaminetetraacetic acid
SDS : sodium dodecyl sulfate
BHA : benzhydrylamine
pMBHA: p-methyobenzhydrylamine
Tos : p-toluenesulfonyl
Bzl : benzyl
Bom : benzyloxymethyl
Boc : t-butoxycarbonyl
DCM : dichloromethane
HOBt :1-hydroxybenztriazole
DCC : N,N'-dicyclohexylcarbodiimido
TFA : trifluoroacetic acid
DIEA : diisopropylethylamine
Gly or G: glycine
Ala or A : alanine
Val or V : valine
Leu or L: leucine
Ile or I : isoleucine
Ser or S : serine
Thr or T : threonine
Cys or C: cysteine
Met or M: methionine
Glu or E: glutamic acid

Asp or D: aspartic acid
Lys or K: lysine
Arg or R: arginine
His or H: histidine
Phe or F: phenylalanine
Tyr or Y : tyrosine
Trp or W: tryptophan
Pro or P : proline
Asn or N: asparagine
Gin or Q: glutamine
pGlu : pyroglutamic acid
Tyr(I) :3-iodotyrosine
DMF : N,N-dimethylformamide
Fmoc : N-9-fluorenyl methoxycarbonyl
Trt : trityl
Pbf :2,2,4,6,7-pentamethyldihydrobenzofuran-5-sulfonyl
Clt :2-chlorotrityl
But : t-butyl
Met(O) : methionine sulfoxide
DNP : dinitrophenol

The sequence identification numbers in the sequence listing of the specification indicate the following sequences.

[SEQ ID NO: 1]
This shows the amino acid sequence of human TGR12.
[SEQ ID NO: 2]
This shows the nucleotide sequence encoding the amino acid sequence represented by SEQ ID NO: 1.
[SEQ ID NO: 3]
This shows the nucleotide sequence of the primer for TGR12 used in PCR in EXAMPLES.
[SEQ ID NO: 4]
This shows the nucleotide sequence of the probe for TGR12 used in PCR in EXAMPLES. [FAM (6-carboxy-fluorescein) was labeled at the 5' end as a reporter dye and TAMRA (6-carboxy-tetramethyl-rhodamine) at the 3' end as a quencher.]
[SEQ ID NO: 5]
This shows the nucleotide sequence of standard DNA (synthetic oligo DNA) for NK1 receptor used in PCR in EXAMPLES.
[SEQ ID NO: 6]
This shows the nucleotide sequence of the primer for NK1 receptor used in PCR in EXAMPLES.
[SEQ ID NO: 7]
This shows the nucleotide sequence of the primer for NK1 receptor used in PCR in EXAMPLES.
[SEQ ID NO: 8]
This shows the nucleotide sequence of the probe for NK1 receptor used in PCR in EXAMPLES. [FAM (6-carboxy-fluorescein) was labeled at the 5' end as a reporter dye and TAMRA (6-carboxy-tetramethyl-rhodamine) at the 3' end as a quencher.]
[SEQ ID NO: 9]
This shows the nucleotide sequence of standard DNA (synthetic oligo DNA) for NK2 receptor used in PCR in EXAMPLES.
[SEQ ID NO: 10]
This shows the nucleotide sequence of the primer for NK2 receptor used in PCR in EXAMPLES.
[SEQ ID NO: 11]
This shows the nucleotide sequence of the primer for NK2 receptor used in PCR in EXAMPLES.
[SEQ ID NO: 12]
This shows the nucleotide sequence of the probe for NK2 receptor used in PCR in EXAMPLES. [FAM (6-carboxy-fluorescein) was labeled at the 5' end as a reporter dye and TAMRA (6-carboxy-tetramethyl-rhodamine) at the 3' end as a quencher.]
[SEQ ID NO: 13]
This shows the nucleotide sequence of standard DNA (synthetic oligo DNA) for NK3 receptor used in PCR in EXAMPLES.
[SEQ ID NO: 14]
This shows the nucleotide sequence of the primer for NK3 receptor used in PCR in EXAMPLES.
[SEQ ID NO: 15]
This shows the nucleotide sequence of the primer for NK3 receptor used in PCR in EXAMPLES.
[SEQ ID NO: 16]
This shows the nucleotide sequence of the probe for NK3 receptor used in PCR in EXAMPLES. [FAM (6-carboxy-fluorescein) was labeled at the 5' end as a reporter dye and TAMRA (6-carboxy-tetramethyl-rhodamine) at the 3' end as a quencher.]
[SEQ ID NO: 17]
This shows the nucleotide sequence of standard DNA (double stranded cDNA) for TGR12 used in PCR in EXAMPLES.
[SEQ ID NO: 18]
This shows the nucleotide sequence of the primer for TGR12 used in PCR in EXAMPLES.
[SEQ ID NO: 19]
This shows the nucleotide sequence of standard DNA (synthetic oligo DNA) for VIP1 receptor used in PCR in EXAMPLES.
[SEQ ID NO: 20]
This shows the nucleotide sequence of the primer for VIP1 receptor used in PCR in EXAMPLES.
[SEQ ID NO: 21]
This shows the nucleotide sequence of the primer for VIP1 receptor used in PCR in EXAMPLES.
[SEQ ID NO: 22]
This shows the nucleotide sequence of the probe for VIP1 receptor used in PCR in EXAMPLES. [FAM (6-carboxy-fluorescein) was labeled at the 5' end as a reporter dye and TAMRA (6-carboxy-tetramethyl-rhodamine) at the 3' end as a quencher.]
[SEQ ID NO: 23]
This shows the nucleotide sequence of standard DNA (synthetic oligo DNA) for VIP2 receptor used in PCR in EXAMPLES.
[SEQ ID NO: 24]
This shows the nucleotide sequence of the primer for VIP2 receptor used in PCR in EXAMPLES.
[SEQ ID NO: 25]
This shows the nucleotide sequence of the primer for VIP2 receptor used in PCR in EXAMPLES.
[SEQ ID NO: 26]
This shows the nucleotide sequence of the probe for VIP2 receptor used in PCR in EXAMPLES. [FAM (6-carboxy-fluorescein) was labeled at the 5' end as a reporter dye and TAMRA (6-carboxy-tetramethyl-rhodamine) at the 3' end as a quencher.]
[SEQ ID NO: 27]
This shows the nucleotide sequence of standard DNA (synthetic oligo DNA) for PACAP receptor used in PCR in EXAMPLES.
[SEQ ID NO: 28]
This shows the nucleotide sequence of the primer for PACAP receptor used in PCR in EXAMPLES.
[SEQ ID NO: 29]
This shows the nucleotide sequence of the primer for PACAP receptor used in PCR in EXAMPLES.
[SEQ ID NO: 30]
This shows the nucleotide sequence of the probe for PACAP receptor used in PCR in EXAMPLES. [FAM (6-carboxy-fluorescein) was labeled at the 5' end as a reporter dye and TAMRA (6-carboxy-tetramethyl-rhodamine) at the 3' end as a quencher.]
[SEQ ID NO: 31]
This shows the nucleotide sequence encoding the amino acid sequence of fused protein of human TGR12 and GFP.
[SEQ ID NO: 32]
This shows the nucleotide sequence of human TGR12 inserted into pAKKO-111H vector.
[SEQ ID NO: 33]
This shows the nucleotide sequence of the primer for human TNF-$\alpha$ used in PCR in EXAMPLES.
[SEQ ID NO: 34]
This shows the nucleotide sequence of the primer for human TNF-$\alpha$ used in PCR in EXAMPLES.
[SEQ ID NO: 35]
This shows the nucleotide sequence of the probe for TNF-$\alpha$ used in PCR in EXAMPLES. [FAM (6-carboxy-fluorescein) was labeled at the 5' end as a reporter dye and TAMRA (6-carboxy-tetramethyl-rhodamine) at the 3' end as a quencher.]
[SEQ ID NO: 36]
This shows the nucleotide sequence of standard DNA (synthetic oligo DNA) for TNF-α used in PCR in EXAMPLES.
[SEQ ID NO: 37]
This shows the nucleotide sequence of the primer for human CCL-5 used in PCR in EXAMPLES.
[SEQ ID NO: 38]
This shows the nucleotide sequence of the primer for human CCL-5 used in PCR in EXAMPLES.
[SEQ ID NO: 39]
This shows the nucleotide sequence of the probe for CCL-5 used in PCR in EXAMPLES. [FAM (6-carboxy-fluorescein) was labeled at the 5' end as a reporter dye and TAMRA (6-carboxy-tetramethyl-rhodamine) at the 3' end as a quencher.]
[SEQ ID NO: 40]
This shows the nucleotide sequence of standard DNA (synthetic oligo DNA) for CCL-5 used in PCR in EXAMPLES.
[SEQ ID NO: 41]
This shows the nucleotide sequence of the primer for human CCL-2 used in PCR in EXAMPLES.
[SEQ ID NO: 42]
This shows the nucleotide sequence of the primer for human CCL-2 used in PCR in EXAMPLES.
[SEQ ID NO: 43]
This shows the nucleotide sequence of the probe for CCL-2 used in PCR in EXAMPLES. [FAM (6-carboxy-fluorescein) was labeled at the 5' end as a reporter dye and TAMRA (6-carboxy-tetramethyl-rhodamine) at the 3' end as a quencher.]
[SEQ ID NO: 44]
This shows the nucleotide sequence of standard DNA (synthetic oligo DNA) for CCL-2 used in PCR in EXAMPLES.
[SEQ ID NO: 45]
This shows the nucleotide sequence encoding the amino acid sequence of fused protein of human TGR12 and GFP.

Hereinafter, the present invention is described in more detail with reference to EXAMPLES, but is not deemed to limit the scope of the present invention thereto.

EXAMPLE 1

TGR12-dependent intracellular calcium increasing activity by physiologically active peptides

CHO/dhfr⁻ cells (hereinafter CHO) were incubated in α-MEM (Invitrogen) supplemented with 10% fetal calf serum. After a termination codon in the TGR12-encoding nucleotide sequence was removed, a nucleotide sequence ligated with the nucleotide sequence encoding GFP (Wako Pure Chemical) [hereinafter briefly referred to as TGR12-GFP (DNA having the nucleotide sequence represented by SEQ ID NO: 31)] was incorporated into vector plasmid pAKKO-111H (the same vector plasmid as pAKK01.11H described in Biochim. Biophys. Acta, 1219, 251-259 (1994)) for animal cell expression, and the vector plasmid was stably expressed to establish TGR12-GFP-expressed CHO cell line (hereinafter CHO-TGR12). CHO-TGR12 was incubated in nucleic acid-free α-MEM medium (Invitrogen) containing 10% dialyzed fetal calf serum.
CHO-TGR12 was plated in a 96-well plate at a density of 30,000 cells/well. After incubation overnight, the medium was discarded and 100 μL/well of assay buffer [Hank's Balanced Salt Solution (Invitrogen), 20 mM HEPES (Dojin Chemical Laboratory) and 2.5 mM Probenecid (Sigma)] containing 4 μM Fluo3-AM (Dojin Chemical Laboratory) was added, which was then allowed to stand for an hour at 37°C. Physiologically active peptides (Peptide Institute, Inc.) shown below or Calcium Ionophore A23187 (Wako Pure Chemical) were diluted in 0.05% CHAPS-containing assay buffer. After the cells were washed with assay buffer, fluorescence reflecting changes in intracellular calcium levels was assayed on FLIPR (Molecular Device). Ten seconds after initiation of the assay, 50 μL of a sample was added at a rate of 50 μL/second. Fluorescence measurement was taken every 1 second during the first 60 seconds and then every 6 seconds during the next 120 seconds; the highest fluorescence intensity over a 3 minute period was taken as the maximum activity under the stimulation condition, representing the mean value of 3 wells. The $EC_{50}$ values were determined using Graphpad PRISM 4 (Graphpad Software).
As a result, the maximum activity in the calcium increasing effect by the addition of assay buffer was 363, and the maximum activity in the calcium increasing effect by the stimulation of 1 μM A23187 was 27379.
The maximum activities in the calcium increasing effect by the stimulation of substance P were 194 at 0.3 nM, 50 at 3 nM, 206 at 30 nM, 15610 at 300 nM and 23842 at 3000 nM.
The maximum activities in the calcium increasing effect by the stimulation of cortistatin-17 were 67 at 0.3 nM, 54 at 3 nM, 94 at 30 nM, 17914 at 300 nM and 23647 at 3000 nM.

The maximum activities in the calcium increasing effect by the stimulation of PAMP-12 were 255 at 0.3 nM, 1617 at 3 nM, 22291 at 30 nM, 24088 at 300 nM and 23743 at 3000 nM.

The maximum activities in the calcium increasing effect by the stimulation of PACAP-27 were 48 at 0.3 nM, 58 at 3 nM, 939 at 30 nM, 23030 at 300 nM and 23728 at 3000 nM.

The maximum activities in the calcium increasing effect by the stimulation of PACAP-38 were 287 at 0.3 nM, 410 at 3 nM, 195 at 30 nM, 23043 at 300 nM and 23315 at 3000 nM.

The maximum activities in the calcium increasing effect by the stimulation of VIP were 56 at 0.3 nM, 60 at 3 nM, 16 at 30 nM, 11153 at 300 nM and 24007 at 3000 nM.

The $EC_{50}$ values in the calcium increasing effect on CHO-TGR12 were about 237 nM in substance P, about 218 nM in cortistatin-17, about 98 nM in PAMP-12, about 93 nM in PACAP-27, about 161 nM in PACAP-38, and about 305 nM in VIP.

Based on these results, substance P, cortistatin-17 and PAMP-12 were found to be ligands for TGR12. In addition, PACAP-27, PACAP-38 and VIP were found to be ligands for TGR12.

EXAMPLE 2

(1) Quantification of gene expression levels of TGR12, NK receptor, VIP receptor and PACAP receptor in human mast cell line LAD 2

Human mast cell line LAD 2, purchased from the U.S. National Institutes of Health, was cultured in StemPro-34 medium (Invitrogen) supplemented with 100 ng/mL stem cell factor (Immuno-Biological Laboratories). A total RNA fraction was prepared from LAD 2 using RNeasy and DNase I kit (Qiagen). Using 1 μg of total RNA as a template, reverse transcription was performed using SuperScript II reverse transcriptase (Invitrogen) according to the manual attached to prepare cDNA. PCR was carried out on 25 μL of a reaction mixture containing the resulting reverse transcription product corresponding to 25 ng of total RNA or standard DNA later described, 1 x Universal PCR Master Mix (Applied Biosystems), 200 nM each of the primers later described and 200 nM of TaqMan probe, using ABI PRISM 7700 Sequence Detector (Applied Biosystems). PCR was performed, after treating at 50°C for 2 minutes and 95°C for 10 minutes, by repeating 40 times the cycle set to include 95°C for 15 seconds and 60°C for 60 seconds. The expression level was calculated using ABI PRISM 7700 SDS software. Cycle numbers at the moment when the fluorescence intensity of a reporter reached preset values were taken on the ordinate and the logarithm of the initial concentration of the standard cDNA on the abscissa to produce a standard curve. The initial concentration of each reverse transcription product was calculated from the standard curve to determine the gene expression levels of human TGR12, NK1 receptor, NK2 receptor, NK3 receptor, VIP1 receptor, VIP2 receptor and PACAP receptor at the respective sites.

As a result, the expression level of TGR12 (standard DNA for TGR12 having the nucleotide sequence represented by SEQ ID NO: 17, primer having the nucleotide sequence represented by SEQ ID NO: 18, primer having the nucleotide sequence represented by SEQ ID NO: 3 and TaqMan probe having the nucleotide sequence represented by SEQ ID NO: 4 were used) in LAD 2 was 687474 copies per 25 ng of total RNA.

In LAD 2, the expression level of NK1 receptor (standard DNA for NK1 receptor having the nucleotide sequence represented by SEQ ID NO: 5, primer having the nucleotide sequence represented by SEQ ID NO: 6, primer having the nucleotide sequence represented by SEQ ID NO: 7 and TaqMan probe having the nucleotide sequence represented by SEQ ID NO: 8 were used) was 50 copies per 25 ng of total RNA.

In LAD 2, the expression level of NK2 receptor (standard DNA for NK2 receptor having the nucleotide sequence represented by SEQ ID NO: 9, primer having the nucleotide sequence represented by SEQ ID NO: 10, primer having the nucleotide sequence represented by SEQ ID NO: 11 and TaqMan probe having the nucleotide sequence represented by SEQ ID NO: 12 were used) was 1 copy per 25 ng of total RNA.

In LAD 2, the expression level of NK3 receptor (standard DNA for NK3 receptor having the nucleotide sequence represented by SEQ ID NO: 13, primer having the nucleotide sequence represented by SEQ ID NO: 14, primer having the nucleotide sequence represented by SEQ ID NO: 15 and TaqMan probe having the nucleotide sequence represented by SEQ ID NO: 16 were used) was 1 copy per 25 ng of total RNA.

In LAD 2, the expression level of VIP1 receptor (standard DNA for the receptor having the nucleotide sequence represented by SEQ ID NO: 19, primer having the nucleotide sequence represented by SEQ ID NO: 20, primer having the nucleotide sequence represented by SEQ ID NO: 21 and TaqMan probe having the nucleotide sequence represented by SEQ ID NO: 22 were used) was 219 copies per 25 ng of total RNA; and the expression level of VIP2 receptor (standard DNA for the receptor having the nucleotide sequence represented by SEQ ID NO: 23, primer having the nucleotide sequence represented by SEQ ID NO: 24, primer having the nucleotide sequence represented by SEQ ID NO: 25 and TaqMan probe having the nucleotide sequence represented by SEQ ID NO: 26 were used) was 179 copies per 25 ng of total RNA.

In LAD 2, the expression level of PACAP receptor (standard DNA for the receptor having the nucleotide sequence represented by SEQ ID NO: 27, primer having the nucleotide sequence represented by SEQ ID NO: 28, primer having the nucleotide sequence represented by SEQ ID NO: 29 and TaqMan probe having the nucleotide sequence represented by SEQ ID NO: 30 were used) was 18 copies per 25 ng of total RNA.

These results revealed that TGR12 was overexpressed in LAD 2. The results further revealed that the NK1, NK2, NK3, VIP1, VIP2 and PACAP receptors were underexpressed in LAD 2.

EXAMPLE 3

TGR12 ligand-dependent degranulation promotion of human mast cells

Human mast cell line LAD 2 was incubated in StemPro-34 medium (Invitrogen) supplemented with 100 ng/mL of stem cell factor (Immuno-Biological Laboratories Co., Ltd.). LAD 2 was washed in Tyrode's buffer (126 mM NaCl, 4.0 mM KCl, 0.69 mM $KH_2PO_4$, 5.6 mM glucose, 1 mM $CaCl_2$, 1 mM $MgCl_2$, and 0.1 % BSA) and 20000 cells of LAD 2 were dispensed into a 96-well plate at 150 μL for each well, followed by incubation at 37°C for 5 minutes. Subsequently, Tyrode's buffer containing 50 μL of TGR12 ligand (Peptide Institute, Inc.) in various concentrations was added and incubation was continued at 37°C for further 20 minutes to induce degranulation. The degree of degranulation was determined by assaying the β-hexosaminidase activity in the degranulation assay supernatant as given below. The supernatant was dispensed into a 96-well plate and 0.1M citrate (pH 4.5)/1 mM 4-methyl umbelliferyl-2-acetamido-2-deoxy-beta-D-glucopyranoside (Wako Pure Chemical) in 5-fold of the supernatant was added thereto, followed by incubation at 37°C for an hour. Next, 0.2 M glycine/$NaCO_3$ (pH 10.7) was added in a volume of 0.1-fold of the reaction solution to stop the reaction. The activity was then assayed using Fusion-α (Perkin-Elmer). The β-hexosaminidase activity was also shown by the activity in the supernatant obtained by centrifugation after freezing and thawing LAD 2 four times, which was made total β-hexosaminidase activity in LAD 2, and the degree of degranulation was shown as the percentage of the β-hexosaminidase activity in the LAD 2 supernatant based on the total β-hexosaminidase activity.

As a result, spontaneous degranulation was approximately 1.5%.

Substance P-stimulated degranulation was 1.8% at 10 nM, 8.7% at 30 nM, 39.2% at100 nM, 58.1% at 300 nM, 66.8% at1 μM, and 71.0% at 3 μM.

Corstatin-17-stimulated degranulation was 1.6% at 10 nM, 2.8% at 30 nM, 26.5% at100 nM, 52.8% at 300 nM, 62.2% at1 μM, and 58.9% at 3 μM.

PAMP-12-stimulated degranulation was 6.3% at 3 nM, 30.7% at 10 nM, 52.1% at 30 nM, 65.2% at100 nM, 67.7% at 300 nM, and 69.3% at 1 μM.

PACAP-27-stimulated degranulation was 2.6% at 10 nM, 19.6% at 30 nM, 59.4% at 100 nM, 71.1% at 300 nM, 75.1% at 1 μM, and 69.1% at 3 μM.

PACAP-38-stimulated degranulation was 7.4% at 10 nM, 24.8% at 30 nM, 49.4% at 100 nM, 65.8% at 300 nM, 68.9% at1 μM, and 63.3% at 3 μM.

VIP-stimulated degranulation was 1.5% at 10 nM, 4.0% at 30 nM, 36.7% at 100 nM, 63.9% at 300 nM, 71.7% at 1 μM, and 76.1% at 3 μM.

The $EC_{50}$ values in the degranulation on LAD 2 were about 91 nM in substance P, about 122 nM in cortistatin-17, about 13 nM in PAMP-12, about 51 nM in PACAP-27, about 51 nM in PACAP-38, and about 105 nM in VIP.

The results revealed that the degranulation of human mast cell line LAD 2 was induced dependently on substance P, cortistatin-17, PAMP-12, PACAP-27, PACAP-38 and VIP.

EXAMPLE 4

Effect of NK1 receptor antagonist against substance P-dependent degranulation promotion of human mast cells

Human mast cell line LAD 2 was incubated in StemPro-34 medium (Invitrogen) supplemented with 100 ng/mL of stem cell factor (Immuno-Biological Laboratories Co., Ltd.). LAD 2 was washed in Tyrode's buffer (126 mMNaCl, 4.0 mM KCl, 0.69 mM $KH_2PO_4$, 5.6 mM glucose, 1 mM $CaCl_2$, 1 mM $MgCl_2$, and 0.1 % BSA) and 20000 cells of LAD 2 were dispensed into a 96-well plate at 100 μL for each well, followed by incubation at 37°C for 5 minutes. Subsequently, Tyrode's buffer containing 50 μL of NK1 antagonist GR-82334 (Sigma, Inc.) in various concentrations or L-703606 (Sigma, Inc.) was added and incubation was continued at 37°C for 5 minutes. Next, Tyrode's buffer containing 50 μL of substance P solution (final concentration of 0.3 μM; Peptide Institute, Inc.) or calcium ionophore A23187 (final concentration of 0.3 μM; Wako Pure Chemical) was added and incubation was continued at 37°C for further 20 minutes to induce degranulation. The degree of degranulation was determined by assaying the β-hexosaminidase activity in the degranulation assay supernatant as given below. The supernatant was dispensed into a 96-well plate and 0.1M citrate (pH 4.5)/1 mM 4-methyl umbelliferyl-2-acetamido-2-deoxy-beta-D-glucopyranoside (Wako Pure Chemical) in 5-fold of the supernatant was added, followed by incubation at 37°C for an hour. Next, 0.2 M glycine/$NaCO_3$ (pH 10.7) was added in a volume of 0.1-fold of the reaction solution to stop the reaction. The activity was then assayed using Fusion-α (Perkin-Elmer). LAD 2 was frozen and thawed four times and then centrifuged. The activity in the supernatant was made the total β-hexosaminidase activity in LAD 2. The inhibition activity by the NK1 receptor antagonist was expressed as the percentage of the β-hexosaminidase activity when the NK1 antagonist was added in various concentrations, based on the β-hexosaminidase activity when no NK1 antagonist was added.

As a result, in the substance P stimulation, degranulation induced by the addition of NK1 receptor peptidic antagonist GR-82334 (Sigma, Inc.) was 101.2% at 100 nM of GR-82334, 105.7% at 300 nM, 102.7% at 1 μM, 99.6% at 3 μM, 95.8% at 10 μM, and 99.5% at 30 μM.

In the A23187 stimulation, degranulation induced by the addition of GR-82334 was 101.1% at 100 nM of GR-82334, 102.0% at 300 nM, 102.0% at 1 μM, 102.8% at 3 μM, 103.2% at 10 μM, and 102.4% at 30 μM.

In the substance P stimulation, degranulation induced by the addition of NK1 receptor low molecular antagonist L-703606 (Sigma, Inc.) was 101.1% at 100 nM of L-703606, 97.9% at 300 nM, 98.8% at 1 μM, 100.0% at 3 μM, 39.4% at 10 μM, and 3.2% at 30 μM.

In the A23187 stimulation, degranulation induced by the addition of L-703606 was 98.4% at 100 nM of L-703606, 97.2% at 300 nM, 97.4% at 1 μM, 91.5% at 3 μM, 37.1% at 10 μM, and 2.4% at 30 μM.

These results revealed that NK1 receptor peptidic antagonist GR-82334 did not inhibit the degranulation by the substance P and A23187 stimulation in the concentration range of 0.1 M to 30 μM. The results further revealed that NK1 receptor low molecular antagonist L-703606 did not inhibit the degranulation by the substance P and A23187 stimulation in the concentration range of 0.1 M to 3 μM but inhibited in the concentration rage of 10 μM to 30 μM non-specifically to TGR12 and NK 1 receptor.

Since the antagonist of NK1 receptor known as substance P receptor did not inhibit the TGR12-dependent degranulation reaction by substance P, antagonists of TGR12, not NK1 receptor antagonists, could be inhibitors of the degranulation reaction by substance P.

## EXAMPLE 5

Effect of substance P on MAPK activation in human TGR12-expressed CHO cells

CHO/dhfr- cells (hereinafter CHO) were incubated in α-MEM (Invitrogen) containing 10% fetal calf serum. Animal cell expression vector plasmid pAKKO-111H (the vector plasmid identical to the pAKK01.11H described in Biochim. Biophys. Acta, Hinuma, S., et al., 1219, 251-259, 1994), in which a nucleotide sequence from the initiation codon to the termination codon of human TGR12 was incorporated (DNA having the nucleotide sequence represented by SEQ ID NO: 32), was stably expressed in CHO to establish human TGR12-expressed CHO cell line (hereinafter CHO-TGR12). CHO-TGR12 was incubated in nucleic acid-free α-MEM (Invitrogen) containing 10% dialyzed fetal calf serum.

The cells were prepared as follows. CHO-TGR12 was detached with 0.05% Trypsin-EDTA (GIBCO) and suspended in medium (MEM-α (GIBCO), 10% FBS (GIBCO), penicillin/streptomycin (BIOWHITTAKER)). After centrifugation, the deposited cells were suspended in the medium at 2 x $10^5$ cells/ml and 1 mL each was plated in a 12-well cell culture dish. After incubation overnight in a $CO_2$ incubator, the medium was aspirated and washed with PBS. Then, 1 mL of serum-free medium (MEM-α (GIBCO), penicillin/streptomycin (BIOWHITTAKER)) was added followed by incubation in a $CO_2$ incubator. Pertussis toxin (hereinafter PTX) was added at a final concentration of 0.1 μg/mL 4 hours before the assay.

Activation of the cells was performed as follows. The medium was aspirated and washed with PBS. Then 750 μL of assay buffer (HBSS (GIBCO) and 10 mM HEPES (Dojin Chemical Laboratory) was added and then preincubated for 15 minutes at 37°C. After adding 250 μL of the substance P solution and the ATP solution as a positive control, incubation was performed at 37°C and the reaction was then discontinued by quenching. The assay buffer was aspirated and washed with PBS. Thereafter, 1 x LDS sample buffer/DTT was added to the cells for recovery, which was ultrasonicated for 30 seconds, then heated at 70°C for 10 minutes and quenched.

Detection by SDS-PAGE and western blotting was performed as follows. After resolving on a 10% Bis-Tris gel (Invitrogen), the proteins were transferred onto Immune-Blot PVDF membrane (BIO-RAD). The membrane was blocked in Block Ace (Dainippon Pharmaceutical), incubated at room temperature for an hour using a primary antibody (phospho-p42/p44 MAP kinase (Thr202/Thr204) antibody (CST) or p42/p44 MAP kinase antibody (CST)) and then incubated at room temperature for an hour using a secondary antibody (anti-rabbit IgG (CST)). The membrane after the antibody reaction was visualized by chemiluminescence with ECL PLUS (Amersham Biosciences), followed by detection and image analysis using LAS 1000 (FUJI FILM).

As a result, phosphorylation of ERK1/2 (p42/p44 MAP kinase) was observed when CHO-TGR12 was stimulated by substance P. Phosphorylation of ERK1/2 (p42/p44 MAP kinase) by CHO-TGR12 reached the maximum reaction about 10 minutes after the stimulation and was observed even 30 minutes after the stimulation. Phosphorylation of ERK1/2 (p42/p44 MAP kinase) by substance P with CHO-TGR12 was attenuated by pretreatment of PTX, whereas attenuation by pretreatment of PTX was not observed in phosphorylation of ERK1/2 (p42/p44 MAP kinase) with ATP.

EXAMPLE 6

Effect of increasing the expression of TNF-α, CCL-2 or CCL-5 in human mast cells by TGR12 ligand

Human mast cell line LAD 2 was cultured in StemPro-34 medium (Invitrogen) supplemented with 100 ng/mL of stem cell factor (Immuno-Biological Laboratories Co., Ltd.). In the experiments where changes in expression level by substance P stimulation were analyzed with passage of time, LAD 2 was centrifuged at 300 x g for 5 minutes and the cells precipitated were counted and suspended in the medium at 6.67 x $10^5$ cells/mL. A 750 μL aliquot was inoculated on a 24-well plate, which was then allowed to stand in a $CO_2$ incubator at 37°C for 30 minutes. Subsequently, 250 μL of the medium containing substance P (Peptide Institute, Inc.) at final concentration of 1 μM or the medium alone was added, followed by further incubation in a $CO_2$ incubator at 37°C for 0.5, 2, 8 and 24 hours. The assay was performed for the respective reaction conditions in n=3. The plate was ice-chilled 0.5, 2, 8 and 24 hours after to stop the activation of LAD 2. After centrifugation at 300 x g for 5 minutes, total RNA was extracted from the precipitated cells using RNeasy Mini Kit (Qiagen) according to the protocol attached. Further in the experiments where expression levels upon stimulation by various TGR12 ligands were analyzed, LAD 2 was centrifuged at 300 x g for 5 minutes and the cells precipitated were counted and suspended in the medium at 1.33 x $10^6$ cells/mL. A 750 μL aliquot was inoculated on a 24-well plate, which was allowed to stand in a $CO_2$ incubator at 37°C for 30 minutes. Subsequently, 250 μL of the medium containing cortistatin-17, PAMP-12, PACAP-27 or VIP (Peptide Institute, Inc.) at final concentration of 1 μM or the medium alone was added, followed by further incubation in a $CO_2$ incubator at 37°C for 2 hours. The assay was performed for the respective reaction conditions in n=3. Two hours after, the plate was ice-chilled to stop the activation of LAD 2. After centrifugation at 300 x g for 5 minutes, total RNA was extracted from the precipitated cells using RNeasy Mini Kit (Qiagen) according to the protocol attached. The total RNA obtained as described above was concentrated using ethachinmate according to the protocol attached and dissolved in RNase-free $H_2O$. Next, using the solution corresponding to 1 μg of total RNA, reverse transcription was performed by the following procedure using SuperScript II reverse transcriptase (Invitrogen) to synthesize single stranded cDNA. To the total RNA solution, 0.1 μg of random primer (Invitrogen) and 1 μL of 10 mM dNTP (Invitrogen) were added and then RNase-free $H_2O$ was added to make the whole volume 12 μL. The mixture was then incubated at 70°C for 10 minutes and ice-cooled for a minute. After 4 μL of 5 x First-Strand Buffer (attached to SuperScript II reverse transcriptase), 2 μL of 0.1M DTT (attached to SuperScript II reverse transcriptase), 1 μL of RNaseOUT (Invitrogen) and 1 μL of SuperScript II reverse transcriptase were added, the mixture was incubated at 42°C for 50 minutes, then at 70°C for 15 minutes and ice-cooled for 5 minutes. The thus obtained cDNA was purified using ethachinmate in accordance with the protocol attached and dissolved in Tris-EDTA buffer (Fluka).

In TaqMan PCR below, hTNFalpha-tF (SEQ ID NO: 33) and hTNFalpha-tR (SEQ ID NO: 34) were used as primers for human TNF-α quantification, hTNFalpha-tP (SEQ ID NO: 35) as a probe and hTNFalpha- standard (SEQ ID NO: 36) as a standard. For human CCL-5 quantification, hCCL5-tF (SEQ ID NO: 37) and hCCL5-tR(SEQ ID NO: 38) were used as primers, hCCL5-tP (SEQ ID NO: 39) as a probe and hCCL5 standard (SEQ ID NO: 40) as a standard. For human CCL-2 quantification, hCCL2-tF (SEQ ID NO: 41) and hCCL2-tR (SEQ ID NO: 42) were used as primers, hCCL2-tP (SEQ ID NO: 43) as a probe and hCCL2 standard (SEQ ID NO: 44) as a standard.

TaqMan PCR was performed in n=2 for each sample in a liquid volume of 15 μl using 12.5 ng of total RNA as a template. The reaction solution was composed of 900 nM primer, 250 nM probe and 1/2 volume of TaqMan Universal PCR Master Mix (Applied Biosystems). The reaction and analysis were performed by ABI PRISM 7900HT Sequence Detection System (Applied Biosystems). After keeping warm at 50°C for 2 minutes and then at 95°C for 10 minutes, the reaction cycle was set in one cycle to include 95°C for 15 seconds and 60°C for 1 minute and repeating the cycle 40 times. The gene expression level was analyzed and determined in such a manner that the correlation coefficient was over 0.995 in the calibration curve.

The results of expression analysis are shown below. The expression level was shown by an average of n=3 as the copy number of mRNA calculated based on standard DNA.

The changes in expression level were analyzed with passage of time when stimulated by substance P and the experimental results are shown below. Without stimulation, the expression level of TNF-α in LAD 2 was 10118 copies. When stimulated by substance P, the expression level of TNF-α in LAD 2 was 56065 copies 0.5 hours after the stimulation, 1346293 copies 2 hours after the stimulation, 54313 copies 8 hours after the stimulation, and 4332 copies 24 hours after the stimulation. On the other hand, when the medium alone was added, the expression level of TNF-α in LAD 2 was 9668 copies 0.5 hours after the stimulation, 6689 copies 2 hours after the stimulation, 6035 copies 8 hours after the stimulation, and 4886 copies 24 hours after the stimulation. Without stimulation, the expression level of CCL-5 in LAD 2 was 15721 copies. When stimulated by substance P, the expression level of CCL-5 in LAD 2 was 38107 copies 0.5 hours after the stimulation, 88872 copies 2 hours after the stimulation, 69921 copies 8 hours after the stimulation and 53605 copies 24 hours after the stimulation. On the other hand, when the medium alone was added, the expression level of CCL-5 in LAD 2 was 14585 copies 0.5 hours after the stimulation, 15625 copies 2 hours after the stimulation, 12538 copies 8 hours after the stimulation and 15003 copies 24 hours after the stimulation.

The expression levels were analyzed on stimulation by various TGR12 ligands and the experimental results are shown below. The expression level of TNF-α in LAD 2 was 1731 copies in the absence of stimulation. Two hours after stimulation, the expression level of TNF-α in LAD 2 was 1331 copies when the medium alone was added, 173462 copies when stimulated by substance P, 163551 copies when stimulated by cortistatin-17, 8918 copies when stimulated by PAMP-12, 219597 copies when stimulated by PACAP-27, and 176630 copies when stimulated by VIP. The expression level of CCL-5 in LAD 2 was 30341 copies in the absence of stimulation. Two hours after stimulation, the expression level of CCL-5 in LAD 2 was 23501 copies when the medium alone was added, 47117 copies when stimulated by substance P, 47322 copies when stimulated by cortistatin-17, 34566 copies when stimulated by PAMP-12, 48242 copies when stimulated by PACAP-27, and 45015 copies when stimulated by VIP. The expression level of CCL-2 in LAD 2 was 643224 copies in the absence of stimulation. Two hours after stimulation, the expression level of CCL-2 in LAD 2 was 404112 copies when the medium alone was added, 2694837 copies when stimulated by substance P, 2944622 copies when stimulated by cortistatin-17, 1143248 copies when stimulated by PAMP-12, 3809873 copies when stimulated by PACAP-27, and 3630286 copies when stimulated by VIP.

EXAMPLE 7

Competitive binding assay for TGR12 by TGR12 ligand

[$^{125}$I]-Labeled rat cortistatin-14 (Tyr0) (hereinafter [$^{125}$I]-rCST14 (Y0)) used for the competitive binding assay was prepared as follows. After 3 nmol/6 μL of rCST14 (Y0) (Funakoshi Co.), 6 μL of 10 μg/mL lactoperoxidase (in 0.1M HEPES (pH 7, 4)), 6 μL of 0.001% $H_2O_2$ and 6 μL of 37 MBq NaI (6 μL of 15 mg/100 mL NaI was used in cold run) were mixed, the mixture was reacted at room temperature for 20 minutes. The monoiodo product was fractionated by HPLC and radioactivity was recovered in the fraction of about 24 minutes (confirmed by MS analysis in the cold run that the monoiodo product has the same molecular weight as in the monoiodo rCST14 (Y0)). For fractionation, TSKgel ODS-80TM was used, 10% MeCN/0.1% TFA was used as eluant A and 60% MeCN/0.1% TFA was used as eluant B at a gradient of 0-25 (2 mins.), 25-27.5 (3 mins.) and 27.5-35 (60 mins.), respectively; and the flow rate was 1 mL/min.

CHO/dhfr- cells (hereinafter CHO) were incubated in α-MEM (Invitrogen) supplemented with 10% fetal calf serum. A nucleotide sequence in which the termination codon-removed nucleotide sequence of TGR12 was ligated with the nucleotide sequence of GFP (Wako Pure Chemical) (hereinafter TGR12-GFP (DNA having the nucleotide sequence represented by SEQ ID NO: 45)) was incorporated into CHO and the resulting vector plasmid pAKKO-111H (same vector plasmid as pAKK01.11H described in Biochim. Biophys. Acta, 1219, 251-259, 1994) for animal cell expression was stably expressed to establish the TGR12-GFP expression CHO cell line (hereinafter CHO-TGR12GFP). CHO-TGR12GFP was incubated in nucleic acid-free α-MEM (Invitrogen) supplemented with 10% dialyzed fetal calf serum.

The binding assay using CHO-TGR12GFP was performed as follows. Various physiologically active peptides were purchased from Peptide Institute, Inc. CHO-TGR12GFP was plated in a 24-well plate at a cell density of 2 x $10^5$ cells/well. After incubation overnight, the cells were washed 3 times with ice-chilled assay buffer (MEM-α, 20 mM HEPES (pH 7.4), 0.05% BSA) and 250 μL of the assay buffer was added thereto. A suspension of [$^{125}$I]-rCST14 (Y0)/physiologically active peptide solution in 250 μL of the assay buffer (the final concentration of [$^{125}$I]-rCST14 (Y0) was 200 pM) was added and the mixture was allowed to stand at 4°C for 2 hours. After washing 3 times with 500 μL of the assay buffer, the cells were lysed in 250 μL of 0.5N NaOH and the lysate was recovered for measurement of the radioactivity.

Based on the above, non-specific binding was estimated as the activity of 10 μM rat cortistatin-14 and the $IC_{50}$ value of each peptide was obtained from Hill plot analysis. As a result, the $IC_{50}$ was 2886 nM in substance P, 47 nM in human cortistatin-17, 18 nM in human PAMP12, 264 nM in human PACAP27, 47 nM in human PACAP38 and 2039 nM in human VIP.

EXAMPLE 8

Intracellular calcium-increasing and degranulation-promoting effects by TGR12 ligand on human TGR12 expression RBL-2H3 cells

RBL-2H3 (hereinafter RBL2H3) was incubated in α-MEM (Invitrogen) supplemented with 10% fetal calf serum. Vector plasmid pcDNA3.1 for animal cell expression, in which the nucleotide sequence containing the initiation codon to the termination codon of human TGR12 (DNA having the nucleotide sequence represented by SEQ ID NO: 32) was incorporated, was stably expressed in RBL-2H3 to establish human TGR12-expressed RBL-2H3 cell line (hereinafter RBL2H3-TGR12). RBL2H3-TGR12 was incubated in α-MEM (Invitrogen) supplemented with 10% fetal calf serum and

200 μg/mL of Geneticin.

The intracellular calcium increasing activity was assayed as follows. RBL2H3 and RBL2H3-TGR12 were plated in a 96-well plate at a density of 40,000 cells/well and incubated overnight. The medium was then discarded and 100 μL/well of 4 μM Fluo3-AM (Dojin Kagaku)-containing assay buffer [Hank's Balanced Salt Solution (Invitrogen), 20 mM HEPES (Dojin Kagaku) and 2.5 mM Probenecid (Sigma)] was added. The mixture was allowed to stand for an hour at 37°C. Various TGR12 (Peptide Institute, Inc.) or calcium ionophore A23187 (Wako Pure Chemical) was diluted in 0.05% CHAPS-containing assay buffer. After the cells were washed with the assay buffer, fluorescence emitted with changes in intracellular calcium was measured using FLIPR (Molecular Device). A sample, 50 μL, was added at a rate of 50 μL/sec. 10 seconds after initiation of the assay. Fluorescence was measured every 1 second during the first 60 seconds and every 6 seconds during the next 120 seconds. The highest fluorescence intensity during 3 minutes was made the maximum activity under the stimulation conditions, which was taken as an average of 3 wells. The $EC_{50}$ values were calculated using Graphpad PRISM 4 (Graphpad Software).

The degranulation promoting effect was determined as follows. RBL2H3 and RBL2H3-TGR12 were plated into a 96-well plate at a density of 40,000 cells/well and incubated overnight. Afterward the medium was discarded. After washing with Tyrode's buffer (126 mM NaCl, 4.0 mM KCl, 0.69mM $KH_2PO_4$, 5.6 mM glucose, 1 mM $CaCl_2$, 1 mM $MgCl_2$ and 0.1% BSA), 150 μL each/well of Tyrode's buffer was added to a 96-well plate, followed by incubation at 37°C for 5 minutes. Next, 50 μL of Tyrode's buffer containing substance P (Peptide Institute, Inc.) in various concentrations was added and the mixture was incubated at 37°C for further 20 minutes to induce degranulation. The β-hexosaminidase activity in the supernatant for the degranulation assay was measured as follows to determine the degree of degranulation. The supernatant was dispensed into a 96-well plate and a 5-fold volume of 0.1M citrate (pH 4.5)/1 mM 4-methyl umbelliferyl-2-acetamido-2-deoxy-beta-D-glucopyranoside (Wako Pure Chemical) based on the volume of the supernatant was added thereto, followed by incubation at 37°C for an hour. Subsequently, a 0.1-fold volume of 0.2 M glycine/$NaCO_3$ (pH 10.7) based on the volume of the reaction solution was added to stop the reaction and the fluorescence was measured using Fusion-α (Perkin Elmer). For the β-hexosaminidase activity, the activity in the supernatant, which was obtained by likewise plating RBL2H3 and RBL2H3-TGR12GFP onto the plate, detaching the same with trypsin/EDTA, freeze-thawing 4 times and then centrifugation, was made the total β-hexosaminidase activity in LAD 2. The degree of degranulation was shown by a ratio of the β-hexosaminidase activity in the LAD 2 supernatant to the total β-hexosaminidase activity.

When the intracellular calcium increasing activity was measured in RBL2H3, the maximum activity in the calcium increasing effect was 51 by the addition of assay buffer and when stimulated by 1 μM A23187, the maximum activity in the calcium increasing effect was 25338. When stimulated by substance P, the maximum activity in the calcium increasing effect was 44 in 1 nM, 51 in 10 nM, 50 in 100 nM, 95 in 1 μM and 41 in 10 μM. When stimulated by cortistatin-17, the maximum activity in the calcium increasing effect was 67 in 1 nM, 78 in 10 nM, 54 in 100 nM, 49 in 1 μM and 52 in 10 μM. When stimulated by PAMP12, the maximum activity in the calcium increasing effect was 52 in 1 nM, 52 in 10 nM, 35 in 100 nM, 44 in 1 μM and 43 in 10 μM. When stimulated by PACAP27, the maximum activity in the calcium increasing effect was 47 in 1 nM, 37 in 10 nM, 63 in 100 nM, 59 in 1 μM and 45 in 10 μM.

When the intracellular calcium increasing activity was measured in RBL2H3-TGR12, the maximum activity in the calcium increasing effect was -2 by the addition of assay buffer and when stimulated by 1 μM A23187, the maximum activity in the calcium increasing effect was 25624. When stimulated by substance P, the maximum activity in the calcium increasing effect was 4 in 1 nM, 13 in 10 nM, 1702 in 100 nM, 10005 in 1 μM and 12649 in 10 μM. When stimulated by cortistatin-17, the maximum activity in the calcium increasing effect was -2 in 1 nM, 33 in 10 nM, 1574 in 100 nM, 11193 in 1 μM and 12762 in 10 μM. When stimulated by PAMP12, the maximum activity in the calcium increasing effect was 41 in 1 nM, 2088 in 10 nM, 11110 in 100 nM, 12039 in 1 μM and 12331 in 10 μM. When stimulated by PACAP27, the maximum activity in the calcium increasing effect was 40 in 1 nM, 29 in 10 nM, 5300 in 100 nM, 11494 in 1 μM and 12667 in 10 μM.

As a result of the analysis of degranulation promoting effect in RBL2H3, spontaneous degranulation was 9.4%. When stimulated by substance P the degranulation was 8.8% in 10 nM, 8.9% in 30 nM, 8.9% in 100 nM, 8.5% in 300 nM, 8.4% in 1 μM and 7.9% in 3 μM.

As a result of the analysis of degranulation promoting effect in RBL2H3-TGR12, spontaneous degranulation was 10.5%. When stimulated by substance P the degranulation was 9.7% in 10 nM, 10.8% in 30 nM, 16.7% in 100 nM, 27% in 300 nM, 36.3% in 1 μM and 42.0% in 3 μM.

INDUSTRIAL APPLICABILITY

The compounds that inhibit the activity/function of the ligands (ligands of the present invention) capable of specifically binding to the proteins comprising the same or substantially the same amino acid sequence as the amino acid sequence represented by SEQ ID NO: 1, their partial peptides, or salts thereof (receptors of the present invention), or to the receptors of the present invention (e.g., the receptor antagonists of the present invention); the antibodies against the receptors of the present invention; the antisense polynucleotides against the receptors of the present invention; etc., can inhibit the physiological activities (e.g., the mast cell degranulation promoting action, eicosanoid production promoting action, cytokine production promoting action, mast cell growth promoting action, mast cell activating action, etc.) possessed by the receptors of the present invention or the ligands of the present invention and thus can be used for low-toxic, safe and excellent mast cell degranulation inhibitors, eicosanoid production inhibitors, cytokine production inhibitors, mast cell growth inhibitors, mast cell activation inhibitors (including, e.g., MAPK activation inhibitors, etc.), etc., for example, as agents for preventing/treating, e.g., immune disorders [e.g., inflammatory disorders (pituitary tumor, thyroiditis, peritonitis, Crohn's disease, ulcerative colitis, erythema nodosum, chronic articular rheumatism, systemic lupus erythematosus, etc.), allergy (e.g., allergic conjunctivitis, allergic rhinitis, pollinosis, metal allergy, etc.), asthma, secretory otitis media, Meniere's disease, contact dermatitis, anaphylaxis, urticaria, myasthenia gravis, glomerulonephritis, Sjögren's syndrome, Basedow's disease, insulin resistant diabetes, atopic dermatitis, leukocyte abnormality, etc.], urinary tract disorders (e.g., renal tubulointerstitial injury (fibril formation), interstitial cystitis, allergic cystitis, etc.), digestive disorders [e.g., irritable bowel syndrome, chronic liver disease, food allergy, allergic enteritis, milk protein-induced proctitis, digestive ulcer (e.g., gastric ulcer, duodenal ulcer, marginal ulcer, Zollinger-Ellison syndrome, etc.), gastritis, reflux esophagitis, NUD (non-ulcer dyspepsia), gastric MALT lymphoma, nonsteroidal anti-inflammatory drug-induced ulcer, acid indigestion, postoperative stress-induced hyperacidity and ulcer, etc.], respiratory disorders [e.g., chronic obstructive pulmonary disease (e.g., chronic bronchitis, pulmonary emphysema), diffuse panbronchiolitis, cystic fibrosis, hypersensitivity penumonitis, idiopathic interstitial pneumonia, pulmonary fibrosis, etc.], circulatory disorders (e.g., arteriosclerosis, acute coronary syndrome, atherosclerotic aortic aneurysm, cardiac anaphylaxis, heart failure, myocardial infarction, angina pectoris, arrhythmia, deep vein thrombosis, restenosis after PTCA, etc.), ophthalmic disorders (e.g., pterygium, vernal conjunctivitis, dry eye, etc.), cancer (e.g., papillary thyroid carcinoma, non-small-cell lung cancer, endometrial cancer, cervical cancer, gastric cancer, pancreatic cancer, lung cancer, renal cancer, hepatic cancer, ovarian cancer, prostate cancer, bladder cancer, breast cancer, colon cancer, rectal cancer, Kaposi's sarcoma, mastocytoma, etc.), cerebral infarction, hyperlipemia, acute renal failure, diabetes mellitus, obesity, edema, granuloma, atopic myelitis, neurofibroma, nasal mucosa hypersensitivity, Hodgkin's disease, endometrial hyperplasia, etc., and the like.

Further by the screening methods or screening kits using the receptors of the present invention or mast cells and the ligands of the present invention, the compounds or their salts having, e.g., the mast cell degranulation inhibitory action, eicosanoid production inhibitory action, cytokine production inhibitory action, mast cell growth inhibitory action, mast cell activation inhibitory action, etc.)

## Claims

**1.** A method of screening for a mast cell degranulation inhibitor, which comprises using (a) a protein comprising the same or substantially the same amino acid sequence as the amino acid sequence of SEQ ID NO: 1, its partial peptide, or a salt thereof and (b) a ligand capable of specifically binding to said protein.

**2.** The screening method according to claim 1, wherein the ligand is substance P, cortistatin, a pituitary adenylate cyclase activating polypeptide, a vasoactive intestinal peptide or proadrenomedullin N-terminal 20 peptide.

**3.** The screening method according to claim 1, wherein a cell stimulating activity mediated by a protein comprising the same or substantially the same amino acid sequence as the amino acid sequence of SEQ ID NO: 1, its partial peptide, or a salt thereof, is assayed and used as a parameter.

**4.** A method of screening for a compound or its salt having an action of inhibiting degranulation induced by a pituitary adenylate cyclase activating polypeptide in human mast cells, which comprises using (a) a protein comprising the same or substantially the same amino acid sequence as the amino acid sequence of SEQ ID NO: 1, its partial peptide, or a salt thereof and (b) a ligand capable of specifically binding to said protein.

**5.** The screening method according to claim 1, which comprises:

(i) the step of contacting (a) a protein comprising the same or substantially the same amino acid sequence as the amino acid sequence of SEQ ID NO: 1, its partial peptide, or a salt thereof, with (b) a ligand capable of specifically binding to said protein in the absence of a test compound and measuring the binding amount of said ligand to said protein, its partial peptide, or a salt thereof;
(ii) the step of contacting (a) a protein comprising the same or substantially the same amino acid sequence as the amino acid sequence of SEQ ID NO: 1, its partial peptide, or a salt thereof, with (b) a ligand capable of specifically binding to said protein in the presence of a test compound and measuring the binding amount of said ligand to said protein, its partial peptide, or a salt thereof; and,
(iii) the step of comparing the binding amounts of said ligand to said protein, its partial peptide, or a salt thereof between the step (i) and the step (ii), and selecting said test compound as a mast cell degranulation inhibitor when the binding amount in the step (ii) is lower than the binding amount in the step (i).

**6.** The screening method according to claim 5, wherein the test compound is selected as a mast cell degranulation inhibitor when the binding amount in the step (ii) is not greater than 50% than the binding amount in the step (i).

**7.** The screening method according to claim 1, which comprises:

(i) the step of contacting (a) a protein comprising the same or substantially the same amino acid sequence as the amino acid sequence of SEQ ID NO: 1, its partial peptide, or a salt thereof, with (b) a ligand capable of specifically binding to said protein in the absence of a test compound and measuring the binding amount of said ligand to said protein, its partial peptide, or a salt thereof;
(ii) the step of contacting (a) a protein comprising the same or substantially the same amino acid sequence as the amino acid sequence of SEQ ID NO: 1, its partial peptide, or a salt thereof, with (b) a ligand capable of specifically binding to said protein in the presence of a test compound and measuring the binding amount of said ligand to said protein, its partial peptide, or a salt thereof;
(iii) the step of comparing the binding amounts of said ligand to said protein, its partial peptide, or a salt thereof between the step (i) and the step (ii), and selecting a test compound when the binding amount in the step (ii) is lower than the binding amount in the step (i); and,
(iv) the step of contacting the test compound obtained in (iii) above with mast cells and selecting a test compound inhibiting degranulation.

**8.** The screening method according to claim 1, wherein the mast cell degranulation inhibitor is a preventive/therapeutic agent for immune disorders, urologic disorders, digestive disorders or respiratory disorders.

**9.** A kit for screening for a mast cell degranulation inhibitor, which comprises (a) a protein comprising the same or substantially the same amino acid sequence as the amino acid sequence of SEQ ID NO: 1, its partial peptide, or a salt thereof and (b) a ligand capable of specifically binding to said protein.

**10.** A mast cell degranulation inhibitor, which comprises an antagonist to a protein comprising the same or substantially the same amino acid sequence as the amino acid sequence of SEQ ID NO: 1, its partial peptide, or a salt thereof.

**11.** A mast cell degranulation inhibitor, which comprises an antibody against a protein comprising the same or substantially the same amino acid sequence as the amino acid sequence of SEQ ID NO: 1, its partial peptide, or a salt thereof.

**12.** A diagnostic agent for immune disorders, urologic disorders, digestive disorders or respiratory disorders, which comprises an antibody against a protein comprising the same or substantially the same amino acid sequence as the amino acid sequence of SEQ ID NO: 1, its partial peptide, or a salt thereof.

**13.** A mast cell degranulation inhibitor, which comprises an antibody against an agonist for a protein comprising the same or substantially the same amino acid sequence as the amino acid sequence of SEQ ID NO: 1, its partial peptide, or a salt thereof.

**14.** A diagnostic agent for immune disorders, urologic disorders, digestive disorders or respiratory disorders, which comprises a polynucleotide comprising a polynucleotide encoding a protein comprising the same or substantially the same amino acid sequence as the amino acid sequence of SEQ ID NO: 1, or its partial peptide.

**15.** A mast cell degranulation inhibitor comprising an siRNA or shRNA for (i) a polynucleotide comprising an entire or part of a nucleotide sequence complementary or substantially complementary to a polynucleotide comprising a polynucleotide encoding a protein comprising the same or substantially the same amino acid sequence as the amino acid sequence of SEQ ID NO: 1, or its partial peptide or (ii) a polynucleotide comprising a polynucleotide encoding said protein or its partial peptide.

**16.** The inhibitor according to claims 10, 11, 13 or 15, wherein the mast cell degranulation inhibitor is a preventive/therapeutic agent for immune disorders, urologic disorders, digestive disorders or respiratory disorders.

**17.** A method of inhibiting degranulation of mast cells, which comprises inhibiting the activities of a protein comprising the same or substantially the same amino acid sequence as the amino acid sequence of SEQ ID NO: 1, its partial peptide, or a salt thereof, or the activities of a ligand capable of specifically binding to said protein.

**18.** A method of screening for a mast cell degranulation inhibitor, which comprises using mast cells and a ligand capable of specifically binding to a protein comprising the same or substantially the same amino acid sequence as the amino acid sequence of SEQ ID NO: 1.

**19.** The screening method according to claim 18, which comprises:

(i) the step of contacting mast cells with a ligand capable of specifically binding to a protein comprising the same or substantially the same amino acid sequence as the amino acid sequence of SEQ ID NO: 1 in the absence of a test compound and measuring the binding amount of said ligand to said mast cell;
(ii) the step of contacting mast cells with a ligand capable of specifically binding to a protein comprising the same or substantially the same amino acid sequence as the amino acid sequence of SEQ ID NO: 1 in the presence of a test compound and measuring the binding amount of said ligand to said mast cell;
(iii) the step of selecting a test compound when the binding amount in the step (ii) is lower than the binding amount in the step (i); and,
(iv) the step of contacting the test compound obtained in (iii) above with mast cells and selecting a test compound inhibiting degranulation.

**20.** A kit for screening for a mast cell degranulation inhibitor, which comprises mast cells and a ligand capable of specifically binding to a protein comprising the same or substantially the same amino acid sequence as the amino acid sequence of SEQ ID NO: 1.

**21.** A method of inhibiting a mast cell degranulation inhibitor, which comprises administering to a mammal an effective dose of (i) an antagonist to a protein comprising the same or substantially the same amino acid sequence as the amino acid sequence of SEQ ID NO: 1, its partial peptide, or a salt thereof, (ii) an antibody against said protein, its partial peptide, or a salt thereof, (iii) a polynucleotide comprising an entire or part of a nucleotide sequence complementary or substantially complementary to a polynucleotide comprising a polynucleotide encoding said protein or its partial peptide, or (iv) siRNA or shRNA for a polynucleotide comprising a polynucleotide encoding said protein or its partial peptide.

**22.** Use of (i) an antagonist to a protein comprising the same or substantially the same amino acid sequence as the amino acid sequence of SEQ ID NO: 1, its partial peptide, or a salt thereof, (ii) an antibody against said protein, its partial peptide, or a salt thereof, (iii) a polynucleotide comprising an entire or part of a nucleotide sequence complementary or substantially complementary to a polynucleotide comprising a polynucleotide encoding said protein or its partial peptide, or (iv) an siRNA or shRNA for a polynucleotide comprising a polynucleotide encoding said protein or its partial peptide, for the manufacture of a mast cell degranulation inhibitor.

**INTERNATIONAL SEARCH REPORT**

International application No.
PCT/JP2006/309212

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
See extra sheet.

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
BIOSIS/MEDLINE/WPIDS/CA(STN), GenBank/EMBL/DDBJ/GeneSeq, SwissProt/PIR/Geneseq

C. DOCUMENTS CONSIDERED TO BE RELEVANT

| Category* | Citation of document, with indication, where appropriate, of the relevant passages | Relevant to claim No. |
|---|---|---|
| Y | WO 2005/028667 A1 (The Institute of Physical and Chemical Research et al.), 31 March, 2005 (31.03.05), Particularly, page 3 (Family: none) | 1-9,18-20 |
| Y | JP 2004-33211 A (Pfizer Inc.), 05 February, 2004 (05.02.04), & AU 2003206014 A1 & EP 1340979 A2 & US 2003/171293 A1 & WO 03/073107 A2 | 1-9,18-20 |
| Y | Ødum L. et al., Pituitary adenylate cyclase activating polypeptide (PACAP) is localized in human dermal neurons and causes histamine release from skin mast cells, Inflammation Research, 1998, Vol.47, No.12, pages 488 to 492 | 4 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 August, 2006 (01.08.06) | 08 August, 2006 (08.08.06) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2006/309212

C (Continuation). DOCUMENTS CONSIDERED TO BE RELEVANT

| Category* | Citation of document, with indication, where appropriate, of the relevant passages | Relevant to claim No. |
|---|---|---|
| P,A | KAMOHARA, M. et al., Identification of MrgX2 as a human G-protein-coupled receptor for proadrenomedullin N-terminal peptides., Biochemical and Biophysical Research Communications, May 2005, Vol.330, No.4, pages 1146 to 1152. | 1-9,18-20 |
| P,A | JP 2005-304438 A (Astellas Pharma Inc.), 04 November, 2005 (04.11.05), (Family: none) | 1-9,18-20 |
| A | JP 2002-355052 A (Takeda Chemical Industries, Ltd.), 10 December, 2002 (10.12.02), & AU 6946301 A & US 2006/057663 A1 & WO 02/04641 A1 | 1-9,18-20 |

Form PCT/ISA/210 (continuation of second sheet) (April 2005)

**INTERNATIONAL SEARCH REPORT**

International application No.
PCT/JP2006/309212

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 17 and 21
because they relate to subject matter not required to be searched by this Authority, namely:
The inhibition of the degranulation of a mast cell *in vivo* relates to a method for treatment of the human body, and therefore the inventions of claims 17 and 21 relate to a subject matter which this international searching authority is not required to search.

2. ☒ Claims Nos.: 10-16 and 22
because they relate to parts of the international application that do not comply with the prescribed requirements to such an extent that no meaningful international search can be carried out, specifically:
Since the scopes of claims 10-16 and 22 are not clearly defined or are not fully supported by the description, and are not clearly and fully disclosed in the description, the international search was not made on these claims. For more detailed comments, see the extra annex sheet.

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, the payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

INTERNATIONAL SEARCH REPORT

International application No.
PCT/JP2006/309212

Continuation of Box No.II-2 of continuation of first sheet

With respect to the "antagonist" in claims 10 and 22, although it is shown in the section "Examples" that L-703606 which is an antagonist of NK1 receptor inhibits the degranulation caused by substance P, it is unclear whether or not this compound is an antagonist of a protein having the amino acid sequence depicted in SEQ ID NO:1 or the like and it is not stated specifically and unclear what types of substances other than this compound are included in the category "the antagonist". Thus, these claims are not supported by or disclosed in the description. Even though the common technical knowledge at the time of the filing of the present application is taken into the consideration, it is quite unclear what types of compounds are specifically included or what types of compounds are not included, and therefore these claims are claimed quite unclearly. Thus, a meaningful international search cannot be made on these claims. Similar comments apply to claim 16 which refers to claims 10.

Similarly, with respect to the "agonist" in claims 13 and 22, it is not specifically stated and unclear what types of compounds are included in the category "the agonist". Thus, these claims are not supported by or disclosed in the description. Even though the common technical knowledge at the time of the filing of the present application is taken into the consideration, it is quite unclear what types of compounds are specifically included or what types of compounds are not included, and therefore these claims are claimed quite unclearly. Thus, a meaningful international search cannot be made on these claims. Similar comments apply to claim 16 which refers to claims 13.

With respect to the antibodies in claims 11-13 and 22, it is not shown in the description that an antibody directed against a protein having the amino acid sequence depicted in SEQ ID NO:1 or an antibody directed against an agonist is prepared actually and the antibody can inhibit the degranulation and that the antibody can be used in the diagnosis of various diseases such as immune diseases and urinary organ diseases, and therefore the antibodies are not regarded to be supported by the description.

With respect to the diagnosis agent comprising the polynucleotide of claim 14, 15 or 22 and a degranulation inhibitor comprising siRNA or shRNA for the polynucleotide, it is not shown in the description that the polynucleotide can be actually used in the diagnosis of various diseases and that the siRNA or shRNA is actually prepared and it can inhibit the degranulation, and the diagnosis agent and the degranulation inhibitor are not regarded to be supported by the description.

Similar comments apply to claim 16 which refers to these claims.

**INTERNATIONAL SEARCH REPORT**

International application No.
PCT/JP2006/309212

Annex:

With respect to the "ligand" in claim 1, although various examples of the ligand are mentioned in the description, those compounds which are supported by the description in the meaning within PCT Article 6 and disclosed in the meaning within PCT Article 5 are only limited to substance P, cortistatin, hypophysis adenylate cyclase activating polypeptide, vasoactive intestinal peptide and PAM-12 and it is unclear what types of substances other than these compounds can actually serve as the ligand.

Same comments apply to the similar parts of claims 4, 5, 7, 9 and 18-20 and to claims 3, 6 and 8 which refer to these claims.

Thus, the search was only made on the parts which are supported by and disclosed in the description, namely the above-mentioned compounds.

Complete search was made on claim 2.

Form PCT/ISA/210 (extra sheet) (April 2005)

**INTERNATIONAL SEARCH REPORT**

International application No.
PCT/JP2006/309212

Continuation of A. CLASSIFICATION OF SUBJECT MATTER
(International Patent Classification (IPC))

C12N15/09(2006.01)i, A61K31/7052(2006.01)i, A61K39/395(2006.01)i, A61K45/00(2006.01)i, A61K48/00(2006.01)i, A61P1/00(2006.01)i, A61P1/04(2006.01)i, A61P1/16(2006.01)i, A61P3/04(2006.01)i, A61P3/06(2006.01)i, A61P3/10(2006.01)i, A61P9/04(2006.01)i, A61P9/06(2006.01)i, A61P9/10(2006.01)i, A61P11/00(2006.01)i, A61P11/06(2006.01)i, A61P11/08(2006.01)i, A61P13/00(2006.01)i, A61P13/10(2006.01)i, A61P13/12(2006.01)i, A61P15/00(2006.01)i, A61P17/00(2006.01)i, A61P21/04(2006.01)i, A61P27/02(2006.01)i, A61P27/16(2006.01)i, A61P29/00(2006.01)i, A61P35/00(2006.01)i, A61P37/00(2006.01)i, A61P37/02(2006.01)i, A61P37/08(2006.01)i, A61P43/00(2006.01)i, C12Q1/02(2006.01)i, C12Q1/68(2006.01)i, G01N33/15(2006.01)i, G01N33/50(2006.01)i, G01N33/53(2006.01)i, C07K14/705(2006.01)n

(According to International Patent Classification (IPC) or to both national classification and IPC)

Continuation of B. FIELDS SEARCHED
Minimum documentation searched (International Patent Classification (IPC))

C12N15/09, A61K31/7052, A61K39/395, A61K45/00, A61K48/00, A61P1/00, A61P1/04, A61P1/16, A61P3/04, A61P3/06, A61P3/10, A61P9/04, A61P9/06, A61P9/10, A61P11/00, A61P11/06, A61P11/08, A61P13/00, A61P13/10, A61P13/12, A61P15/00, A61P17/00, A61P21/04, A61P27/02, A61P27/16, A61P29/00, A61P35/00, A61P37/00, A61P37/02, A61P37/08, A61P43/00, C12Q1/02, C12Q1/68, G01N33/15, G01N33/50, G01N33/53, C07K14/705

Minimum documentation searched (classification system followed by classification symbols)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03073107 A
- WO 0198330 A
- WO 2005028667 A


### Non-patent literature cited in the description


- *Proc. Natl. Acad. Sci.,* 1987, vol. 84, 2975-2979
- *Arch. Dermatol. Res,* 1993, vol. 285, 341-346
- *Brain Behav. Immun,* 2005, vol. 19, 252-262
- *Br. J. Dermatol,* 2002, vol. 147, 71-79
- *Arch. Dermatol. Res,* 1997, vol. 289, 256-260
- *Arch. Dermatol. Res,* 2000, vol. 292, 418-421
- *Gastroenterology,* 2004, vol. 126, 693-702
- *Scand. J. Gastroentorl,* 2005, vol. 40, 129-140
- *Am. J. Physiol. Gastrointest. Liver Physiol.,* 2000, vol. 279, G1298-G1306
- *Am. J. Physiol. Renal Physiol.,* 2002, vol. 283, F616-F629
- *Biochem. Biophys. Res. Commun.,* 2005, vol. 330, 1146-1152
- *Blood,* 2001, vol. 97, 2045-2052
- *Clin. Exp. Immunol,* 2001, vol. 124, 150-156
- *Br. J. Dermatol,* 1994, vol. 131, 348-353
- *Int. Arch. Allergy Immunol,* 1994, vol. 117, 48-51
- *Exp. Dermatol,* 2001, vol. 10, 312-320
- **M. MOUSLI et al.** *FEBS Lett,* January 1990, vol. 259 (2), 260-262
- **R. SABAN et al.** *Am. J. Physiol. Renal Physiol.,* May 2002, vol. 283, F616-F629
- **A. H. Y. LAU et al.** *European Journal of Pharmacology,* 2001, vol. 414, 295-303
- **D. LORENZ et al.** *J. Gen. Physiol.,* November 1998, vol. 112, 577-591
- **S. GUHL et al.** *J. Neuroimmunology,* April 2005, vol. 163, 92-101
- *Inflamm. Res,* 1998, vol. 47, 488-492
- *Ann. N. Y. Acad. Sci,* 1998, vol. 865, 141-146
- *Regul. Pept,* 1999, vol. 82, 65-69
- *Br. J. Pharmacol,* 1988, vol. 95, 121-130
- *Genomics,* 1999, vol. 56, 12-21
- *Biochim. Biophys. Acta,* 1999, vol. 1446, 57-70
- **M. BODANSZKY ; M.A. ONDETTI.** Peptide Synthesis. Interscience Publishers, 1966
- **SCHROEDER ; LUEBKE.** The Peptide. Academic Press, 1965
- **NOBUO IZUMIYA et al.** Peptide Gosei-no-Kiso to Jikken (Basics and experiments of peptide synthesis. Maruzen Co, 1975
- **HARUAKI YAJIMA ; SHUNPEI SAKAKIBARA.** *Seikagaku Jikken Koza (Biochemical Experiment) 1, Tanpakushitsu no Kagaku (Chemistry of Proteins,* 1977, vol. IV, 205
- Zoku Iyakuhin no Kaihatsu (A sequel to Development of Pharmaceuticals. Hirokawa Shoten, vol. 14
- **J. SAMBROOK et al.** Molecular Cloning. Cold Spring Harbor Lab. Press, 1989
- *Proc. Natl. Acad. Sci. U.S.A.,* 1968, vol. 60, 160
- *Nucleic Acids Research,* 1981, vol. 9, 309
- *Journal of Molecular Biology,* 1978, vol. 120, 517
- *Journal of Molecular Biology,* 1969, vol. 41, 459
- *Genetics,* 1954, vol. 39, 440
- *Gene,* 1983, vol. 24, 255
- *Journal of Biochemistry,* 1984, vol. 95, 87
- **VAUGHN, J. L et al.** *In Vivo,* 1977, vol. 13, 213-217
- **MAEDA et al.** *Nature,* 1985, vol. 315, 592
- *Proc. Natl. Acad. Sci. U.S.A.,* 1972, vol. 69, 2110
- *Gene,* 1982, vol. 17, 107
- *Molecular & General Genetics,* 1979, vol. 168, 111
- *Methods in Enzymology,* 1991, vol. 194, 182-187
- *Proc. Natl. Acad. Sci. U.S.A.,* 1978, vol. 75, 1929
- *Bio/Technology,* 1988, vol. 6, 47-55
- Shin Saibo Kogaku Jikken Protocol (New Cell Engineering Experimental Protocol. Saibo Kogaku (Cell Engineering. Shujunsha, 1995, 263-267
- *Virology,* 1973, vol. 52, 456
- **MILLER.** Journal of Experiments in Molecular Genetics. Cold Spring Harbor Laboratory, 1972, 431-433
- **BOSTIAN, K. L et al.** *Proc. Natl. Acad. Sci. U.S.A.,* 1980, vol. 77, 4505

- **BITTER, G. A et al.** *Proc. Natl. Acad. Sci. U.S.A.,* 1984, vol. 81, 5330
- **GRACE, T. C. C.** *Nature,* 1962, vol. 195, 788
- *Science,* 1952, vol. 122, 501
- *Virology,* 1959, vol. 8, 396
- *The Journal of the American Medical Association,* 1967, vol. 199, 519
- *Proceeding of the Society for the Biological Medicine,* 1950, vol. 73, 1
- **KOEHLER ; MILSTEIN.** *Nature,* 1975, vol. 256, 495
- **J. KAWAKAMI et al.** *Pharm. Tech.,* 1992, vol. 8, 247
- *Pharm. Tech.,* 1992, vol. 8, 395
- **S. T. CROOKE et al.** Antisense Research and Applications. CRC Press, 1993
- *Molecular and Cellular Biology,* 1995, vol. 15, 6188-6195
- Radioimmunoassay. Kodansha, 1974
- Sequel to the Radioimmunoassay. Kodansha, 1979
- Enzyme immunoassay. Igakushoin, 1978
- Immunoenzyme assay. Igakushoin, 1982
- Immunoenzyme assay. Igakushoin, 1987
- Immunochemical Techniques (Part A. Methods in ENZYMOLOGY. vol. 70
- Immunochemical Techniques (Part B. Methods in ENZYMOLOGY. vol. 73
- Immunochemical Techniques (Part C. Methods in ENZYMOLOGY. vol. 74
- Immunochemical Techniques (Part D: Selected Immunoassays. Methods in ENZYMOLOGY. vol. 84
- Immunochemical Techniques (Part E: Monoclonal Antibodies and General Immunoassay Methods. *Methods in ENZYMOLOGY,* vol. 92
- Immunochemical Techniques (Part I: Hybridoma Technology and Monoclonal Antibodies. Methods in ENZYMOLOGY. Academic Press Publishing, vol. 121
- *Genomics,* 1989, vol. 5, 874-879
- *Proceedings of the National Academy of Sciences of the United States of America,* 1989, vol. 86, 2766-2770
- *Nature,* 2001, vol. 411, 494
- *TRENDS in Molecular Medicine,* 2001, vol. 7, 221
- *Biochim. Biophys. Acta,* 1994, vol. 1219, 251-259
- **HINUMA, S et al.** *Biochim. Biophys. Acta,* 1994, vol. 1219, 251-259

Sequence Listing

<110> Takeda Pharmaceutical Company Limited

<120> Degranulation Inhibitor

<130> G07-0076

<140> PCT/JP2006/309212
<141> 2006-04-27

<150> JP2005-133503
<151> 2005-04-28

<160> 45

<210> 1
<211> 330
<212> PRT
<213> Homo sapiens

<400> 1
Met Asp Pro Thr Thr Pro Ala Trp Gly Thr Glu Ser Thr Thr Val Asn
5 10 15
Gly Asn Asp Gln Ala Leu Leu Leu Leu Cys Gly Lys Glu Thr Leu Ile
20 25 30
Pro Val Phe Leu Ile Leu Phe Ile Ala Leu Val Gly Leu Val Gly Asn
35 40 45
Gly Phe Val Leu Trp Leu Leu Gly Phe Arg Met Arg Arg Asn Ala Phe
50 55 60
Ser Val Tyr Val Leu Ser Leu Ala Gly Ala Asp Phe Leu Phe Leu Cys
65 70 75 80
Phe Gln Ile Ile Asn Cys Leu Val Tyr Leu Ser Asn Phe Phe Cys Ser
85 90 95
Ile Ser Ile Asn Phe Pro Ser Phe Phe Thr Thr Val Met Thr Cys Ala
100 105 110
Tyr Leu Ala Gly Leu Ser Met Leu Ser Thr Val Ser Thr Glu Arg Cys
115 120 125
Leu Ser Val Leu Trp Pro Ile Trp Tyr Arg Cys Arg Arg Pro Arg His
130 135 140
Leu Ser Ala Val Val Cys Val Leu Leu Trp Ala Leu Ser Leu Leu Leu
145 150 155 160
Ser Ile Leu Glu Gly Lys Phe Cys Gly Phe Leu Phe Ser Asp Gly Asp
165 170 175
Ser Gly Trp Cys Gln Thr Phe Asp Phe Ile Thr Ala Ala Trp Leu Ile
180 185 190
Phe Leu Phe Met Val Leu Cys Gly Ser Ser Leu Ala Leu Leu Val Arg

195 200 205
Ile Leu Cys Gly Ser Arg Gly Leu Pro Leu Thr Arg Leu Tyr Leu Thr
210 215 220
Ile Leu Leu Thr Val Leu Val Phe Leu Leu Cys Gly Leu Pro Phe Gly
225 230 235 240
Ile Gln Trp Phe Leu Ile Leu Trp Ile Trp Lys Asp Ser Asp Val Leu
245 250 255
Phe Cys His Ile His Pro Val Ser Val Val Leu Ser Ser Leu Asn Ser
260 265 270
Ser Ala Asn Pro Ile Ile Tyr Phe Phe Val Gly Ser Phe Arg Lys Gln
275 280 285
Trp Arg Leu Gln Gln Pro Ile Leu Lys Leu Ala Leu Gln Arg Ala Leu
290 295 300
Gln Asp Ile Ala Glu Val Asp His Ser Glu Gly Cys Phe Arg Gln Gly
305 310 315 320
Thr Pro Glu Met Ser Arg Ser Ser Leu Val
325 330

<210> 2
<211> 990
<212> DNA
<213> Homo sapiens

<400> 2
atggatccaa ccaccccggc ctggggaaca gaaagtacaa cagtgaatgg aaatgaccaa 60
gcccttcttc tgctttgtgg caaggagacc ctgatcccgg tcttcctgat ccttttcatt 120
gccctggtcg ggctggtagg aaacgggttt gtgctctggc tcctgggctt ccgcatgcgc 180
aggaacgcct tctctgtcta cgtcctcagc ctggccgggg ccgacttcct cttcctctgc 240
ttccagatta taaattgcct ggtgtacctc agtaacttct tctgttccat ctccatcaat 300
ttccctagct tcttcaccac tgtgatgacc tgtgcctacc ttgcaggcct gagcatgctg 360
agcaccgtca gcaccgagcg ctgcctgtcc gtcctgtggc ccatctggta tcgctgccgc 420
cgccccagac acctgtcagc ggtcgtgtgt gtcctgctct gggccctgtc cctactgctg 480
agcatcttgg aagggaagtt ctgtggcttc ttatttagtg atggtgactc tggttggtgt 540
cagacatttg atttcatcac tgcagcgtgg ctgatttttt tattcatggt tctctgtggg 600
tccagtctgg ccctgctggt caggatcctc tgtggctcca ggggtctgcc actgaccagg 660
ctgtacctga ccatcctgct cacagtgctg gtgttcctcc tctgcggcct gccctttggc 720
attcagtggt tcctaatatt atggatctgg aaggattctg atgtcttatt ttgtcatatt 780
catccagttt cagttgtcct gtcatctctt aacagcagtg ccaaccccat catttacttc 840
ttcgtgggct cttttaggaa gcagtggcgg ctgcagcagc cgatcctcaa gctggctctc 900
cagagggctc tgcaggacat tgctgaggtg gatcacagtg aaggatgctt ccgtcagggc 960
accccggaga tgtcgagaag cagtctggtg 990

<210> 3
<211> 23
<212> DNA
<213> Artificial

<220>
<223> Primer

<400> 3
aggctgagga cgtagacaga gaa 23

<210> 4
<211> 23
<212> DNA
<213> Artificial

<220>
<223> Primer

<400> 4
tgtgctctgg ctcctgggct tcc 23

<210> 5
<211> 77
<212> DNA
<213> Artificial

<220>
<223> synthetic oligo DNA

<400> 5
ctctgaccgc taccacgagc aagtctctgc caagcgcaag gtggtcaaaa tgatgattgt 60
cgtggtgtgc accttcg 77

<210> 6
<211> 21
<212> DNA
<213> Artificial

<220>
<223> Primer

<400> 6
accgctacca cgagcaagtc t 21

<210> 7
<211> 21
<212> DNA
<213> Artificial

<220>
<223> Primer

<400> 7
gtgcacacca cgacaatcat c 21

<210> 8
<211> 23
<212> DNA
<213> Artificial

<220>
<223> Probe

<400> 8
ttgaccacct tgcgcttggc aga 23

<210> 9
<211> 100
<212> DNA
<213> Artificial

<220>
<223> synthetic oligo DNA

<400> 9
acctgtgaca ttgtgactga agccaatatc tcatctggcc ctgagagcaa caccacgggc 60
atcacagcct tctccatgcc cagctggcag ctggcactgt 100

<210> 10
<211> 22
<212> DNA
<213> Artificial

<220>
<223> Primer

<400> 10
tgtgacattg tgactgaagc ca 22

<210> 11
<211> 20
<212> DNA
<213> Artificial

<220>
<223> Primer

<400> 11

ggcatggaga aggctgtgat 20

<210> 12
<211> 24
<212> DNA
<213> Artificial

<220>
<223> Probe

<400> 12
catctggccc tgagagcaac acca 24

<210> 13
<211> 89
<212> DNA
<213> Artificial

<220>
<223> synthetic oligo DNA

<400> 13
actgccgctt ccagaacttc tttcctatca cagctgtgtt cgccagcatc tactccatga 60
cggccattgc ggtggacagg tatatggct 89

<210> 14
<211> 24
<212> DNA
<213> Artificial

<220>
<223> Primer

<400> 14
cgcttccaga acttctttcc tatc 24

<210> 15
<211> 21
<212> DNA
<213> Artificial

<220>
<223> Primer

<400> 15
tatacctgtc caccgcaatg g 21

<210> 16
<211> 28
<212> DNA
<213> Artificial

<220>
<223> Primer

<400> 16
cagctgtgtt cgccagcatc tactccat 28

<210> 17
<211> 993
<212> DNA
<213> Homo sapiens

<400> 17
atggatccaa ccaccccggc ctggggaaca gaaagtacaa cagtgaatgg aaatgaccaa 60
gcccttcttc tgctttgtgg caaggagacc ctgatcccgg tcttcctgat ccttttcatt 120
gccctggtcg ggctggtagg aaacgggttt gtgctctggc tcctgggctt ccgcatgcgc 180
aggaacgcct tctctgtcta cgtcctcagc ctggccgggg ccgacttcct cttcctctgc 240
ttccagatta taaattgcct ggtgtacctc agtaacttct tctgttccat ctccatcaat 300
ttccctagct tcttcaccac tgtgatgacc tgtgcctacc ttgcaggcct gagcatgctg 360
agcaccgtca gcaccgagcg ctgcctgtcc gtcctgtggc ccatctggta tcgctgccgc 420
cgccccagac acctgtcagc ggtcgtgtgt gtcctgctct gggccctgtc cctactgctg 480
agcatcttgg aagggaagtt ctgtggcttc ttatttagtg atggtgactc tggttggtgt 540
cagacatttg atttcatcac tgcagcgtgg ctgatttttt tattcatggt tctctgtggg 600
tccagtctgg ccctgctggt caggatcctc tgtggctcca ggggtctgcc actgaccagg 660
ctgtacctga ccatcctgct cacagtgctg gtgttcctcc tctgcggcct gccctttggc 720
attcagtggt tcctaatatt atggatctgg aaggattctg atgtcttatt ttgtcatatt 780
catccagttt cagttgtcct gtcatctctt aacagcagtg ccaaccccat catttacttc 840
ttcgtgggct cttttaggaa gcagtggcgg ctgcagcagc cgatcctcaa gctggctctc 900
cagagggctc tgcaggacat tgctgaggtg gatcacagtg aaggatgctt ccgtcagggc 960
accccggaga tgtcgagaag cagtctggtg tag 993

<210> 18
<211> 19
<212> DNA
<213> Artificial

<220>
<223> Primer

<400> 18
ggtcgggctg gtaggaaac 19

<210> 19

<211> 155
<212> DNA
<213> Artificial

<220>
<223> synthetic olig DNA

<400> 19
ggtttggatg acaaggcagc gagtttggat gagcagcaga ccatgttcta cggttctgtg 60
aagaccggct acaccattgg ctacggcctg tccctcgcca cccttctggt cgccacagct 120
atcctgagcc tgttcaggaa gctccactgc acgcg 155

<210> 20
<211> 20
<212> DNA
<213> Artificial

<220>
<223> Primer

<400> 20
ggatgacaag gcagcgagtt 20

<210> 21
<211> 21
<212> DNA
<213> Artificial

<220>
<223> Primer

<400> 21
gcagtggagc ttcctgaaca g 21

<210> 22
<211> 25
<212> DNA
<213> Artificial

<220>
<223> Probe

<400> 22
ccacccttct ggtcgccaca gctat 25

<210> 23
<211> 129

<212> DNA
<213> Artificial

<220>
<223> synthetic oligo DNA

<400> 23
tcggcggcaa cgaccagtct cagtacaaga ggctggccaa gtccacgctc ctgcttatcc 60
cgctgttcgg cgtccactac atggtgtttg ccgtgtttcc catcagcatc tcctccaaat 120
accagatac 129

<210> 24
<211> 22
<212> DNA
<213> Artificial

<220>
<223> Primer

<400> 24
ggcaacgacc agtctcagta ca 22

<210> 25
<211> 23
<212> DNA
<213> Artificial

<220>
<223> Primer

<400> 25
tggtatttgg aggagatgct gat 23

<210> 26
<211> 26
<212> DNA
<213> Artificial

<220>
<223> Probe

<400> 26
ctggccaagt ccacgctcct gcttat 26

<210> 27
<211> 102
<212> DNA

<213> Artificial

<220>
<223> synthetic oligo DNA

<400> 27
actgcacacg caacttcatc cacatgaacc tgtttgtgtc gttcatgctg agggcgatct 60
ccgtcttcat caaagactgg attctgtatg cggagcagga ca 102

<210> 28
<211> 21
<212> DNA
<213> Artificial

<220>
<223> Primer

<400> 28
acacgcaact tcatccacat g 21

<210> 29
<211> 20
<212> DNA
<213> Artificial

<220>
<223> Primer

<400> 29
tgctccgcat acagaatcca 20

<210> 30
<211> 24
<212> DNA
<213> Artificial

<220>
<223> Probe

<400> 30
acggagatcg ccctcagcat gaac 24

<210> 31
<211> 1791
<212> DNA
<213> Artificial

<220>
<223> base sequence encoding a fusion protein

<400> 31
atggatccaa ccaccccggc ctggggaaca gaaagtacaa cagtgaatgg aaatgaccaa 60
gcccttcttc tgctttgtgg caaggagacc ctgatcccgg tcttcctgat ccttttcatt 120
gccctggtcg ggctggtagg aaacgggttt gtgctctggc tcctgggctt ccgcatgcgc 180
aggaacgcct tctctgtcta cgtcctcagc ctggccgggg ccgacttcct cttcctctgc 240
ttccagatta taaattgcct ggtgtacctc agtaacttct tctgttccat ctccatcaat 300
ttccctagct tcttcaccac tgtgatgacc tgtgcctacc ttgcaggcct gagcatgctg 360
agcaccgtca gcaccgagcg ctgcctgtcc gtcctgtggc ccatctggta tcgctgccgc 420
cgccccagac acctgtcagc ggtcgtgtgt gtcctgctct gggccctgtc cctactgctg 480
agcatcttgg aagggaagtt ctgtggcttc ttatttagtg atggtgactc tggttggtgt 540
cagacatttg atttcatcac tgcagcgtgg ctgatttttt tattcatggt tctctgtggg 600
tccagtctgg ccctgctggt caggatcctc tgtggctcca ggggtctgcc actgaccagg 660
ctgtacctga ccatcctgct cacagtgctg gtgttcctcc tctgcggcct gccctttggc 720
attcagtggt tcctaatatt atggatctgg aaggattctg atgtcttatt ttgtcatatt 780
catccagttt cagttgtcct gtcatctctt aacagcagtg ccaaccccat catttacttc 840
ttcgtgggct cttttaggaa gcagtggcgg ctgcagcagc cgatcctcaa gctggctctc 900
cagagggctc tgcaggacat tgctgaggtg gatcacagtg aaggatgctt ccgtcagggc 960
accccggaga tgtcgagaag cagtctggtg gctagcaaag gagaagaact cttcactgga 1020
gttgtcccaa ttcttgttga attagatggt gatgttaacg gccacaagtt ctctgtcagt 1080
ggagagggtg aaggtgatgc aacatacgga aaacttaccc tgaagttcat ctgcactact 1140
ggcaaactgc ctgttccatg gccaacacta gtcactactc tctctcatgg tgttcaatgc 1200
ttttcaagat acccggatca tatgaaacgg catgactttt tcaagagtgc catgcccgaa 1260
ggttatgtac aggaaaggac catcttcttc aaagatgacg gcaactacaa gacacgtgct 1320
gaagtcaagt ttgaaggtga tacccttgtt aatagaatcg agttaaaagg tattgatttt 1380
aaagaagatg gaaacattct tggacacaaa ttggaataca actataactc acacaatgta 1440
tacatcatgg cagacaaaca aaagaatgga atcaaagcga acttcaagat ccgccacaac 1500
attgaagatg gaagcgttca actagcagac cattatcaac aaaatactcc aattggcgat 1560
ggccctgtcc ttttaccaga caaccattac ctgtccacac aatctgccct ttcgaaagat 1620
cccaacgaaa agagagacca catggtcctt cttgagtttg taacagctgc tgggattaca 1680
catggcatgg atgaactgta caacatcgat ggaggcggag gtggagggcc cggttaccgg 1740
taccggatcc agatatctgg gcggccgctc agcaagcttc gcgaattctg a 1791

<210> 32
<211> 993
<212> DNA
<213> Homo sapiens

<400> 32
atggatccaa ccaccccggc ctggggaaca gaaagtacaa cagtgaatgg aaatgaccaa 60
gcccttcttc tgctttgtgg caaggagacc ctgatcccgg tcttcctgat ccttttcatt 120
gccctggtcg ggctggtagg aaacgggttt gtgctctggc tcctgggctt ccgcatgcgc 180
aggaacgcct tctctgtcta cgtcctcagc ctggccgggg ccgacttcct cttcctctgc 240
ttccagatta taaattgcct ggtgtacctc agtaacttct tctgttccat ctccatcaat 300
ttccctagct tcttcaccac tgtgatgacc tgtgcctacc ttgcaggcct gagcatgctg 360

agcaccgtca gcaccgagcg ctgcctgtcc gtcctgtggc ccatctggta tcgctgccgc 420
cgccccagac acctgtcagc ggtcgtgtgt gtcctgctct gggccctgtc cctactgctg 480
agcatcttgg aagggaagtt ctgtggcttc ttatttagtg atggtgactc tggttggtgt 540
cagacatttg atttcatcac tgcagcgtgg ctgatttttt tattcatggt tctctgtggg 600
tccagtctgg ccctgctggt caggatcctc tgtggctcca ggggtctgcc actgaccagg 660
ctgtacctga ccatcctgct cacagtgctg gtgttcctcc tctgcggcct gccctttggc 720
attcagtggt tcctaatatt atggatctgg aaggattctg atgtcttatt ttgtcatatt 780
catccagttt cagttgtcct gtcatctctt aacagcagtg ccaaccccat catttacttc 840
ttcgtgggct cttttaggaa gcagtggcgg ctgcagcagc cgatcctcaa gctggctctc 900
cagagggctc tgcaggacat tgctgaggtg gatcacagtg aaggatgctt ccgtcagggc 960
accccggaga tgtcgagaag cagtctggtg tag 993

<210> 33
<211> 20
<212> DNA
<213> Artificial

<220>
<223> Primer

<400> 33
ggagaagggt gaccgactca 20

<210> 34
<211> 18
<212> DNA
<213> Artificial

<220>
<223> Primer

<400> 34
tgcccagact cggcaaag 18

<210> 35
<211> 25
<212> DNA
<213> Artificial

<220>
<223> Probe

<400> 35
cgctgagatc aatcggcccg actat 25

<210> 36
<211> 79
<212> DNA
<213> Artificial

<220>
<223> synthetic oligo DNA

<400> 36
cagctggaga agggtgaccg actcagcgct gagatcaatc ggcccgacta tctcgacttt 60
gccgagtctg ggcaggtct 79

<210> 37
<211> 22
<212> DNA
<213> Artificial

<220>
<223> Primer

<400> 37
acccagcagt cgtctttgtc ac 22

<210> 38
<211> 21
<212> DNA
<213> Artificial

<220>
<223> Primer

<400> 38
cccgaaccca tttcttctct g 21

<210> 39
<211> 26
<212> DNA
<213> Artificial

<220>
<223> Probe

<400> 39

cgaaagaacc gccaagtgtg tgccaa 26

<210> 40
<211> 82
<212> DNA
<213> Artificial

<220>
<223> synthetic oligo DNA

<400> 40
ctccaaccca gcagtcgtct ttgtcacccg aaagaaccgc caagtgtgtg ccaacccaga 60
gaagaaatgg gttcgggagt ac 82

<210> 41
<211> 24
<212> DNA
<213> Artificial

<220>
<223> Primer

<400> 41
ctcgcgagct atagaagaat cacc 24

<210> 42
<211> 21
<212> DNA
<213> Artificial

<220>
<223> Primer

<400> 42
tccttggcca caatggtctt g 21

<210> 43
<211> 29
<212> DNA
<213> Artificial

<220>
<223> Probe

<400> 43
cagcaagtgt cccaaagaag ctgtgatct 29

<210> 44
<211> 87
<212> DNA
<213> Artificial

<220>
<223> synthetic oligo DNA

<400> 44
agaggctcgc gagctataga agaatcacca gcagcaagtg tcccaaagaa gctgtgatct 60
tcaagaccat tgtggccaag gagatct 87

<210> 45
<211> 1791
<212> DNA
<213> Artificial

<220>
<223> base sequence encoding a fusion protein

<400> 45
atggatccaa ccaccccggc ctggggaaca gaaagtacaa cagtgaatgg aaatgaccaa 60
gcccttcttc tgctttgtgg caaggagacc ctgatcccgg tcttcctgat ccttttcatt 120
gccctggtcg ggctggtagg aaacgggttt gtgctctggc tcctgggctt ccgcatgcgc 180
aggaacgcct tctctgtcta cgtcctcagc ctggccgggg ccgacttcct cttcctctgc 240
ttccagatta taaattgcct ggtgtacctc agtaacttct tctgttccat ctccatcaat 300
ttccctagct tcttcaccac tgtgatgacc tgtgcctacc ttgcaggcct gagcatgctg 360
agcaccgtca gcaccgagcg ctgcctgtcc gtcctgtggc ccatctggta tcgctgccgc 420
cgccccagac acctgtcagc ggtcgtgtgt gtcctgctct gggccctgtc cctactgctg 480
agcatcttgg aagggaagtt ctgtggcttc ttatttagtg atggtgactc tggttggtgt 540
cagacatttg atttcatcac tgcagcgtgg ctgatttttt tattcatggt tctctgtggg 600
tccagtctgg ccctgctggt caggatcctc tgtggctcca ggggtctgcc actgaccagg 660
ctgtacctga ccatcctgct cacagtgctg gtgttcctcc tctgcggcct gccctttggc 720
attcagtggt tcctaatatt atggatctgg aaggattctg atgtcttatt ttgtcatatt 780
catccagttt cagttgtcct gtcatctctt aacagcagtg ccaaccccat catttacttc 840
ttcgtgggct cttttaggaa gcagtggcgg ctgcagcagc cgatcctcaa gctggctctc 900
cagagggctc tgcaggacat tgctgaggtg gatcacagtg aaggatgctt ccgtcagggc 960
accccggaga tgtcgagaag cagtctggtg gctagcaaag gagaagaact cttcactgga 1020
gttgtcccaa ttcttgttga attagatggt gatgttaacg gccacaagtt ctctgtcagt 1080
ggagagggtg aaggtgatgc aacatacgga aaacttaccc tgaagttcat ctgcactact 1140
ggcaaactgc ctgttccatg gccaacacta gtcactactc tctctcatgg tgttcaatgc 1200

```
ttttcaagat acccggatca tatgaaacgg catgactttt tcaagagtgc catgcccgaa   1260
ggttatgtac aggaaaggac catcttcttc aaagatgacg gcaactacaa gacacgtgct   1320
gaagtcaagt ttgaaggtga tacccttgtt aatagaatcg agttaaaagg tattgatttt   1380
aaagaagatg gaaacattct tggacacaaa ttggaataca actataactc acacaatgta   1440
tacatcatgg cagacaaaca aaagaatgga atcaaagcga acttcaagat ccgccacaac   1500
attgaagatg gaagcgttca actagcagac cattatcaac aaaatactcc aattggcgat   1560
ggccctgtcc ttttaccaga caaccattac ctgtccacac aatctgccct ttcgaaagat   1620
cccaacgaaa agagagacca catggtcctt cttgagtttg taacagctgc tgggattaca   1680
catggcatgg atgaactgta caacatcgat ggaggcggag gtggagggcc cggttaccgg   1740
taccggatcc agatatctgg gcggccgctc agcaagcttc gcgaattctg a            1791
```